(19) **Europäisches Patentamt**
**European Patent Office**
**Office européen des brevets**

(11) **EP 1 716 169 B1**

(12) **EUROPEAN PATENT SPECIFICATION**

(45) Date of publication and mention
of the grant of the patent:
**02.12.2009 Bulletin 2009/49**

(51) Int Cl.:
***C07K 14/195*** *(2006.01)*     ***A61K 39/02*** *(2006.01)*

(21) Application number: **05701653.7**

(86) International application number:
**PCT/EP2005/050577**

(22) Date of filing: **09.02.2005**

(87) International publication number:
**WO 2005/077972 (25.08.2005 Gazette 2005/34)**

(54) **ORNITHOBACTERIUM RHINOTRACHEALE  SUBUNIT VACCINES**

IMPFSTOFFE MIT ORNITHOBAKTERIUM RHINOTRACHEALE-UNTEREINHEIT

VACCIN DE SOUS-UNITÉS D' ORNITHOBACTERIUM RHINOTRACHÉALE

(84) Designated Contracting States:
**AT BE BG CH CY CZ DE DK EE ES FI FR GB GR HU IE IS IT LI LT LU MC NL PL PT RO SE SI SK TR**

(30) Priority: **11.02.2004 EP 04075427**

(43) Date of publication of application:
**02.11.2006 Bulletin 2006/44**

(73) Proprietor: **Intervet International BV**
**5831 AN Boxmeer (NL)**

(72) Inventors:
- **SCHUIJFFEL, Danielle Francisca**
**NL-5353 LG Langenboom (NL)**
- **NUIJTEN, Petrus Johannes Maria**
**NL-5836 CD Sambeek (NL)**

(74) Representative: **Keus, Jacobus Albertus Ronald**
**INTERVET INTERNATIONAL B.V.**
**P.O. Box 31**
**5830 AA Boxmeer (NL)**

(56) References cited:
**EP-A- 0 625 190**

- **LOPES VANESSA ET AL: "Minimization of pathologic changes in Ornithobacterium rhinotracheale infection in turkeys by temperature-sensitive mutant strain" AVIAN DISEASES, vol. 46, no. 1, January 2002 (2002-01), pages 177-185, XP001197201 ISSN: 0005-2086**

- **SPRENGER S J ET AL: "ORNITHOBACTERIUM RHINOTRACHEALE INFECTION IN TURKEYS: IMMUNOPROPHYLAXIS STUDIES" AVIAN DISEASES, AMERICAN ASSOCIATION OF AVIAN PATHOLOGISTS, KENNET SQ., PA, US, vol. 44, July 2000 (2000-07), pages 549-555, XP002951930 ISSN: 0005-2086**

- **LOPES V C ET AL: "Development, characterization, and preliminary evaluation of a temperature-sensitive mutant of Ornithobacterium rhinotracheale for potential use as a live vaccine in turkeys" AVIAN DISEASES, vol. 46, no. 1, January 2002 (2002-01), pages 162-168, XP001197000 ISSN: 0005-2086**

- **VAN EMPEL PAUL ET AL: "Vaccination of chickens against Ornithobacterium rhinotracheale infection" AVIAN DISEASES, vol. 42, no. 3, July 1998 (1998-07), pages 572-578, XP008032542 ISSN: 0005-2086**

- **CAUWERTS K ET AL: "The effect of Ornithobacterium rhinotracheale vaccination of broiler breeder chickens on the performance of their progeny." AVIAN PATHOLOGY, vol. 31, no. 6, December 2002 (2002-12), pages 619-624, XP008032541 ISSN: 0307-9457**

- **VAN EMPEL P C M ET AL: "Ornithobacterium rhinotracheale: A review" June 1999 (1999-06), AVIAN PATHOLOGY, VOL. 28, NR. 3, PAGE(S) 217-227 , XP008032443 ISSN: 0307-9457 cited in the application the whole document**

EP 1 716 169 B1

**Description**

The present invention relates to nucleic acids encoding *Ornithobacterium rhinotracheale* proteins, to DNA fragments, recombinant DNA molecules, live recombinant carriers and host cells comprising such nucleic acids, to *Ornithobacterium rhinotracheale* proteins, to antibodies against such proteins, to such proteins for use in vaccines, to the use of such proteins in the manufacturing of such vaccines, to vaccines comprising such nucleic acids, DNA fragments, recombinant DNA molecules, live recombinant carriers, host cells, proteins or antibodies against such proteins, and to methods for the preparation of such vaccines.

*Ornithobacterium rhinotracheale* is a relatively recently discovered bacterium that is found more and more frequently in poultry farms, and in wild birds. Especially animals in commercial chicken farms, turkey farms and duck farms are frequently infected.

In commercial poultry, infection is associated with respiratory diseases: airsacculitis and pneumonia are the most common features of infection with *Ornithobacterium rhinotracheale.* These signs can be induced by aerosol in intra-tracheal or intra-thoracic administration of the organism and are aggravated by other factors such as respiratory viruses, bacteria or sub-optimal housing conditions. Osteitis, meningitis and joint-infections which can be induced by intravenous application have been associated with *Ornithobacterium rhinotracheale.* The infection can be transmitted horizontally, as well as vertically through eggs, which probably accounts for its rapid and worldwide spread. An extensive review of *Ornithobacterium rhinotracheale* has been given by van Empel, P.C.M. ad Hafez, H.M. in Avian Pathology 28:217-227 (1999). European Patent EP0.625.190 relates to both the *Ornithobacterium rhinotracheale* bacterium and to vaccines against *Ornithobacterium rhinotracheale.*

Serological research has revealed that *Ornithobacterium rhinotracheale* strains may have different serotypes, to a certain degree depending on the geographic origin of the strain and the host animal from which they were isolated. At this moment, eighteen different serotypes are found.

Therapeutic treatment of the disease can be difficult because acquired resistance against the regular antibiotics is very common within the genus. Moreover, there is an increasing reluctance against the use of antibiotics in food animals for both public health- and environmental reasons.

Vaccination offers an alternative for therapeutic treatment with antibiotics, but up till now, only vaccination with live attenuated vaccines and inactivated whole cell vaccines was possible.

Avian Diseases, vol. 44, pages 549-555, 2000, S.J. Sprenger et al. discloses the vaccination of 6 week old turkeys with either a live or killed *Ornithobacterium rhinotracheale* vaccine. At 14 or 21 weeks the birds were challenged with live *Ornithobacterium rhinotracheale.* The turkeys inoculated with the live or killed *Ornithobacterium rhinotracheale* vaccine were found to be protected from pathologic changes.

Avian Diseases, vol. 46, pages 177-185, 2002, V. Lopes et al. discloses the investigation of the protection elicited by a temperature sensitive (Ts) mutant of *Ornithobacterium rhinotracheale* vaccine against challenge with a pathogenic strain. The results indicated that the use of the Ts mutant strain of *Ornithobacterium rhinotracheale* as a live vaccine would be suitable to evoke protection against *Ornithobacterium rhinotracheale* infection in turkeys.

The success of live attenuated vaccines specifically for *Ornithobacterium rhinotracheale* depends highly on the right balance between attenuation and triggering of the immune system. Inactivated whole cell vaccines are basically safe and therefore, from a safety point of view would seem the preferred type of vaccine.

Inactivated whole cell vaccines however need to be given in a higher dose compared to live attenuated vaccines. As a general rule, most of the proteins present in a bacterium play no role in the triggering of the immune system, i.e. they are not relevant immunogens. This means that, in the case of inactivated whole cell vaccines, in order to provide humans or animals with a sufficient level of relevant immunogens a lot of non-protective material is additionally and unavoidably administered. This is not a desirable situation.

The use of subunit vaccines could overcome this problem, and would therefore be highly preferred, but currently no immunogenic subunit vaccines are known in the art for combating *Ornithobacterium rhinotracheale.*

Moreover, although live attenuated vaccines and inactivated whole cell preparations are known to provide a certain level of cross-protection against all *Ornithobacterium rhinotracheale* strains, subunit vaccines might or might not induce cross-reactivity.

The present invention aims at providing for the first time vaccines that are based upon *Ornithobacterium rhinotracheale* subunits that do induce cross-reactivity.

This objective is reached by providing eight novel *Ornithobacterium rhinotracheale* proteins that surprisingly play an important role in triggering a protective immune response, and by providing vaccines comprising one or more of these novel immunogenic proteins.

Even more surprisingly, these eight novel proteins were found no only to induce a protective homologous immune response, but to also induce a protective cross-reactive immune response.

A homologous immune response is a response against strains of the same serotype, whereas a cross-reactive immune response is a response against both serologically homologous and heterologous strains.

The first novel protein, Or01, having a molecular weight of 59.8 kD is encoded by a nucleic acid having a nucleotide sequence as depicted in SEQ ID NO: 1.

It is well-known in the art, that many different nucleotide sequences can encode one and the same protein. This phenomenon is commonly known as wobble in the second and especially the third base of each triplet encoding an amino acid. This phenomenon can result in a heterology of about 20-30% for two nucleotide sequences still encoding the same protein. Therefore, two nucleic acids having a nucleotide sequence homology of about 80 % can still encode one and the same protein.

Thus, one embodiment relates to a nucleic acid encoding a 59.8 kD *Ornithobacterium rhinotracheale* protein or a part of said nucleic acid that encodes an immunogenic fragment of said protein wherein said nucleic acid or said part thereof has at least 80 % homology with the nucleic acid of the *Ornithobacterium rhinotracheale* protein gene as depicted in SEQ ID NO: 1.

The molecular weight of the protein (and the seven other proteins) is determined on the basis of the molecular weight of the amino acids as given in the amino acid sequence.

Preferably, a nucleic acid according to the invention encoding this 59.8 kD *Ornithobacterium rhinotracheale* protein or a part of that nucleic acid that encodes an immunogenic fragment of that protein has at least 85 %, preferably 90 %, more preferably 95 % homology with the nucleic acid of the *Ornithobacterium rhinotracheale* protein gene as depicted in SEQ ID NO: 1.

Even more preferred is a homology level of 98 %, 99 % or even 100 %.

The level of nucleotide homology can be determined with the computer program "BLAST 2 SEQUENCES" by selecting sub-program: "BLASTN" that can be found at www.ncbi.nlm.nih.gov/blast/bl2sea/bl2.html.
A reference for this program is Tatiana A. Tatusova, Thomas L. Madden FEMS Microbiol. Letters 174: 247-250 (1999). Parameters used are the default parameters:

Reward for a match: +1. Penalty for a mismatch: -2. Open gap: 5. Extension gap: 2. Gap x_dropoff: 50.

Another approach for deciding if a certain nucleic acid sequence is or is not a nucleic acid sequence according to the invention relates to the question if that certain nucleic acid sequence does hybridize under stringent conditions to the nucleotide sequence as depicted in SEQ ID NO: 1 (or in SEQ ID NO: 3, 5,7, 9, 11, 13 or 15,. see below).
If a nucleic acid sequence hybridizes under stringent conditions to the nucleotide sequence as depicted in SEQ ID NO: 1, or of course as depicted in SEQ ID NO: 3, 5,7, 9, 11, 13 and 15, it is considered to be a nucleic acid sequence according to the invention.

The definition of stringent conditions follows from the formula of Meinkoth and Wahl (1984. Hybridization of nucleic acids immobilized on solid supports. Anal. Biochem. 138: 267-284.).

$$Tm = [81.5°C + 16.6(\log M) + 0.41(\% GC) - 0.61(\% \text{formamide}) - 500/L] - 1°C/1\% \text{ mismatch}$$

In this formula, M is molarity of monovalent cations; %GC is the percentage of guanosine and cytosine nucleotides in the DNA; L is the length of the hybrid in base pairs.

Stringent conditions are those conditions under which nucleic acid sequences or fragments thereof still hybridize, if they have a mismatch of 20 % at the most, preferably 10%, more preferably 8, 6, 5, 4,3, 2, 1 or 0% in that order or preference, to the nucleic acid sequence as depicted in any of the SEQ ID NO: 1, 3, 5,7, 9, 11, 13 or 15.

Another embodiment relates to a nucleic acid encoding a 58.2 kD *Ornithobacterium rhinotracheale* protein Or02, or a part of said nucleic acid that encodes an immunogenic fragment of said protein wherein said nucleic acid or said part thereof has at least 80 % homology with the nucleic acid of the *Ornithobacterium rhinotracheale* protein gene as depicted in SEQ ID NO: 3.

Preferably, a nucleic acid according to the invention encoding this 58.2 kD *Ornithobacterium rhinotracheale* protein or a part of that nucleic acid that encodes an immunogenic fragment of that protein has at least 85 %, preferably 90 %, more preferably 95 % homology with the nucleic acid of the *Ornithobacterium rhinotracheale* protein gene as depicted in SEQ ID NO: 3.

Even more preferred is a homology level of 98 %, 99 % or even 100 %.

Still another embodiment relates to a nucleic acid encoding a 46.0 kD *Ornithobacterium rhinotracheale* protein Or03 or a part of said nucleic acid that encodes an immunogenic fragment of said protein wherein said nucleic acid or said part thereof has at least 80 % homology with the nucleic acid of the *Ornithobacterium rhinotracheale* protein gene as depicted in SEQ ID NO: 5.

Preferably, a nucleic acid according to the invention encoding this 46.0 kD *Ornithobacterium rhinotracheale* protein or a part of that nucleic acid that encodes an immunogenic fragment of that protein has at least 85 %, preferably 90 %, more preferably 95 % homology with the nucleic acid of the *Ornithobacterium rhinotracheale* protein gene as depicted in SEQ ID NO: 5.

Even more preferred is a homology level of 98 %, 99 % or even 100 %.

Again another embodiment relates to a nucleic acid encoding a 37.2 kD *Ornithobacterium rhinotracheale* protein Or04 or a part of said nucleic acid that encodes an immunogenic fragment of said protein wherein said nucleic acid or said part thereof has at least 80 % homology with the nucleic acid of the *Ornithobacterium rhinotracheale* protein gene as depicted in SEQ ID NO: 7.

Preferably, a nucleic acid according to the invention encoding this 37.2 kD *Ornithobacterium rhinotracheale* protein or a part of that nucleic acid that encodes an immunogenic fragment of that protein has at least 85 %, preferably 90 %, more preferably 95 % homology with the nucleic acid of the *Ornithobacterium rhinotracheale* protein gene as depicted in SEQ ID NO: 7.

Even more preferred is a homology level of 98 %, 99 % or even 100 %.

Another embodiment relates to a nucleic acid encoding a 45.6 kD *Ornithobacterium rhinotracheale* protein Or11 or a part of said nucleic acid that encodes an immunogenic fragment of said protein wherein said nucleic acid or said part thereof has at least 80 % homology with the nucleic acid of the *Ornithobacterium rhinotracheale* protein gene as depicted in SEQ ID NO: 9.

Preferably, a nucleic acid according to the invention encoding this 45.6 kD *Ornithobacterium rhinotracheale* protein or a part of that nucleic acid that encodes an immunogenic fragment of that protein has at least 85 %, preferably 90 %, more preferably 95 % homology with the nucleic acid of the *Ornithobacterium rhinotracheale* protein gene as depicted in SEQ ID NO: 9.

Even more preferred is a homology level of 98 %, 99 % or even 100 %.

Again another embodiment relates to a nucleic acid encoding a 42.2 kD *Ornithobacterium rhinotracheale* protein Or77 or a part of said nucleic acid that encodes an immunogenic fragment of said protein wherein said nucleic acid or said part thereof has at least 80 % homology with the nucleic acid of the *Ornithobacterium rhinotracheale* protein gene as depicted in SEQ ID NO: 11.

Preferably, a nucleic acid according to the invention encoding this 42.2 kD *Ornithobacterium rhinotracheale* protein or a part of that nucleic acid that encodes an immunogenic fragment of that protein has at least 85 %, preferably 90 %, more preferably 95 % homology with the nucleic acid of the *Ornithobacterium rhinotracheale* protein gene as depicted in SEQ ID NO: 11.

Even more preferred is a homology level of 98 %, 99 % or even 100 %.

Also another embodiment relates to a nucleic acid encoding a 34.0 kD *Ornithobacterium rhinotracheale* protein Or98A or a part of said nucleic acid that encodes an immunogenic fragment of said protein wherein said nucleic acid or said part thereof has at least 80 % homology with the nucleic acid of the *Ornithobacterium rhinotracheale* protein gene as depicted in SEQ ID NO: 13.

Preferably, a nucleic acid according to the invention encoding this 34.0 kD *Ornithobacterium rhinotracheale* protein or a part of that nucleic acid that encodes an immunogenic fragment of that protein has at least 85 %, preferably 90 %, more preferably 95 % homology with the nucleic acid of the *Ornithobacterium rhinotracheale* protein gene as depicted in SEQ ID NO: 13.

Even more preferred is a homology level of 98 %, 99 % or even 100 %.

Another embodiment relates to a nucleic acid encoding a 32.9 kD *Ornithobacterium rhinotracheale* protein Or98B or a part of said nucleic acid that encodes an immunogenic fragment of said protein wherein said nucleic acid or said part thereof has at least 80 % homology with the nucleic acid of the *Ornithobacterium rhinotracheale* protein gene as depicted in SEQ ID NO: 15.

Preferably, a nucleic acid according to the invention encoding this 32.9 kD *Ornithobacterium rhinotracheale* protein or a part of that nucleic acid that encodes an immunogenic fragment of that protein has at least 85 %, preferably 90 %, more preferably 95 % homology with the nucleic acid of the *Ornithobacterium rhinotracheale* protein gene as depicted in SEQ ID NO: 15.

Even more preferred is a homology level of 98 %, 99 % or even 100 %.

Nucleotide sequences that are complementary to the sequence depicted in SEQ ID NO 1, 3,5,7,9,11,13 or 15 or nucleotide sequences that comprise tandem arrays of the sequences according to the invention are also within the scope of the invention.

Since the present invention discloses nucleic acids encoding 8 novel *Ornithobacterium rhinotracheale* proteins, it is now for the first time possible to obtain these proteins in significant quantities. This can e.g. be done by using expression systems to express the whole or parts of a gene encoding the protein or an immunogenic fragment thereof.

Therefore, in a preferred form of this embodiment, the invention relates to DNA fragments comprising a nucleic acid according to the invention. A DNA fragment is a stretch of nucleotides that functions as a carrier for a nucleic acid according to the invention. Such DNA fragments can e.g. be plasmids, into which a nucleic acid according to the invention is cloned. Such DNA fragments are e.g. useful for enhancing the amount of DNA for use as a primer and for expression of a nucleic acid according to the invention, as described below.

An essential requirement for the expression of the nucleic acid is an adequate promoter functionally linked to the nucleic acid, so that the nucleic acid is under the control of the promoter. It is obvious to those skilled in the art that the choice of a promoter extends to any eukaryotic, prokaryotic or viral promoter capable of directing gene transcription in cells used as host cells for protein expression.

Therefore, a more preferred form of this embodiment relates to a recombinant DNA molecule comprising a DNA fragment and/or a nucleic acid according to the invention wherein the nucleic acid according to the invention is placed under the control of a functionally linked promoter. This can be obtained by means of e.g. standard molecular biology techniques.

(Maniatis/Sambrook (Sambrook, J. Molecular cloning: a laboratory manual, 1989. ISBN 0-87969-309-6). Functionally linked promoters are promoters that are capable of controlling the transcription of the nucleic acids to which they are linked.

Such a promoter can be the native promoter of the novel gene, i.e. the promoter that is involved in the transcription of the nucleic acid encoding a protein according to the invention, or another promoter of *Ornithobacterium rhinotracheale,* provided that that promoter is functional in the cell used for expression. It can also be a heterologous promoter. When the host cells are bacteria, useful expression control sequences which may be used include the Trp promoter and operator (Goeddel, et al., NucL Acids Res., 8, 4057, 1980); the lac promoter and operator (Chang, et al., Nature, 275, 615, 1978); the outer membrane protein promoter (Nakamura, K. and Inouge, M., EMBO J., 1, 771-775,1982); the bacteriophage lambda promoters and operators (Remaut, E. et al., Nucl. Acids Res., 11, 4677-4688, 1983); the $\alpha$-amylase (B. subtilis) promoter and operator, termination sequences and other expression enhancement and control sequences compatible with the selected host cell.

When the host cell is yeast, useful expression control sequences include, e.g., $\alpha$-mating factor. For insect cells the polyhedrin or p10 promoters of baculoviruses can be used (Smith, G.E. et al., Mol. Cell. Biol. 3, 2156-65, 1983). When the host cell is of vertebrate origin illustrative useful expression control sequences include the (human) cytomegalovirus immediate early promoter (Seed, B. et al., Nature 329, 840-842, 1987; Fynan, E.F. et al., PNAS 90, 11478-11482,1993; Ulmer, J.B. et aL, Science 259, 1745-1748, 1993), Rous sarcoma virus LTR (RSV, Gorman, C.M. et al., PNAS 79, 6777-6781, 1982; Fynan et al., supra; Ulmer et al., supra), the MPSV LTR (Stacey et aL, J. Virology 50, 725-732, 1984), SV40 immediate early promoter (Sprague J. et al., J. Virology 45, 773 ,1983), the SV-40 promoter (Berman, P.W. et al., Science, 222, 524-527, 1983), the metallothionein promoter (Brinster, R.L. et al., Nature 296, 39-42, 1982), the heat shock promoter (Voellmy et al., Proc. Natl. Acad. Sci. USA, 82, 4949-53, 1985), the major late promoter of Ad2 and the $\beta$-actin promoter (Tang et al., Nature 356 152-154, 1992). The regulatory sequences may also include terminator and poly-adenylation sequences. Amongst the sequences that can be used are the well known bovine growth hormone poly-adenylation sequence, the SV40 poly-adenylation sequence, the human cytomegalovirus (hCMV) terminator and poly-adenylation sequences.

Bacterial, yeast, fungal, insect and vertebrate cell expression systems are very frequently used systems. Such systems are well-known in the art and generally available, e.g. commercially through Clontech Laboratories, Inc. 4030 Fabian Way, Palo Alto, California 94303-4607, USA. Next to these expression systems, parasite-based expression systems are attractive expression systems. Such systems are e.g. described in the French Patent Application with Publication number 2 714 074, and in US NTIS Publication No US 08/043109 (Hoffman, S. and Rogers, W.: Public. Date 1 December 1993).

An even more preferred form of this embodiment of the invention relates to Live Recombinant Carriers (LRCs) comprising a nucleic acid encoding an *Ornithobacterium rhinotracheale* protein or an immunogenic fragment thereof according to the invention, a DNA fragment according to the invention or a recombinant DNA molecule according to the invention. These LRCs are micro-organisms or viruses in which additional genetic information, in this case a nucleic acid encoding an *Ornithobacterium rhinotracheale* protein or an immunogenic fragment thereof, a DNA fragment or a recombinant DNA molecule according to the invention has been cloned. Chickens infected with such LRCs will produce an immunological response not only against the immunogens of the carrier, but also against the immunogenic parts of the protein(s) for which the genetic code is additionally cloned into the LRC, e.g. an *Ornithobacterium rhinotracheale* protein gene according to the invention.

As an example of bacterial LRCs, attenuated Salmonella strains known in the art can very attractively be used. Also, live recombinant carrier parasites have i.a. been described by Vermeulen, A. N. (Int. Journ. Parasitol. 28: 1121-1130 (1998)).

Furthermore, LRC viruses may be used as a way of transporting the nucleic acid into a target cell. Live recombinant carrier viruses are also called vector viruses. Viruses often used as vectors are Vaccinia viruses (Panicali et al; Proc. Natl. Acad. Sci. USA, 79: 4927 (1982), Herpesviruses (E.P.A. 0473210A2), and Retroviruses (Valerio, D. et al; in Baum, S.J., Dicke, K.A., Lotzova, E. and Pluznik, D.H. (Eds.), Experimental Haematology today - 1988. Springer Verlag, New York: pp. 92-99 (1989)).

Viruses known and used in the art as very suitable vector viruses specifically in poultry are Fowlpox virus, Marek's serotype 3 virus, Herpes virus of Turkey, Semliki Forest virus and Newcastle Disease virus.

Live Recombinant Carriers are also known in the art as "live vectors", or shortly "vectors". Vaccines based upon a Live Recombinant Carrier are therefore also known in the art as vector vaccines.

The technique of *in vivo* homologous recombination, well-known in the art, can be used to introduce a recombinant nucleic acid into the genome of a bacterium, parasite or virus of choice, capable of inducing expression of the inserted nucleic acid according to the invention in the host animal.

Finally another form of this embodiment of the invention relates to a host cell comprising a nucleic acid encoding a protein according to the invention, a DNA fragment comprising such a nucleic acid or a recombinant DNA molecule comprising such a nucleic acid under the control of a functionally linked promoter. This form also relates to a host cell containing a live recombinant carrier comprising a nucleic acid molecule encoding an *Ornithobacterium rhinotracheale* protein or an immunogenic fragment thereof according to the invention.

A host cell may be a cell of bacterial origin, e.g. *Escherichia coli, Bacillus subtilis* and *Lactobacillus* species, in combination with bacteria-based plasmids as pBR322, or bacterial expression vectors as pGEX, or with bacteriophages. The host cell may also be of eukaryotic origin, e.g. yeast-cells in combination with yeast-specific vector molecules, or higher eukaryotic cells like insect cells (Luckow et al; Bio-technology 6: 47-55 (1988)) in combination with vectors or recombinant baculoviruses, plant cells in combination with e.g. Ti-plasmid based vectors or plant viral vectors (Barton, K.A. et al; Cell 32: 1033 (1983), mammalian cells like Hela cells, Chinese Hamster Ovary cells (CHO) or Crandell Feline Kidney-cells, also with appropriate vectors or recombinant viruses.

Another embodiment of the invention relates to an *Ornithobacterium rhinotracheale* protein and to immunogenic fragments thereof according to the invention.

The concept of immunogenic fragments will be defined below.

One form of this embodiment relates to a 59.8 kD *Ornithobacterium rhinotracheale* protein and to immunogenic fragments thereof, having an amino acid sequence homology of at least 80 % with the amino acid sequence as depicted in SEQ ID NO: 2.

In a preferred form, the embodiment relates to such *Ornithobacterium rhinotracheale* proteins and immunogenic fragments thereof, that have a sequence homology of at least 85 %, preferably 90 %, more preferably 95 % homology to the amino acid sequence as depicted in SEQ ID NO: 2.

Even more preferred is a homology level of 98 %, 99 % or even 100 %.

The level of protein homology can be determined with the computer program "BLAST 2 SEQUENCES" by selecting sub-program: "BLASTP", that can be found at www.ncbi.nlm.nih.gov/blast/bl2seq/bl2.html.

A reference for this program is Tatiana A. Tatusova, Thomas L. Madden FEMS Microbiol. Letters 174: 247-250 (1999). Matrix used: "blosum62". Parameters used are the default parameters:

Open gap: 11. Extension gap: 1. Gap x_dropoff: 50.

Another form of this embodiment relates to a 58.2 kD *Ornithobacterium rhinotracheale* protein and to immunogenic fragments thereof, having an amino acid sequence homology of at least 80 % with the amino acid sequence as depicted in SEQ ID NO: 4.

In a preferred form, the embodiment relates to such *Ornithobacterium rhinotracheale* proteins and immunogenic fragments thereof, that have a sequence homology of at least 85 %, preferably 90 %, more preferably 95 % homology to the amino acid sequence as depicted in SEQ ID NO: 4.

Even more preferred is a homology level of 98 %, 99 % or even 100 %.

Still another form of this embodiment relates to a 46.0 kD *Ornithobacterium rhinotracheale* protein and to immunogenic fragments thereof, having an amino acid sequence homology of at least 80 % with the amino acid sequence as depicted in SEQ ID NO: 6.

In a preferred form, the embodiment relates to such *Ornithobacterium rhinotracheale* proteins and immunogenic fragments thereof, that have a sequence homology of at least 85 %, preferably 90 %, more preferably 95 % homology to the amino acid sequence as depicted in SEQ ID NO: 6.

Even more preferred is a homology level of 98 %, 99 % or even 100 %.

Again another form of this embodiment relates to a 37.2 kD *Ornithobacterium rhinotracheale* protein and to immunogenic fragments thereof, having an amino acid sequence homology of at least 80 % with the amino acid sequence as depicted in SEQ ID NO: 8.

In a preferred form, the embodiment relates to such *Ornithobacterium rhinotracheale* proteins and immunogenic fragments thereof, that have a sequence homology of at least 85 %, preferably 90 %, more preferably 95 % homology to the amino acid sequence as depicted in SEQ ID NO: 8.

Even more preferred is a homology level of 98 %, 99 % or even 100 %.

Still another form of this embodiment relates to a 45.6 kD *Ornithobacterium rhinotracheale* protein and to immunogenic fragments thereof, having an amino acid sequence homology of at least 80 % with the amino acid sequence as depicted in SEQ ID NO: 10.

In a preferred form, the embodiment relates to such *Ornithobacterium rhinotracheale* proteins and immunogenic fragments thereof, that have a sequence homology of at least 85 %, preferably 90 %, more preferably 95 % homology to the amino acid sequence as depicted in SEQ ID NO: 10.

Even more preferred is a homology level of 98 %, 99 % or even 100 %.

One other form of this embodiment relates to a 42.2 kD *Ornithobacterium rhinotracheale* protein and to immunogenic fragments thereof, having an amino acid sequence homology of at least 80 % with the amino acid sequence as depicted in SEQ ID NO: 12.

In a preferred form, the embodiment relates to such *Ornithobacterium rhinotracheale* proteins and immunogenic fragments thereof, that have a sequence homology of at least 85 %, preferably 90 %, more preferably 95 % homology to the amino acid sequence as depicted in SEQ ID NO: 12.

Even more preferred is a homology level of 98 %, 99 % or even 100 %.

And again another form of this embodiment relates to a 34.0 kD *Ornithobacterium rhinotracheale* protein and to immunogenic fragments thereof, having an amino acid sequence homology of at least 80 % with the amino acid sequence as depicted in SEQ ID NO: 14.

In a preferred form, the embodiment relates to such *Ornithobacterium rhinotracheale* proteins and immunogenic fragments thereof, that have a sequence homology of at least 85 %, preferably 90 %, more preferably 95 % homology to the amino acid sequence as depicted in SEQ ID NO: 14.

Even more preferred is a homology level of 98 %, 99 % or even 100 %.

Finally another form of this embodiment relates to a 32.9 kD *Ornithobacterium rhinotracheale* protein and to immunogenic fragments thereof, having an amino acid sequence homology of at least 80 % with the amino acid sequence as depicted in SEQ ID NO: 16.

In a preferred form, the embodiment relates to such *Ornithobacterium rhinotracheale* proteins and immunogenic fragments thereof, that have a sequence homology of at least 85 %, preferably 90 %, more preferably 95 % homology to the amino acid sequence as depicted in SEQ ID NO: 16.

Even more preferred is a homology level of 98 %, 99 % or even 100 %.

Another form of this embodiment relates to such *Ornithobacterium rhinotracheale* proteins and immunogenic fragments of said proteins according to the invention, wherein the proteins and immunogenic fragments thereof are encoded by a nucleic acid according to the invention.

It will be understood that, for the particular proteins embraced herein, natural variations can exist between individual *Ornithobacterium rhinotracheale* strains. These variations may be demonstrated by (an) amino acid difference (s) in the overall sequence or by deletions, substitutions, insertions, inversions or additions of (an) amino acid(s) in said sequence. Amino acid substitutions which do not essentially alter biological and immunological activities, have been described, e.g. by Neurath et al in "The Proteins" Academic Press New York (1979). Amino acid replacements between related amino acids or replacements which have occurred frequently in evolution are, inter alia, Ser/Ala, Ser/Gly, Asp/Gly, Asp/Asn, Ile/Val (see Dayhof, M.D., Atlas of protein sequence and structure, Nat. Biomed. Res. Found., Washington D.C., 1978, vol. 5, suppl. 3). Other amino acid substitutions include Asp/Glu, Thr/Ser, Ala/Gly, Ala/Thr, Ser/Asn, Ala/Val, Thr/Phe, Ala/Pro, Lys/Arg, Leu/Ile, Leu/Val and Ala/Glu. Based on this information, Lipman and Pearson developed a method for rapid and sensitive protein comparison (Science,227, 1435-1441, 1985) and determining the functional similarity between homologous proteins. Such amino acid substitutions of the exemplary embodiments of this invention, as well as variations having deletions and/or insertions are within the scope of the invention as long as the resulting proteins retain their immune reactivity.

This explains why *Ornithobacterium rhinotracheale* proteins according to the invention, when isolated from different field isolates, may have homology levels as low as about 80%, while still representing the same protein with the same immunological characteristics.

Those variations in the amino acid sequence of a certain protein according to the invention that still provide a protein capable of inducing an immune response against infection with *Ornithobacterium rhinotracheale* or at least against the clinical manifestations of the infection are considered as "not essentially influencing the immunogenicity".

When a protein is used for e.g. vaccination purposes or for raising antibodies, it is however not necessary to use the whole protein. It is also possible to use a fragment of that protein that is capable, as such or coupled to a carrier such as e.g. KLH, of inducing an immune response against that protein, a so-called immunogenic fragment. An "immunogenic fragment" is understood to be a fragment of the full-length protein that still has retained its capability to induce an immune response in a vertebrate host, e.g. comprises a B- or T-cell epitope. Shortly, an immunogenic fragment is a fragment that is capable of inducing an antigenic response against an *Ornithobacterium rhinotracheale* protein according to the invention. At this moment, a variety of techniques is available to easily identify DNA fragments encoding antigenic fragments (determinants). The method described by Geysen et al (Patent Application WO 84/03564, Patent Application WO 86/06487, US Patent NR. 4,833,092, Proc. Natl Acad. Sci. 81: 3998-4002 (1984), J. Imm. Meth. 102,259-274 (1987), the so-called PEPSCAN method is an easy to perform, quick and well-established method for the detection of epitopes;

the immunologically important regions of the protein. The method is used worldwide and as such well-known to man skilled in the art. This (empirical) method is especially suitable for the detection of B-cell epitopes. Also, given the sequence of the gene encoding any protein, computer algorithms are able to designate specific protein fragments as the immunologically important epitopes on the basis of their sequential and/or structural agreement with epitopes that are now known. The determination of these regions is based on a combination of the hydrophilicity criteria according to Hopp and Woods (Proc. Natl. Acad. Sci. 78: 38248-3828 (1981)), and the secondary structure aspects according to Chou and Fasman (Advances in Enzymology 47: 45-148 (1987) and US Patent 4,554,101). T-cell epitopes can likewise be predicted from the sequence by computer with the aid of Berzofsky's amphiphilicity criterion (Science 235, 1059-1062 (1987) and US Patent application NTIS US 07/005,885). A condensed overview is found in: Shan Lu on common principles: Tibtech 9: 238-242 (1991), Good et al on Malaria epitopes; Science 235: 1059-1062 (1987), Lu for a review; Vaccine 10: 3-7 (1992), Berzofsky for HIV-epitopes; The FASEB Journal 5:2412-2418 (1991). An immunogenic fragment usually has a minimal length of 8 amino acids, preferably more then 8, such as 9, 10, 12, 15 or even 20 amino acids. The nucleic acids encoding such a fragment therefore have a length of at least 24, but preferably 27, 30, 36, 45 or even 60 nucleic acids.

Therefore, one form of still another embodiment of the invention relates to vaccines for combating *Ornithobacterium rhinotracheale* infection, that comprise an *Ornithobacterium rhinotracheale* protein or immunogenic fragments thereof, according to the invention as described above together with a pharmaceutically acceptable carrier.

Still another embodiment of the present invention relates to an *Ornithobacterium rhinotracheale* protein according to the invention or immunogenic fragments thereof for use in a vaccine.

Still another embodiment of the present invention relates to the use of a nucleic acid, a DNA fragment, a recombinant DNA molecule, a live recombinant carrier, a host cell or a protein or an immunogenic fragment thereof according to the invention for the manufacturing of a vaccine for combating *Ornithobacterium rhinotracheale* infection.

One way of making a vaccine according to the invention is by growing the bacteria, followed by biochemical purification of an *Ornithobacterium rhinotracheale* protein or an immunogenic fragment thereof, from the bacterium. This is however a very time-consuming way of making the vaccine.

It is therefore much more convenient to use the expression products of the gene encoding an *Ornithobacterium rhinotracheale* protein or immunogenic fragments thereof in vaccines. This is possible for the first time now because the nucleic acids encoding the *Ornithobacterium rhinotracheale* proteins are provided in the present invention.

Vaccines based upon the expression products of these genes can easily be made by admixing the protein according to the invention or immunogenic fragments thereof according to the invention with a pharmaceutically acceptable carrier as described below.

Alternatively, a vaccine according to the invention can comprise live recombinant carriers as described above, capable of expressing the protein according to the invention or immunogenic fragments thereof. Such vaccines, e.g. based upon a *Salmonella* carrier or a viral carrier e.g. a Herpesvirus vector have the advantage over subunit vaccines that they better mimic the natural way of infection of *Ornithobacterium rhinotracheale.* Moreover, their self-propagation is an advantage since only low amounts of the recombinant carrier are necessary for immunization.

Vaccines can also be based upon host cells as described above, that comprise the protein or immunogenic fragments thereof according to the invention.

All vaccines described above contribute to active vaccination, i.e. they trigger the host's defense system.

Alternatively, antibodies can be raised in e.g. rabbits or can be obtained from antibody-producing cell lines as described below. Such antibodies can then be administered to the chicken. This method of vaccination, passive vaccination, is the vaccination of choice when an animal is already infected, and there is no time to allow the natural immune response to be triggered. It is also the preferred method for vaccinating animals that are prone to sudden high infection pressure. The administered antibodies against the protein according to the invention or immunogenic fragments thereof can in these cases bind directly to *Ornithobacterium rhinotracheale.* This has the advantage that it decreases or stops *Ornithobacterium rhinotracheale* multiplication.

Therefore, one other form of this embodiment of the invention relates to a vaccine for combating *Ornithobacterium rhinotracheale* infection that comprises antibodies against a *Ornithobacterium rhinotracheale* protein according to the invention or an immunogenic fragment of that protein, and a pharmaceutically acceptable carrier.

Still another embodiment of this invention relates to antibodies against a *Ornithobacterium rhinotracheale* protein according to the invention or an immunogenic fragment of that protein.

Methods for large-scale production of antibodies according to the invention are also known in the art. Such methods rely on the cloning of (fragments of) the genetic information encoding the protein according to the invention in a filamentous phage for phage display. Such techniques are described i.a. at the "Antibody Engineering Page" under "filamentous phage display" at bttp:aximt1.imt.uni-marburg.de/~rek/aepphage.html., and in review papers by Cortese, R. et al., (1994) in Trends Biotechn. 12: 262 267., by Clackson, T. & Wells, J.A. (1994) in Trends Biotechn. 12: 173-183, by Marks, J.D. et al., (1992) in J. Biol. Chem. 267: 16007-16010, by Winter, G. et aL, (1994) in Annu. Rev. Immunol. 12: 433-455, and by Little, M. et al., (1994) Biotechn. Adv. 12: 539-555. The phages are subsequently used to screen camelid expression libraries expressing camelid heavy chain antibodies. (Muyldermans, S. and Lauwereys, M., Journ. Molec. Recogn. 12: 131-140 (1999) and Ghahroudi, M.A. et al., FEBS Letters 414: 512-526 (1997)). Cells from the library that express the desired antibodies can be replicated and subsequently be used for large scale expression of antibodies.

Still another embodiment relates to a method for the preparation of a vaccine according to the invention that comprises the admixing of antibodies according to the invention and a pharmaceutically acceptable carrier.

An alternative and efficient way of vaccination is direct vaccination with DNA encoding the relevant antigen. Direct vaccination with DNA encoding proteins has been successful for many different proteins. (As reviewed in e.g. Donnelly et al., The Immunologist 2: 20-26 (1993)). This way of vaccination is also attractive for the vaccination of chickens against *Ornithobacterium rhinotracheale* infection.

Therefore, still other forms of this embodiment of the invention relate to vaccines comprising nucleic acids encoding a protein according to the invention or immunogenic fragments thereof, comprising DNA fragments that comprise such nucleic acids or comprising recombinant DNA molecules according to the invention, and a pharmaceutically acceptable carrier.

Examples of DNA plasmids that are suitable for use in a DNA vaccine according to the invention are conventional cloning or expression plasmids for bacterial, eukaryotic and yeast host cells, many of said plasmids being commercially available. Well-known examples of such plasmids are pBR322 and pcDNA3 (Invitrogen). The DNA fragments or recombinant DNA molecules according to the invention should be able to induce protein expression of the nucleotide sequences. The DNA fragments or recombinant DNA molecules may comprise one or more nucleotide sequences according to the invention. In addition, the DNA fragments or recombinant DNA molecules may comprise other nucleotide sequences such as the immune-stimulating oligonucleotides having unmethylated CpG di-nucleotides, or nucleotide sequences that code for other antigenic proteins or adjuvating cytokines.

The nucleotide sequence according to the present invention or the DNA plasmid comprising a nucleotide sequence according to the present invention, preferably operably linked to a transcriptional regulatory sequence, to be used in the vaccine according to the invention can be naked or can be packaged in a delivery system. Suitable delivery systems are lipid vesicles, iscoms, dendromers, niosomes, polysaccharide matrices and the like, (see further below) all well-known in the art. Also very suitable as delivery system are attenuated live bacteria such as Salmonella species, and attenuated live viruses such as Herpesvirus vectors, as mentioned above.

DNA vaccines can e.g. easily be administered through intradermal application such as by using a needle-less injector. This way of administration delivers the DNA directly into the cells of the animal to be vaccinated. Amounts of DNA in the range between 10 pg and 1000 $\mu$g provide good results. Preferably, amounts in the microgram range between 1 and 100 $\mu$g are used.

In a further embodiment, the vaccine according to the present invention comprises one or more additional antigens derived from a virus or micro-organism pathogenic to poultry, an antibody against such an antigen or genetic information encoding said antigen.

Of course, such antigens can be e.g. other *Ornithobacterium rhinotracheale* antigens. It is beneficial to combine, in one vaccine, two or more of the proteins or immunogenic fragments thereof according to the invention, antibodies against such proteins or immunogenic fragments thereof, or genetic information encoding such proteins or immunogenic fragments thereof.

Next to this, it is beneficial to include in a vaccine according to the invention, antigens derived from another micro-organism or a virus pathogenic to poultry, an antibody against such an antigen or genetic information encoding said antigen.

Preferably, the virus or micro-organism is selected from the group consisting of Fowlpox virus, Infectious Bronchitis virus, Infectious Bursal Disease (Gumboro), Marek's Disease Virus, Chicken Anaemia agent, Avian Reovirus, *Mycoplasma gallisepticum,* Turkey Rhinotracheitis virus, *Haemophilus paragallinarum* (Coryza), Chicken Poxvirus, Avian Encephalomyelitisvirus, Duck Plague virus, Newcastle Disease virus, Egg Drop syndrome virus, Infectious Laryngotracheitis virus, Herpes Virus of Turkeys, Eimeria species, *Ornithobacterium rhinotracheale, Pasteurella multocida, Mycoplasma synoviae, Salmonella* species and *E. coli.*

Vaccines based upon the *Ornithobacterium rhinotracheale* proteins according to the invention are also very suitable as marker vaccines. A marker vaccine is a vaccine that allows to discriminate between vaccinated and field-infected chickens e.g. on the basis of a characteristic antibody panel, different from the antibody panel induced by wild type infection. A different antibody panel is induced e.g. when an immunogenic protein present on a wild type bacterium is not present in a vaccine: the host will then not make antibodies against that protein after vaccination. Thus, a vaccine based upon an *Ornithobacterium rhinotracheale* protein according to the invention would only induce antibodies against that protein, whereas a vaccine based upon a live wild-type, live attenuated or inactivated whole *Ornithobacterium rhinotracheale* would induce antibodies against all or most of the bacterial proteins.

A simple ELISA test, having wells comprising one protein according to the invention and wells comprising another protein according to the invention suffices to test serum from chickens and to tell if the chickens are either vaccinated with a subunit vaccine according to the invention or suffered from *Ornithobacterium rhinotracheale* field infection; chickens vaccinated with a vaccine comprising one protein according to the invention would not have antibodies against another protein according to the invention. Chickens that have encountered a field infection with *Ornithobacterium rhinotracheale* would however have antibodies against all immunogenic *Ornithobacterium rhinotracheale* proteins and thus also against another protein according to the invention.

All vaccines according to the present invention comprise a pharmaceutically acceptable carrier. A pharmaceutically acceptable carrier can be e.g. sterile water or a sterile physiological salt solution. In a more complex form the carrier can e.g. be a buffer.

Methods for the preparation of a vaccine comprise the admixing of a protein or an immunogenic fragment thereof, according to the invention and/or antibodies against that protein or an immunogenic fragment thereof, and/or a nucleic acid and/or a DNA fragment, a recombinant DNA molecule, a live recombinant carrier or host cell according to the invention, and a pharmaceutically acceptable carrier.

Vaccines according to the present invention may in a preferred presentation also contain an immunostimulatory substance, a so-called adjuvant. Adjuvants in general comprise substances that boost the immune response of the host in a non-specific manner. A number of different adjuvants are known in the art. Examples of adjuvants frequently used in chicken vaccines are muramyldipeptides, lipopolysaccharides, several glucans and glycans and Carbopol(R) (a homopolymer).

The vaccine may also comprise a so-called "vehicle". A vehicle is a compound to which the protein adheres, without being covalently bound to it. Such vehicles are i.a. bio-microcapsules, micro-alginates, liposomes and macrosols, all known in the art.

A special form of such a vehicle, in which the antigen is partially embedded in the vehicle, is the so-called ISCOM (EP 109.942, EP 180.564, EP 242.380)

In addition, the vaccine may comprise one or more suitable surface-active compounds or emulsifiers, e.g. Span or Tween.

Often, the vaccine is mixed with stabilisers, e.g. to protect degradation-prone proteins from being degraded, to enhance the shelf-life of the vaccine, or to improve freeze-drying efficiency. Useful stabilisers are i.a. SPGA (Bovarnik et al; J. Bacteriology 59: 509 (1950)), carbohydrates e.g. sorbitol, mannitol, trehalose, starch, sucrose, dextran or glucose, proteins such as albumin or casein or degradation products thereof, and buffers, such as alkali metal phosphates.

In addition, the vaccine may be suspended in a physiologically acceptable diluent.

It goes without saying, that other ways of adjuvating, adding vehicle compounds or diluents, emulsifying or stabilising a protein are also embodied in the present invention.

Vaccines according to the invention that are based upon the protein according to the invention or immunogenic fragments thereof can very suitably be administered in amounts ranging between 1 and 100 micrograms of protein per animal, although smaller doses can in principle be used. A dose exceeding 100 micrograms will, although immunologically very suitable, be less attractive for commercial reasons.

Vaccines based upon live attenuated recombinant carrier, such as the LRC-viruses and bacteria described above can be administered in much lower doses, because they multiply themselves during the infection. Therefore, very suitable amounts would range between $10^3$ and $10^9$ CFU/PFU for respectively bacteria/viruses.

Vaccines according to the invention can be administered e.g. intradermally, subcutaneously, intramuscularly, intraperitoneally, intravenously, or at mucosal surfaces such as orally or intranasally.

Live recombinant carrier vaccines or vector vaccines can most efficiently be administered by spraying, by aerosol or by drinking water administration.

Examples.

Example 1: Library construction, sera and screening.

For the construction of an expression library of *Ornithobacterium rhinotracheale* serotype G strain O-95029 nr.16279, genomic DNA was isolated from cells grown in Todd Hewitt broth (THB) for 24 hours at 37°C on a 100 rpm shaker, according to the method described in Maniatis/Sambrook (Sambrook, J. et al. Molecular cloning: a laboratory manual. ISBN 0-87969-309-6). DNA fragments of 1 - 4 kb were obtained by restriction enzyme digestion and ligated into λTriplEx vector arms (Clontech, Palo Alto, CA, USA). Subsequent packaging was performed using the Stratagene (La Jolla, CA, USA) *in vitro* packaging extract. *Escherichia coli* XL1 Blue cells, grown in Luria Bertani (LB) broth supplemented with 10 mM $MgSO_4$ and 0.2% maltose, were used for transfection. The complexity of the constructed expression library was tested 6.9 and it contained 97% recombinants.

The *Ornithobacterium rhinotracheale* serotype G expression library was screened with polyclonal antisera directed against whole live organisms of several *Ornithobacterium rhinotracheale* serotypes. Sera were collected from broiler chickens that were vaccinated by aerosol spraying with live *Ornithobacterium rhinotracheale* bacteria of serotype B (strain GGD 1261), serotype G (strain O-95029 nr.16279) or serotype M (strain TOP 98036 4500) at two weeks of age. Three weeks later the chickens were intravenously challenged with *Ornithobacterium rhinotracheale* serotype A (strain B3263/91). Sera were collected one week after challenge. All vaccinated birds showed reduced pathology (ranging from 10% to 60%) in comparison to unvaccinated control birds. Before use in expression library screening, the antisera were adsorbed with *Escherichia coli* XL1 Blue cell lysate as described in Maniatis/Sambrook (Sambrook, J. *et al*. Molecular cloning: a laboratory manual. ISBN 0-87969-309-6) in order to reduce a-specific background signal.

The expression library was screened by plaque lift using an initial screening of approximately 20.000 plaques. The procedure was done as described in the manufacturers handbook (Clontech, Palo Alto, CA, USA). All library screenings were done under native conditions. In short, phage-infected *Escherichia coli* XL1 Blue cells were plated in LB top agar onto LB agar plates both supplemented with 10 mM MgSO$_4$. The plates were then incubated at 42°C for 4 hours. A nitrocellulose filter disc (Schleicher and Schuell, Dassel, Germany), previously soaked in 10 mM IPTG, was placed on each plate in order to induce expression of the proteins encoded by the cloned *Ornithobacterium rhinotracheale* inserts. After 4 hours incubation at 37°C all filters were removed from the plates. After washing and blocking, filters were incubated with chicken antiserum (pooled from 10 animals, 1:250 dilution). The antiserum used in the first screening was obtained from chickens live vaccinated with *Ornithobacterium rhinotracheale* serotype G followed by a challenge with *Ornithobacterium rhinotracheale* serotype A. As secondary antibody rabbit anti-chicken IgG peroxidase (Nordic, Tilburg, The Netherlands) was use at 1:1000 dilution. As substrate solution Vector SG (Vector, Burlingame, CA, USA) was used. From the initial screening of 20.000 plaques, 200 reactive plaques were located on the agar plates and isolated. A plaque lift and screen as described above was repeated twice resulting in 175 single, pure reactive plaques. The pure clones were then spotted *in duplo* onto an *E.coli* XL1 Blue top agar lawn to give confluent plaques of approximately 5 mm diameter. Again a plaque lift was performed and the filters were incubated with the antisera obtained from birds live vaccinated with *Ornithobacterium rhinotracheale* serotype B or serotype M prior to *Ornithobacterium rhinotracheale* serotype A challenge. Out of 175 reactive plaques, 30 plaques were selected to be cross-reactive with sera from birds live vaccinated with *Ornithobacterium rhinotracheale* serotype B, serotype G, or serotype M, and challenged with *Ornithobacterium rhinotracheale* serotype A.

Example 2: Identification of open reading frames (ORFs) encoding antigenic proteins and expression in *Escherichia coli.*

The DNA inserts of the 30 selected plaques were analysed in order to identify the open reading frames encoding the antigenic proteins. Oligonucleotide primers designed for the λ TriplEx vector arms were used for both PCR amplification and sequencing. PCR was performed in a final reaction volume of 50 μl containing 50 μM dNTP's (Promega, WI, USA), 10 pmol of both primers, 20 U/ml Supertaq plus polymerase and 10X Supertaq buffer (both HT Biotechnology Ltd, Cambridge, UK) in water. Phage DNA was added by picking a freshly plated plaque using a tooth pick, and transferring this DNA from tooth pick to reaction mix. The following conditions were used: denaturation at 94°C for 3 min, followed by 30 cycles of denaturation at 94°C for 1 min, annealing at 50°C for 2 min and elongation at 68°C for 2 min 30 sec, followed by a final extension at 68°C for 10 min. To determine the nucleotide sequence of the amplified DNA inserts a sequence reaction was done (94°C 10 sec; 50°C 5 sec; 60°C 2 min for 25 cycles) using Big dye Terminator Ready reaction mix (Qiagen Inc., CA, USA), 50 ng template DNA (PCR product) and 2.4 pmol primer in a 20μl reaction volume.

After sequence analysis the 30 clones appeared to represent 8 different genes. Since most open reading frames where a fusion with the lacZ gene of the λTriplEx vector, the 5'end of the gene was missing. For that reason a sequence reaction was performed using internal primers and chromosomal DNA of *Ornithobacterium rhinotracheale* serotype G as a template to sequence the missing 5'gap.

Oligonucleotide primers were designed to amplify the full length open reading frames encoding the 8 cross-reactive antigens (Or01, Or02, Or03, Or04, Or11, Or77, Or98A and Or98B) from genomic DNA of *Ornithobacterium rhinotracheale* serotype G strain 0-95029 nr.16279 (see table 1). The 5'oligonucleotide primers contain a restriction site (underlined) preceding the ATG initiation codon (bold) followed by sequences derived from the gene of interest (italic). The 3'oligonucleotides contain coding sequences (italic) followed by a restriction site (underlined). The PCR products were cloned in the expression vector of interest. Ligation products were transformed to *E.coli* BL21 (DE3) codon RIL pLysS host cells (Novagen, Madison, WI, USA) for protein expression. By using the pET plasmid vector (pET22b) and a T7 RNA polymerase expression system (Novagen, Madison, WI, USA), the recombinant proteins were expressed in *E.coli,* with an *E.coli pelB* leader peptide fused at the amino terminal portion *(Ornithobacterium rhinotracheale* leader peptides of proteins Or02, Or03, Or11, and Or77 were replaced) and 6 histidine residues at the carboxy terminal portion of the protein. *E.coli* strain BL21 (DE3) codon RIL pLysS (Novagen, Madison, WI, USA) was used for high level expression during IPTG-induction as described in the pET system manual (Novagen, Madison, WI, USA).

Example 3: Purification of antigens, vaccine formulations and serological analysis. Recombinant antigens expressed in *E.coli* were isolated from supernatant (Or77), purified by metal affinity chromatography using talon resin (Clontech Inc., Palo Alto, CA, USA) as described by the manufacturer (Or03, Or04, Or98A and Or98B), or by repeated freeze-thawing, sonification, and centrifugation cycli (Or01, Or02 and Or11). Polyacrylamide gel electrophoresis (PAGE) followed by Coomassie brilliant blue staining was used to assess the purity of the recombinant proteins. Protein concentrations were estimated using bovine serum albumin as the standard.

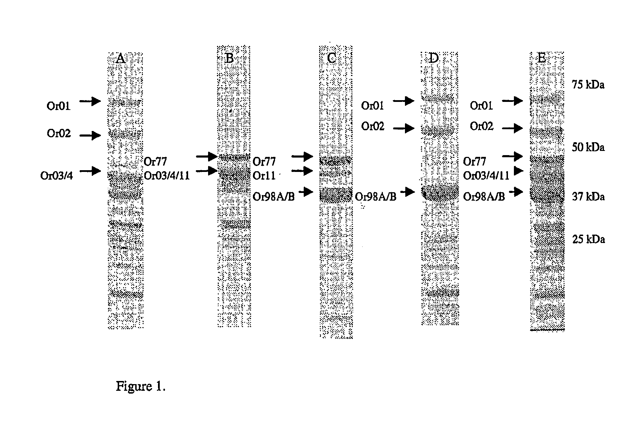

All purified recombinant proteins (Or01, Or02, Or03, Or04, Or11, Or77, Or98A and Or98B) were formulated individually in a water in oil emulsion. Furthermore, five different subunit vaccines (A, B, C, D and E) were formulated, containing different compositions of the 8 recombinant antigens (table 2). Coomassie staining of the 5 combination vaccines showed clearly identifiable protein bands corresponding to recombinant proteins Or01, Or02 and Or77. As the molecular weights of Or03, Or04 and Or11, and the molecular weights of Or98A and Or98B are approximately the same, individual protein bands could not be distinguished (figure 1). All proteins are present in approximately equal concentrations of 50 mg/antigen/l (25 $\mu$g/dose). Therefore, the total antigenic load of vaccine A to D is 200 mg/l. The antigen concentration of vaccine E is 400 mg/l. The protein background is rest material from *E.coli* strain used to express the recombinant *Ornithobacterium rhinotracheale* antigens.

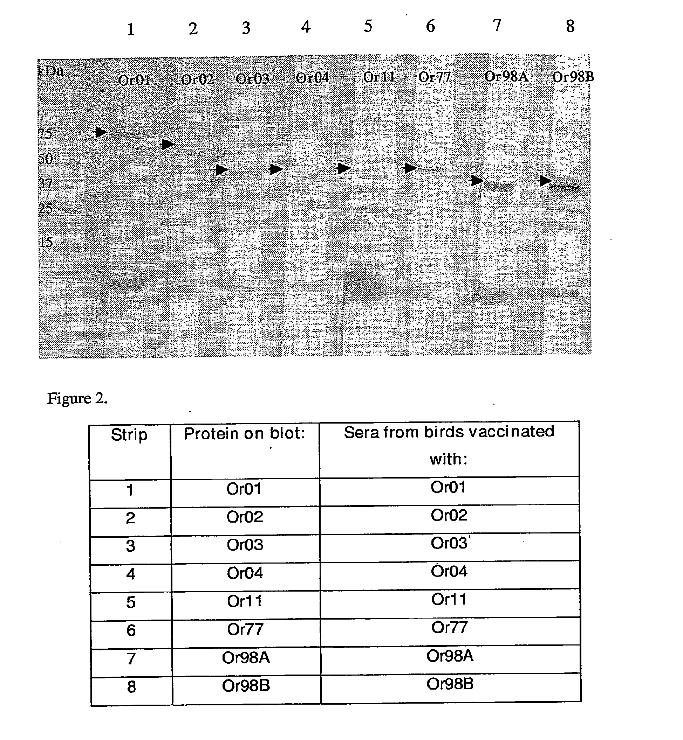

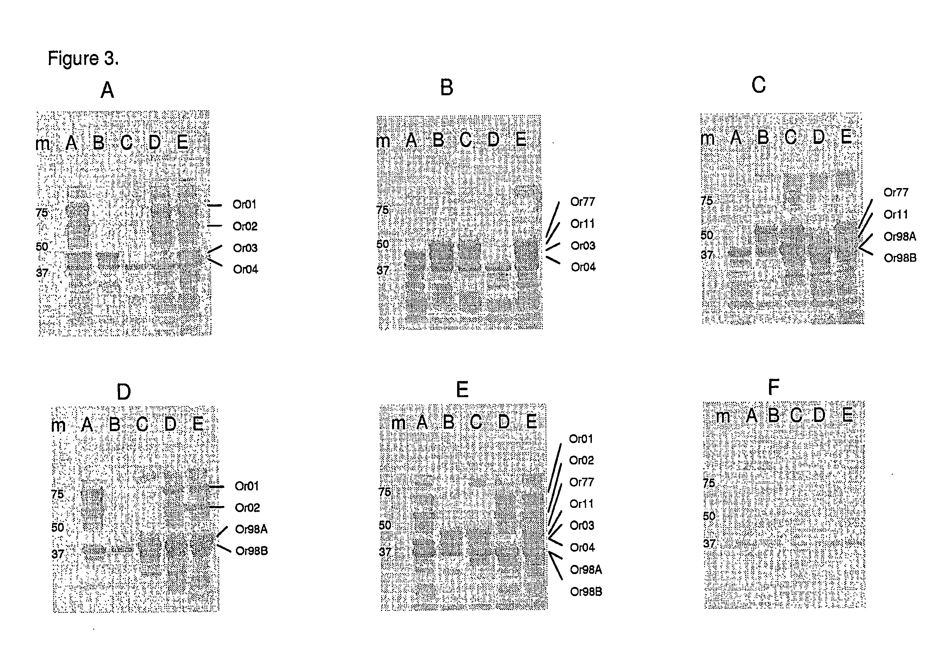

The ability of the different subunit vaccines to stimulate the humoral immune response to produce protein-specific antibodies was studied by subcutaneous injection of 2-weeks-old SPF-broiler chickens with 0.5 ml vaccine. Four weeks after vaccination serum-samples were collected and tested for the presence of antibodies reactive against the recombinant proteins. Semi-dry Western blotting was performed according to Towbin, H., Staehlin, T., and Gordon, J. (1979) Proc. Nat. Acad. Sci. 76:43-50. The protein phase of the vaccines was blotted and incubated with pooled serum (1:100 dilution) from vaccinated and unvaccinated birds. Sera obtained from birds vaccinated with each of the 8 individual vaccines Or01 to Or98B showed protein-specific reactivity (figure 2). Figure 3 shows the reactivity of antisera obtained from birds vaccinated with subunit vaccine A to E (see table 2 and figure 1), directed against the same vaccines on Western blot. For example: blot A is loaded with vaccine A, B, C, D, and E (corresponding with lanes A to E). The serum used for primary antibody binding is obtained from birds vaccinated with vaccine A (corresponds with blot-number). For this reason, $\alpha$-Or01, $\alpha$-Or02, $\alpha$-Or03 and $\alpha$-Or04 antibodies are present in this serum. On blot A, these four proteins are stained in lane A, D, and E, which are the lanes that were loaded with the three vaccines that contain these antigens (A, D, and E). Blot B is loaded as blot A and the serum used is obtained from birds vaccinated with vaccine B. $\alpha$-Or77, $\alpha$-Or11, $\alpha$-Or03, and $\alpha$-Or04 antibodies stain the corresponding antigens on blot B in lane B, C, and E. The other antigens that were not present in vaccine B could not be detected on this blot. On blot E, all proteins are stained because vaccine E contains all eight *Ornithobacterium rhinotracheale* antigens. The serum used on Westemblot F is obtained from unvaccinated birds that served as a negative control. No recombinant *Ornithobacterium rhinotracheale* antigens could be detected using this serum.

Example 4: Protection studies.

To assess the cross-protective capacity of the antibody response induced by different subunit vaccines (combi vaccines A, B, C, D, E, and individual vaccine Or77), an animal experiment was performed. SPF-broilers were vaccinated at 2 weeks of age as described before. At 5 weeks of age birds were primed with ND LaSota (dose:1*10^6 E.I.D.per bird) by aerosol spraying. At 6 weeks of age, birds were challenged with *Ornithobacterium rhinotracheale* serotype A strain B3263/91 (heterologous challenge). The challenge was done by aerosol spraying of a fresh bacterial culture containing 8.5*10^8 colony forming units (CFU) per ml THB. During aerosol challenge the bacterial culture was administered as a fine spray to the birds in an isolator of approximately 1.5m^3, using a commercial paint sprayer. The developed mist in the isolators was maintained for at least 10 min with the air circulation closed.

Challenge control groups and ND priming groups were included in the test One week after challenge, at 7 weeks of age, birds were sacrificed and organ lesions were macroscopically scored using an *Ornithobacterium rhinotracheale* scoring system for respiratory disease as follows: for thoracic air sacs, 0= no abnormalities, 1= one air sac seriously affected by fibrinous airsacculitis or limited pin-head sized foci of fibrinous exudates in both air sacs, 2= both air sacs seriously affected by fibrinous airsacculitis; for abdominal air sacs, 0= no abnormalities, 1= pin-head sized foci of fibrinous exudates or slight diffuse fibrinous airsacculitis, 2= severe fibrinous airsacculitis. The airsacculitis score is given as the sum of both scores. For lungs, 0= no abnormalities, 1= unilateral pneumonia, 2= bilateral pneumonia. The average group scores are given as a percentage of the maximum possible score. Statistical analysis was performed using Kruskal-Wallis non-parametric one-way ANOVA.

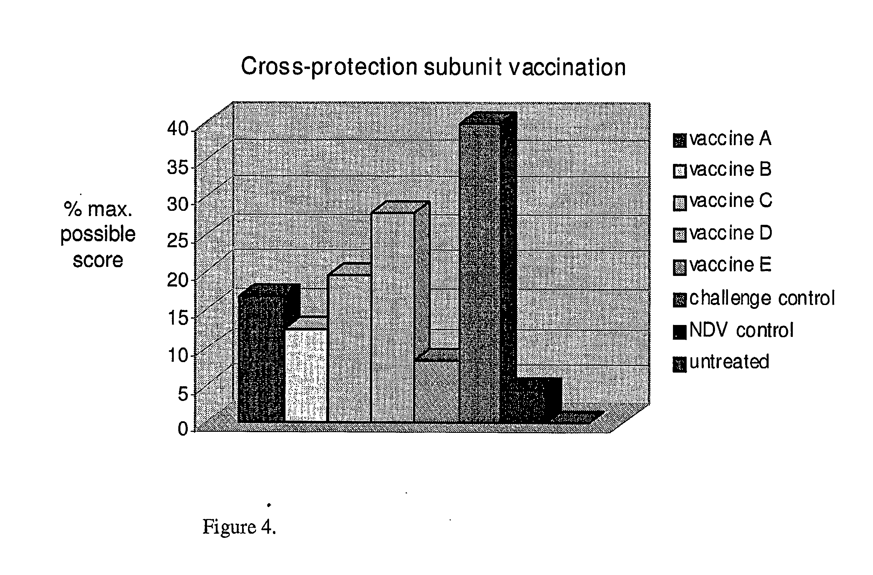

Figure 4 shows the cross-protective capacity of the 5 different subunit vaccines A to E. The challenge control group was not vaccinated but primed and challenged and showed the highest score. Birds vaccinated with vaccine E (containing all 8 antigens) showed almost complete protection comparable to the results of the group that did not receive vaccination and challenge but was primed with Newcastle Disease virus. A somewhat lesser, but still significant cross-protection ($P<0.05$) could be observed in birds vaccinated with vaccine A, B and C. Combination vaccine D showed cross-protection of less significance ($p=0.19$). Untreated birds showed no organ lesions.

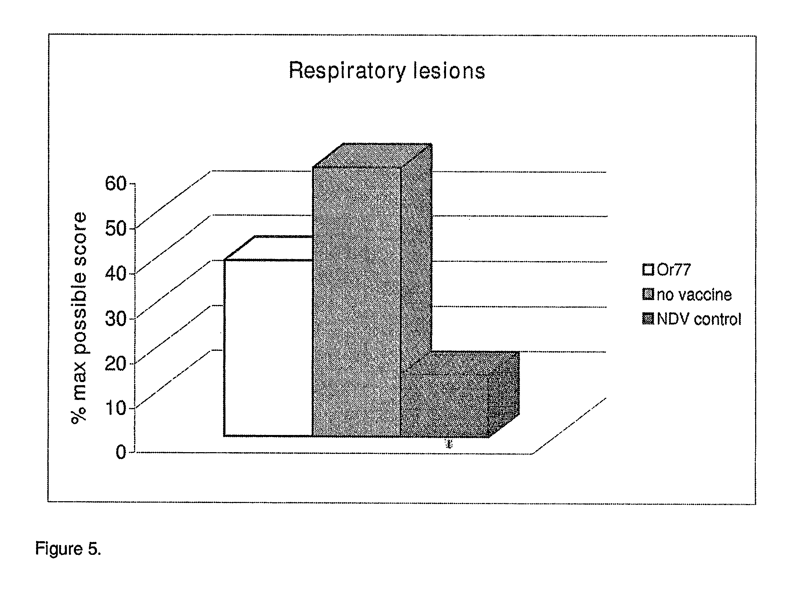

As can be seen from figure 5, the Or77 (= serotype G strain)-vaccinated and serotype A challenged animals also show a significant ($p<0.05$)) reduction in respiratory lesion scores compared to the unvaccinated control group.

Legend to the figures:

Figure 1: Coomassie staining of the 5 combination vaccines (A to E). Each vaccine containing a different composition of the 8 purified recombinant proteins. Subunit vaccine A corresponds with lane A, subunit vaccine B corresponds with lane B, subunit vaccine C corresponds with lane C, subunit vaccine D corresponds with lane D, subunit vaccine E corresponds with lane E. Recombinant proteins with approximately equal molecular weights are indicated by a single arrow.

Figure 2: Reactivity of monovalent antisera, obtained from chickens vaccinated with the single recombinant subunit vaccines, against the same protein on Western blot. The reactive vaccine proteins are indicated with black arrows.

Figure 3: Reactivity of antisera, obtained from chickens vaccinated with subunit vaccines A to E on Western blot. Each blot contains the proteins of vaccine A, B, C, D, and E (corresponding to lanes A to E). The serum used for screening is obtained from birds vaccinated with vaccine A (blot A), vaccine B (blot B), vaccine C (blot C), vaccine D (blot D) or vaccine E (blot E). The serum used on Western blot F is obtained from unvaccinated birds. The reactive vaccine proteins are indicated with a black line.

Figure 4: Cross-protective capacity of subunit vaccines A to E, in comparison to challenge and NDV control groups, represented as the maximum possible respiratory organ lesion score.

Figure 5: Cross-protective capacity of subunit vaccine Or77, in comparison to challenge and NDV control groups, represented as the maximum possible respiratory organ lesion score.

Table 1: Oligonucleotide sets used for cloning selected *Ornithobacterium rhinotracheale* genes encoding cross-reactive antigens

| Gene | 5'oligonucleotide | Restriction site | 3'oligonucleotide | Restriction site |
|---|---|---|---|---|
| Or01 | 5'-GCTGGCCATG*GCTGAAATTATAAAAATGCC3*' | Mscl | 5'-CCGCTCGAG*GCACAAGCATAGACATTGG-3*' | Xhol |
| Or02 | 5'-CAGTCCATG*GCATGTAGCGATTTTGAT3*' | Ncol | 5'-CCGCTCGAG*GTGGTCTTTATAAAAATG-3*' | Xhol |
| Or03 | 5'-CAGTCCATG*GCGATGATAATCAGTTCTTATG-3*' | Ncol | 5'-CCGCTCGAG*AATAAATTCATCATTAAGG3*' | Xhol |
| Or04 | 5'-CGATGGCCATG*AAAGATATATTTGAAT 3*' | Mscl | 5'-CCGCTCGAG*TTCTTCACTTGGTATI-TTGA-3*' | Xhol |
| Or11 | 5'-CGATGGCCAT*GGGGGCACAAGGTGTAGC-3*' | Mscl | 5'-GCGGCCG*CTACGATAAACCTAGACCAAA-3*' | Notl |
| Or77 | 5'-CATGCCATG*GTCTGTAGCAGTGATGATTAG3*' | Ncol | 5'-CCGCTCGAG*GTTAATTGAAACTCTTAAGC3*' | Xhol |
| Or98A | 5'-CAGTCCATG*GTAAAAGACTTTTCAG-3*' | Ncol | 5'-CCGCTCGAG*TGCTATTAATTCTAATCG-3*' | Xhol |
| Or98B | 5'-CAGTCCATG*GAATTAGCGAAAAACGAG3*' | Ncol | 5'-CCGCTCGAG*TTTTAATTCATTTTTTCTG-3*' | Xhol |

Restriction site: underlined
ATG start codon: bold
Gene of interest: *italic*

| Vaccine | Antigen | | | | | | | |
|---|---|---|---|---|---|---|---|---|
| | Or01 | Or02 | Or03 | Or04 | Or11 | Or77 | Or98A | Or98B |
| A | ▓▓▓ | ▓▓▓ | ▓▓▓ | ▓▓▓ | | | | |
| B | | | ▓▓▓ | ▓▓▓ | ▓▓▓ | ▓▓▓ | | |
| C | | | | | ▓▓▓ | ▓▓▓ | ▓▓▓ | ▓▓▓ |
| D | ▓▓▓ | ▓▓▓ | | | | | ▓▓▓ | ▓▓▓ |
| E | ▓▓▓ | ▓▓▓ | ▓▓▓ | ▓▓▓ | ▓▓▓ | ▓▓▓ | ▓▓▓ | ▓▓▓ |

▓▓▓ : antigen is present in the vaccine

Table 2: Subunit vaccines (A to E) consisting of different protein subset combinations

## Claims

1. Nucleic acid encoding an immunogenic 59.8 kD *Ornithobacterium rhinotracheale* protein or a part of said nucleic acid that encodes an immunogenic fragment of said protein, said nucleic acid or said part thereof having at least 80 % homology with the nucleic acid of the *Ornithobacterium rhinotracheale* protein gene as depicted in SEQ ID NO: 1.

2. Nucleic acid or part thereof according to claim 1, **characterized in that** the sequence has at least 85 %, preferably 90 %, more preferably 95 % homology with the nucleic acid of the *Ornithobacterium rhinotracheale* protein gene as depicted in SEQ ID NO: 1.

3. Nucleic acid encoding an immunogenic 58.2 kD *Ornithobacterium rhinotracheale* protein or a part of said nucleic acid that encodes an immunogenic fragment of said protein, said nucleic acid or said part thereof having at least 80 % homology with the nucleic acid of the *Ornithobacterium rhinotracheale* protein gene as depicted in SEQ ID NO: 3.

4. Nucleic acid or part thereof according to claim 3, **characterized in that** the sequence has at least 85 %, preferably 90 %, more preferably 95 % homology with the nucleic acid of the *Ornithobacterium rhinotracheale* protein gene as depicted in SEQ ID NO: 3.

5. Nucleic acid encoding an immunogenic 46.0 kD *Ornithobacterium rhinotracheale* protein or a part of said nucleic acid that encodes an immunogenic fragment of said protein, said nucleic acid or said part thereof having at least 80 % homology with the nucleic acid of the *Ornithobacterium rhinotracheale* protein gene as depicted in SEQ ID NO: 5.

6. Nucleic acid or part thereof according to claim 5, **characterized in that** the sequence has at least 85 %, preferably 90 %, more preferably 95 % homology with the nucleic acid of the *Ornithobacterium rhinotracheale* protein gene as depicted in SEQ ID NO: 5.

7. Nucleic acid encoding an immunogenic 37.2 kD *Ornithobacterium rhinotracheale* protein or a part of said nucleic acid that encodes an immunogenic fragment of said protein, said nucleic acid or said part thereof having at least 80 % homology with the nucleic acid of the *Ornithobacterium rhinotracheale* protein gene as depicted in SEQ ID NO: 7.

8. Nucleic acid or part thereof according to claim 7, **characterized in that** the sequence has at least 85 %, preferably 90 %, more preferably 95 % homology with the nucleic acid of the *Ornithobacterium rhinotracheale* protein gene as depicted in SEQ ID NO: 7.

9. Nucleic acid encoding an immunogenic 45.6 kD *Ornithobacterium rhinotracheale* protein or a part of said nucleic acid that encodes an immunogenic fragment of said protein, said nucleic acid or said part thereof having at least 80 % homology with the nucleic acid of the *Ornithobacterium rhinotracheale* protein gene as depicted in SEQ ID NO: 9.

10. Nucleic acid or part thereof according to claim 9, **characterized in that** the sequence has at least 85 %, preferably 90 %, more preferably 95 % homology with the nucleic acid of the *Ornithobacterium rhinotracheale* protein gene as depicted in SEQ ID NO: 9.

11. Nucleic acid encoding an immunogenic 42.2 kD *Ornithobacterium rhinotracheale* protein or a part of said nucleic acid that encodes an immunogenic fragment of said protein, said nucleic acid or said part thereof having at least 80 % homology with the nucleic acid of he *Ornit\*hobacterium rhinotracheale* protein gene as depicted in SEQ ID NO: 11.

12. Nucleic acid or part thereof according to claim 11, **characterized in that** the sequence has at least 85 %, preferably 90 %, more preferably 95 % homology with the nucleic. acid of the *Ornithobacterium rhinotracheale* protein gene as depicted in SEQ ID NO: 11.

13. Nucleic acid encoding an immunogenic 34.0 kD *Ornithobacterium rhinotracheale* protein or a part of said nucleic acid that encodes an immunogenic fragment of said protein, said nucleic acid or said part thereof having at least 80 % homology with the nucleic acid of the *Ornithobacterium rhinotracheale* protein gene as depicted in SEQ ID NO: 13.

14. Nucleic acid or part thereof according to claim 13, **characterized in that** the sequence has at least 85 %, preferably 90 %, more preferably 95 % homology with the nucleic acid of the *Ornithobacterium rhinotracheale* protein gene as depicted in SEQ ID NO: 13.

15. Nucleic acid encoding an immunogenic 32.9 kD *Ornithobacterium rhinotracheale* protein or a part of said nucleic acid that encodes an immunogenic fragment of said protein, said nucleic acid or said part thereof having at least 80 % homology with the nucleic acid of the *Ornithobacterium rhinotracheale* protein gene as depicted in SEQ ID NO: 15.

16. Nucleic acid or part thereof according to claim 15, **characterized in that** the sequence has at least 85 %, preferably 90 %, more preferably 95 % homology with the nucleic acid of the *Ornithobacterium rhinotracheale* protein gene as depicted in SEQ ID NO: 15.

17. DNA fragment comprising a nucleic acid according to claim 1-16.

18. Recombinant DNA molecule comprising a nucleic acid according to claims 1-16 or a DNA fragment according to claim 17, under the control of a functionally linked promoter.

19. Live recombinant carrier comprising a nucleic acid according to claims 1-16, a DNA fragment according to claim 17 or a recombinant DNA molecule according to claim 18.

20. Host cell comprising a nucleic acid according to claims 1-16, a DNA fragment according to claim 17, a recombinant DNA molecule according to claim 18 or a live recombinant carrier according to claim 19.

21. An immunogenic 59.8 kD *Ornithobacterium rhinotracheale* protein or an immunogenic fragment of said protein, said protein or immunogenic fragment thereof having an amino acid sequence homology of at least 80 % with the amino acid sequence as depicted in SEQ ID NO: 2.

22. An immunogenic *Ornithobacterium rhinotracheale* protein or an immunogenic fragment of said protein, according to claim 21, said protein or immunogenic fragment thereof having an amino acid sequence homology of at least 85 %, preferably 90 %, more preferably 95.% to the amino acid sequence as depicted in SEQ ID NO: 2.

23. An immunogenic 59.8 kD *Ornithobacterium rhinotracheale* protein or an immunogenic fragment thereof, **characterized in that** it is encoded by a nucleic acid according to claim 1 or 2.

24. An immunogenic 58.2 kD *Ornithobacterium rhinotracheale* protein or an immunogenic fragment of said protein, said protein or immunogenic fragment thereof having an amino acid sequence homology of at least 80 % to the amino acid sequence as depicted in SEQ ID NO: 4.

25. An immunogenic *Ornithobacterium rhinotracheale* protein or an immunogenic fragment of said protein, according to claim 24, said protein or immunogenic fragment thereof having an amino acid sequence homology of at least 85 %, preferably 90 %, more preferably 95.% to the amino acid sequence as depicted in SEQ ID NO: 4.

26. An immunogenic 58.2 kD *Ornithobacterium rhinotracheale* protein or an immunogenic fragment thereof, **characterized in that** it is encoded by a nucleic acid according to claim 3 or 4.

27. An immunogenic 46.0 kD *Ornithobacterium rhinotracheale* protein or an immunogenic fragment of said protein, said protein or immunogenic fragment thereof having an amino acid sequence homology of at least 80 % with the amino acid sequence as depicted in SEQ ID NO: 6.

28. An immunogenic *Ornithobacterium rhinotracheale* protein or an immunogenic fragment of said protein, according to claim 27, said protein or immunogenic fragment thereof having an amino acid sequence homology of at least 85 %, preferably 90 %, more preferably 95 % to the amino acid sequence as depicted in SEQ ID NO: 6.

29. An immunogenic 46.0 kD *Ornithobacterium rhinotracheale* protein or an immunogenic fragment thereof, **characterized in that** it is encoded by a nucleic acid according to claim 5 or 6.

30. An immunogenic 37.2 kD *Ornithobacterium rhinotracheale* protein or an immunogenic fragment of said protein, said protein or immunogenic fragment thereof having an amino acid sequence homology of at least 80 % with the amino acid sequence as depicted in SEQ ID NO: 8.

31. *An immunogenic Ornithobacterium rhinotracheale* protein or an immunogenic fragment of said protein, according to claim 30, said protein or immunogenic fragment thereof having an amino acid sequence homology of at least 85 %, preferably 90 %, more preferably 95 % to the amino acid sequence as depicted in SEQ ID NO: 8.

32. An immunogenic 37.2 kD *Ornithobacterium rhinotracheale* protein or an immunogenic fragment thereof, **characterized in that** it is encoded by a nucleic acid according to claim 7 or 8.

33. An immunogenic 45.6 kD *Ornithobacterium rhinotracheale* protein or an immunogenic fragment of said protein, said protein or immunogenic fragment thereof having an amino acid sequence homology of at least 80 % with the amino acid sequence as depicted in SEQ ID NO: 10.

34. An immunogenic *Ornithobacterium rhinotracheale* protein or an immunogenic fragment of said protein, according to claim 33, said protein or immunogenic fragment thereof having an amino acid sequence homology of at least 85 %, preferably 90 %, more preferably 95 % to the amino acid sequence as depicted in SEQ ID NO: 10.

35. An immunogenic 45.6 kD *Ornithobacterium rhinotracheale* protein or an immunogenic fragment thereof, **characterized in that** it is encoded by a nucleic acid according to claim 9 or 10.

36. An immunogenic 42.2 kD *Ornithobacterium rhinotracheale* protein or an immunogenic fragment of said protein, said protein or immunogenic fragment thereof having an amino acid sequence homology of at least 80 % with the amino acid sequence as depicted in SEQ ID NO: 12.

37. An immunogenic *Ornithobacterium rhinotracheale* protein or an immunogenic fragment of said protein, according to claim 36, said protein or immunogenic fragment thereof having an amino acid sequence homology of at least 85 %, preferably 90 %, more preferably 95 % to the amino acid sequence as depicted in SEQ ID NO: 12.

38. An immunogenic 42.2 KD *Ornithobacterium rhinotracheale* protein or an immunogenic fragment thereof, **characterized in that** it is encoded by a nucleic acid according to claim 11 or 12.

39. An immunogenic 34.0 KD *Orinithobacterium rhinotracheale* protein or an immunogenic fragment of said protein, said protein or immunogenic fragment thereof having an amino acid sequence homology of at least 80 % with the amino acid sequence as depicted in SEQ ID NO: 14.

40. An immunogenic *Ornithobacterium rhinotracheale* protein or an immunogenic fragment of said protein, according to claim 39, said protein or immunogenic fragment thereof having an amino acid sequence homology of at least 85 %, preferably 90 %, more preferably 95 % to the amino acid sequence as depicted in SEQ ID NO: 14.

41. An immunogenic 34.0 kD *Orinithobacterium rhinotracheale* protein or an immunogenic fragment thereof, **characterized in that** it is encoded by a nucleic acid according to claim 13 or 14.

42. An immunogenic 32.9 kD *Ornithobacterium rhinotracheale* protein or an immunogenic fragment of said protein, said protein or immunogenic fragment thereof having an amino acid sequence homology of at least 80 % with the amino acid sequence as depicted in SEQ ID NO:16.

43. An immunogenic *Ornithobacterium rhinotrocheale* protein or an immunogenic fragment of said protein, according to claim 42, said protein or immunogenic fragment thereof having an amino acid sequence homology of at least 85 %, preferably 90 %, more preferably 95 % to the amino acid sequence as depicted in SEQ ID NO: 16.

44. An immunogenic 32.9 kD O*rnithobacterium rhinotracheale* protein or an immunogenic fragment thereof, **characterized in that** it is encoded by a nucleic acid according to claim 15 or 16.

45. A nucleic acid according to claims 1-16, a DNA fragment according to claim 17, a recombinant DNA molecule according to claim 18, a live recombinant carrier according to claim 19, a host cell according to claim 20 or a protein according to claims 21-44 or an immunogenic fragment thereof, for use in a vaccine.

46. Use of a nucleic acid according to claims 1-16, a DNA fragment according to claim 17, a recombinant DNA molecule according to claim 18, a live recombinant carrier according to claim 19, a host cell according to claim 20 or a protein according to claims 21-4.4 or an immunogenic fragment thereof for the manufacturing of a vaccine for combating *Ornithobacterium rhinotracheale* infection.

**47.** Vaccine for combating *Ornithobacterium rhinotracheale* infection, **characterized in that** it comprises a nucleic acid according to claims 1-16, a DNA fragment according to claim 17, a recombinant DNA molecule according to claim 18, a live recombinant carrier according to claim 19, a host cell according to claim 20 or a protein according to claims 21-44 or an immunogenic fragment thereof, and a pharmaceutically acceptable carrier.

**48.** Vaccine for combating *Ornithobacterium rhinotracheale* infection, **characterized in that** it comprises antibodies against a protein according to claims 21-44 or an immunogenic fragment of said protein, and a pharmaceutically acceptable carrier.

**49.** Vaccine according to claim 47, **characterized in that** it comprises an adjuvant.

**50.** Vaccine according to claim 47-49, **characterized in that** it comprises an additional antigen derived from a virus or micro-organism pathogenic to poultry, an antibody against such an antigen or genetic information encoding said antigen.

**51.** Vaccine according to claim 50, **characterized in that** said virus or micro-organism pathogenic to chickens is selected from the group consisting of Fowlpox virus, Infectious Bronchitis virus, Infectious Bursal Disease (Gumboro), Marek's Disease Virus, Chicken Anaemia agent, Avian Reovirus, *Mycoplasma gallisepticum,* Turkey Rhinotracheitis virus, *Haemophilus paragallinarum* (Coryza), Chicken Poxvirus, Avian Encephalomyelitisvirus, Duck Plague virus, Newcastle Disease virus, Egg Drop syndrome virus, Infectious Laryngotracheitis virus, Herpes Virus of Turkeys, Eimeria species, *Ornithobacterium rhinotracheale, Pasteurella multocida, Mycoplasma synoviae, Salmonella* species and *E. coli.*

**52.** Method for the preparation of a vaccine according to claims 47-51, said method comprising the admixing of a nucleic acid according to claims 1-16, a DNA fragment according to claim 17, a recombinant DNA molecule according to claim 18, a live recombinant carrier according to claim 19, a host cell according to claim 20, a protein according to claims 21-44 or an immunogenic fragment thereof, or antibodies against a protein according to claims 21-44 and a pharmaceutically acceptable carrier.

**Patentansprüche**

**1.** Nukleinsäure, codierend für ein immunogenes, 59,8 kD großes Protein aus *Ornithobacterium rhinotracheale,* oder ein Teil der Nukleinsäure, der für ein immunogenes Fragment des Proteins codiert, wobei die Nukleinsäure bzw. der Teil davon wenigstens 80% Homologie mit der Nukleinsäure des Gens für das Protein aus *Ornithobacterium rhinotracheale,* wie sie in der SEQ ID NO: 1 dargestellt ist, aufweist.

**2.** Nukleinsäure oder Teil davon nach Anspruch 1, **dadurch gekennzeichnet, dass** die Sequenz wenigstens 85%, vorzugsweise 90%, stärker bevorzugt 95% Homologie zu der Nukleinsäure des Gens für das Protein aus *Ornithobacterium rhinotracheale,* wie sie in der SEQ ID NO: 1 dargestellt ist, aufweist.

**3.** Nukleinsäure, codierend für ein immunogenes, 58,2 kD großes Protein aus *Ornithobacterium rhinotracheale,* oder ein Teil der Nukleinsäure, der für ein immunogenes Fragment des Proteins codiert, wobei die Nukleinsäure bzw. der Teil davon wenigstens 80% Homologie mit der Nukleinsäure des Gens für das Protein aus *Ornithobacterium rhinotracheale,* wie sie in der SEQ ID NO: 3 dargestellt ist, aufweist.

**4.** Nukleinsäure oder Teil davon nach Anspruch 3, **dadurch gekennzeichnet, dass** die Sequenz wenigstens 85%, vorzugsweise 90%, stärker bevorzugt 95% Homologie zu der Nukleinsäure des Gens für das Protein aus *Ornithobacterium rhinotracheale,* wie sie in der SEQ ID NO: 3 dargestellt ist, aufweist.

**5.** Nukleinsäure, codierend für ein immunogenes, 46,0 kD großes Protein aus *Ornithobacterium rhinotracheale,* oder ein Teil der Nukleinsäure, der für ein immunogenes Fragment des Proteins codiert, wobei die Nukleinsäure bzw. der Teil davon wenigstens 80% Homologie mit der Nukleinsäure des Gens für das Protein aus *Ornithobacterium rhinotracheale,* wie sie in der SEQ ID NO: 5 dargestellt ist, aufweist.

**6.** Nukleinsäure oder Teil davon nach Anspruch 5, **dadurch gekennzeichnet, dass** die Sequenz wenigstens 85%, vorzugsweise 90%, stärker bevorzugt 95% Homologie zu der Nukleinsäure des Gens für das Protein aus *Ornithobacterium rhinotracheale,* wie sie in der SEQ ID NO: 5 dargestellt ist, aufweist.

7. Nukleinsäure, codierend für ein immunogenes, 37,2 kD großes Protein aus *Ornithobacterium rhinotracheale,* oder ein Teil der Nukleinsäure, der für ein immunogenes Fragment des Proteins codiert, wobei die Nukleinsäure bzw. der Teil davon wenigstens 80% Homologie mit der Nukleinsäure des Gens für das Protein *aus Ornithobacterium rhinotracheale,* wie sie in der SEQ ID NO: 7 dargestellt ist, aufweist.

8. Nukleinsäure oder Teil davon nach Anspruch 7, **dadurch gekennzeichnet, dass** die Sequenz wenigstens 85%, vorzugsweise 90%, stärker bevorzugt 95% Homologie zu der Nukleinsäure des Gens für das Protein aus *Ornithobacterium rhinotracheale,* wie sie in der SEQ ID NO: 7 dargestellt ist, aufweist.

9. Nukleinsäure, codierend für ein immunogenes, 45,6 kD großes Protein aus *Ornithobacterium rhinotracheale,* oder ein Teil der Nukleinsäure, der für ein immunogenes Fragment des Proteins codiert, wobei die Nukleinsäure bzw. der Teil davon wenigstens 80% Homologie mit der Nukleinsäure des Gens für das Protein aus *Ornithobacterium rhinotracheale,* wie sie in der SEQ ID NO: 9 dargestellt ist, aufweist.

10. Nukleinsäure oder Teil davon nach Anspruch 9, **dadurch gekennzeichnet, dass** die Sequenz wenigstens 85%, vorzugsweise 90%, stärker bevorzugt 95% Homologie zu der Nukleinsäure des Gens für das Protein aus *Ornithobacterium rhinotracheale,* wie sie in der SEQ ID NO: 9 dargestellt ist, aufweist.

11. Nukleinsäure, codierend für ein immunogenes, 42,2 kD großes Protein aus *Ornithobacterium rhinotracheale,* oder ein Teil der Nukleinsäure, der für ein immunogenes Fragment des Proteins codiert, wobei die Nukleinsäure bzw. der Teil davon wenigstens 80% Homologie mit der Nukleinsäure des Gens für das Protein aus *Ornithobacterium rhinotracheale,* wie sie in der SEQ ID NO: 11 dargestellt ist, aufweist.

12. Nukleinsäure oder Teil davon nach Anspruch 11, **dadurch gekennzeichnet, dass** die Sequenz wenigstens 85%, vorzugsweise 90%, stärker bevorzugt 95% Homologie zu der Nukleinsäure des Gens für das Protein aus *Ornithobacterium rhinotracheale,* wie sie in der SEQ ID NO: 11 dargestellt ist, aufweist.

13. Nukleinsäure, codierend für ein immunogenes, 34,0 kD großes Protein aus *Ornithobacterium rhinotracheale,* oder ein Teil der Nukleinsäure, der für ein immunogenes Fragment des Proteins codiert, wobei die Nukleinsäure bzw. der Teil davon wenigstens 80% Homologie mit der Nukleinsäure des Gens für das Protein aus *Ornithobacterium rhinotracheale,* wie sie in der SEQ ID NO: 13 dargestellt ist, aufweist.

14. Nukleinsäure oder Teil davon nach Anspruch 13, **dadurch gekennzeichnet, dass** die Sequenz wenigstens 85%, vorzugsweise 90%, stärker bevorzugt 95% Homologie zu der Nukleinsäure des Gens für das Protein aus *Ornithobacterium rhinotracheale,* wie sie in der SEQ ID NO: 13 dargestellt ist, aufweist.

15. Nukleinsäure, codierend für ein immunogenes, 32,9 kD großes Protein aus *Ornithobacterium rhinotracheale,* oder ein Teil der Nukleinsäure, der für ein immunogenes Fragment des Proteins codiert, wobei die Nukleinsäure bzw. der Teil davon wenigstens 80% Homologie mit der Nukleinsäure des Gens für das Protein aus *Ornithobacterium rhinotracheale,* wie sie in der SEQ ID NO: 15 dargestellt ist, aufweist.

16. Nukleinsäure oder Teil davon nach Anspruch 15, **dadurch gekennzeichnet, dass** die Sequenz wenigstens 85%, vorzugsweise 90%, stärker bevorzugt 95% Homologie zu der Nukleinsäure des Gens für das Protein aus *Ornithobacterium rhinotracheale,* wie sie in der SEQ ID NO: 15 dargestellt ist, aufweist.

17. DNA-Fragment, umfassend eine Nukleinsäure nach Anspruch 1-16.

18. Rekombinantes DNA-Molekül, umfassend eine Nukleinsäure nach Anspruch 1-16 oder ein DNA-Fragment nach Anspruch 17, unter der Kontrolle eines in funktioneller Verknüpfung stehenden Promotors.

19. Lebender rekombinanter Träger, umfassend eine Nukleinsäure nach Anspruch 1-16, ein DNA-Fragment nach Anspruch 17 oder ein rekombinantes DNA-Molekül nach Anspruch 18.

20. Wirtszelle, umfassend eine Nukleinsäure nach Anspruch 1-16, ein DNA-Fragment nach Anspruch 17, ein rekombinantes DNA-Molekül nach Anspruch 18 oder einen lebenden rekombinanten Träger nach Anspruch 19. '

21. Immunogenes, 59,8 kD großes Protein aus *Ornithobacterium rhinotracheale* oder ein immunogenes Fragment des Proteins, wobei das Protein bzw. immunogene Fragment davon eine Aminosäuresequenzhomologie von wenigstens 80% zu der Aminosäuresequenz, wie sie in der SEQ ID NO: 2 dargestellt ist, aufweist.

22. Immunogenes Protein aus *Ornithobacterium rhinotracheale* oder ein immunogenes Fragment des Proteins nach Anspruch 21, wobei das Protein bzw. immunogene Fragment davon eine Aminosäuresequenzhomologie von wenigstens 85%, vorzugsweise 90%, stärker bevorzugt 95% zu der Aminosäuresequenz, wie sie in der SEQ ID NO: 2 dargestellt ist, aufweist.

23. Immunogenes, 59,8 kD großes Protein aus *Ornithobacterium rhinotracheale* oder ein immunogenes Fragment davon, **dadurch gekennzeichnet, dass** es durch eine Nukleinsäure nach Anspruch 1 oder 2 codiert ist.

24. Immunogenes, 58,2 kD großes Protein aus *Ornithobacterium rhinotracheale* oder ein immunogenes Fragment des Proteins, wobei das Protein bzw. immunogene Fragment davon eine Aminosäuresequenzhomologie von wenigstens 80% zu der Aminosäuresequenz, wie sie in der SEQ ID NO: 4 dargestellt ist, aufweist.

25. Immunogenes Protein aus *Ornithobacterium rhinotracheale* oder ein immunogenes Fragment des Proteins nach Anspruch 24, wobei das Protein bzw. immunogene Fragment davon eine Aminosäuresequenzhomologie von wenigstens 85%, vorzugsweise 90%, stärker bevorzugt 95% zu der Aminosäuresequenz, wie sie in der SEQ ID NO: 4 dargestellt ist, aufweist.

26. Immunogenes, 58,2 kD großes Protein aus *Ornithobacterium rhinotracheale* oder ein immunogenes Fragment davon, **dadurch gekennzeichnet, dass** es durch eine Nukleinsäure nach Anspruch 3 oder 4 codiert ist.

27. Immunogenes, 46,0 kD großes Protein aus *Ornithobacterium rhinotracheale* oder ein immunogenes Fragment des Proteins, wobei das Protein bzw. immunogene Fragment davon eine Aminosäuresequenzhomologie von wenigstens 80% zu der Aminosäuresequenz, wie sie in der SEQ ID NO: 6 dargestellt ist, aufweist.

28. Immunogenes Protein aus *Orni-thobacterium rhinotracheale* oder ein immunogenes Fragment des Proteins nach Anspruch 27, wobei das Protein bzw. immunogene Fragment davon eine Aminosäuresequenzhomologie von wenigstens 85%, vorzugsweise 90%, stärker bevorzugt 95% zu der Aminosäuresequenz, wie sie in der SEQ ID NO: 6 dargestellt ist, aufweist.

29. Immunogenes, 46,0 kD großes Protein aus *Ornithobacterium rhinotracheale* oder ein immunogenes Fragment davon, **dadurch gekennzeichnet, dass** es durch eine Nukleinsäure nach Anspruch 5 oder 6 codiert ist.

30. Immunogenes, 37,2 kD großes Protein aus *Ornithobacterium rhinotracheale* oder ein immunogenes Fragment des Proteins, wobei das Protein bzw. immunogene Fragment davon eine Aminosäuresequenzhomologie von wenigstens 80% zu der Aminosäuresequenz, wie sie in der SEQ ID NO: 8 dargestellt ist, aufweist.

31. Immunogenes Protein aus *Ornithobacterium rhinotracheale* oder ein immunogenes Fragment des Proteins nach Anspruch 30, wobei das Protein bzw. immunogene Fragment davon eine Aminosäuresequenzhomologie von wenigstens 85%, vorzugsweise 90%, stärker bevorzugt 95% zu der Aminosäuresequenz, wie sie in der SEQ ID NO: 8 dargestellt ist, aufweist.

32. Immunogenes, 37,2 kD großes Protein aus *Ornithobacterium rhinotracheale* oder ein immunogenes Fragment davon, **dadurch gekennzeichnet, dass** es durch eine Nukleinsäure nach Anspruch 7 oder 8 codiert ist.

33. Immunogenes, 45,6 kD großes Protein aus *Ornithobacterium rhinotracheale* oder ein immunogenes Fragment des Proteins, wobei das Protein bzw. immunogene Fragment davon eine Aminosäuresequenzhomologie von wenigstens 80% zu der Aminosäuresequenz, wie sie in der SEQ ID NO: 10 dargestellt ist, aufweist.

34. Immunogenes Protein aus *Ornithobacterium rhinotracheale* oder ein immunogenes Fragment des Proteins nach Anspruch 33, wobei das Protein bzw. immunogene Fragment davon eine Aminosäuresequenzhomologie von wenigstens 85%, vorzugsweise 90%, stärker bevorzugt 95% zu der Aminosäuresequenz, wie sie in der SEQ ID NO: 10 dargestellt ist, aufweist.

35. Immunogenes, 45,6 kD großes Protein aus *Ornithobacterium rhinotracheale* oder ein immunogenes Fragment davon, **dadurch gekennzeichnet, dass** es durch eine Nukleinsäure nach Anspruch 9 oder 10 codiert ist.

36. Immunogenes, 42,2 kD großes Protein aus *Ornithobacterium rhinotracheale* oder ein immunogenes Fragment des Proteins, wobei das Protein bzw. immunogene Fragment davon eine Aminosäuresequenzhomologie von wenigstens 80% zu der Aminosäuresequenz, wie sie in der SEQ ID NO: 12 dargestellt ist, aufweist.

37. Immunogenes Protein aus *Ornithobacterium rhino*tracheale oder ein immunogenes Fragment des Proteins nach Anspruch 36, wobei das Protein bzw. immunogene Fragment davon eine Aminosäuresequenzhomologie von wenigstens 85%, vorzugsweise 90%, stärker bevorzugt 95% zu der Aminosäuresequenz, wie sie in der SEQ ID NO: 12 dargestellt ist, aufweist.

38. Immunogenes, 42,2 kD großes Protein aus *Ornithobacterium rhinotracheale* oder ein immunogenes Fragment davon, **dadurch gekennzeichnet, dass** es durch eine Nukleinsäure nach Anspruch 11 oder 12 codiert ist.

39. Immunogenes, 34,0 kD großes Protein aus *Ornithobacterium rhinotracheale* oder ein immunogenes Fragment des Proteins, wobei das Protein bzw. immunogene Fragment davon eine Aminosäuresequenzhomologie von wenigstens 80% zu der Aminosäuresequenz, wie sie in der SEQ ID NO: 14 dargestellt ist, aufweist.

40. Immunogenes Protein aus *Ornithobacterium rhinotracheale* oder ein immunogenes Fragment des Proteins nach Anspruch 39, wobei das Protein bzw. immunogene Fragment davon eine Aminosäuresequenzhomologie von wenigstens 85%, vorzugsweise 90%, stärker bevorzugt 95% zu der Aminosäuresequenz, wie sie in der SEQ ID NO: 14 dargestellt ist, aufweist.

41. Immunogenes, 34,0 kD großes Protein aus *Ornithobacterium rhinotracheale* oder ein immunogenes Fragment davon, **dadurch gekennzeichnet, dass** es durch eine Nukleinsäure nach Anspruch 13 oder 14 codiert ist.

42. Immunogenes, 32,9 kD großes Protein aus *Ornithobacterium rhinotracheale* oder ein immunogenes Fragment des Proteins, wobei das Protein bzw. immunogene Fragment davon eine Aminosäuresequenzhomologie von wenigstens 80% zu der Aminosäuresequenz, wie sie in der SEQ ID NO: 16 dargestellt ist, aufweist.

43. Immunogenes Protein aus *Ornithobacterium rhino*tracheale oder ein immunogenes Fragment des Proteins nach Anspruch 42, wobei das Protein bzw. immunogene Fragment davon eine Aminosäuresequenzhomologie von wenigstens 85%, vorzugsweise 90%, stärker bevorzugt 95% zu der Aminosäuresequenz, wie sie in der SEQ ID NO: 16 dargestellt ist, aufweist.

44. Immunogenes, 32,9 kD großes Protein aus *Ornithobacterium rhinotracheale* oder ein immunogenes Fragment davon, **dadurch gekennzeichnet, dass** es durch eine Nukleinsäure nach Anspruch 15 oder 16 codiert ist.

45. Nukleinsäure nach Anspruch 1-16, DNA-Fragment nach Anspruch 17, rekombinantes DNA-Molekül nach Anspruch 18, lebender rekombinanter Träger nach Anspruch 19, Wirtszelle nach Anspruch 20 oder Protein nach Anspruch 21-44 oder ein immunogenes Fragment davon zur Verwendung in einem Impfstoff.

46. Verwendung einer Nukleinsäure nach Anspruch 1-16, eines DNA-Fragments nach Anspruch 17, eines rekombinanten DNA-Moleküls nach Anspruch 18, eines lebenden rekombinanten Trägers nach Anspruch 19, einer Wirtszelle nach Anspruch 20 oder eines Proteins nach Anspruch 21-44 oder eines immunogenen Fragments davon zur Herstellung eines Impfstoffs zur Bekämpfung einer Infektion mit *Ornithobacterium rhinotracheale.*

47. Impfstoff zur Bekämpfung einer Infektion mit *Ornithobacterium rhinotracheale,* **dadurch gekennzeichnet, dass** er eine Nukleinsäure nach Anspruch 1-16, ein DNA-Fragment nach Anspruch 17, ein rekombinantes DNA-Molekül nach Anspruch 18, einen lebenden rekombinanten Träger nach Anspruch 19, eine Wirtszelle nach Anspruch 20 oder ein Protein nach Anspruch 21-44 oder ein immunogenes Fragment davon sowie einen pharmazeutisch unbedenklichen Trägerstoff umfasst.

48. Impfstoff zur Bekämpfung einer Infektion mit *ornithobacterium rhinotracheale,* **dadurch gekennzeichnet, dass** er Antikörper gegen ein Protein nach Anspruch 21-44 oder gegen ein immunogenes Fragment des Proteins sowie einen pharmazeutisch unbedenklichen Trägerstoff umfasst.

49. Impfstoff nach Anspruch 47, **dadurch gekennzeichnet, dass** er ein Adjuvans umfasst.

50. Impfstoff nach Anspruch 47-49, **dadurch gekennzeichnet, dass** er ein zusätzliches, aus einem Geflügelkrankheiten verursachenden Virus oder Mikroorganismus stammendes Antigen, einen Antikörper gegen ein solches Antigen oder für das Antigen codierende genetische Informationen umfasst.

**51.** Impfstoff nach Anspruch 50, **dadurch gekennzeichnet, dass** das Hühnerkrankheiten verursachende Virus bzw. der Hühnerkrankheiten verursachende Mikroorganismus aus der Gruppe Fowlpox-Virus, Infektiöse-Bronchitis-Virus, Gumboro-Krankheit (IBD), Marek'sche-Krankheit-Virus, Hühneranämie-Agens, Vogel-Reovirus, *Mycoplasma gallisepticum,* Truthahn-Rhinotracheitis-Virus, *Haemophilus paragallinarum* (Coryza), Hühnerpockenvirus, Aviäre-Encephalomyelitis-Virus, Entenpestvirus, Newcastle-Disease-Virus, EDS(Egg Drop Syndrome)-Virus, Infektiöse-Laryngotracheitis-Virus, Truthahn-Herpesvirus, Eimeria-Spezies, *Ornithobacterium rhinotracheale, Pasteurella multocida, Mycoplasma synoviae, Salmonella-Spezies* und *E. coli* ausgewählt ist.

**52.** Verfahren zur Herstellung eines Impfstoffs nach Anspruch 47-51, wobei man in dem Verfahren eine Nukleinsäure nach Anspruch 1-16, ein DNA-Fragment nach Anspruch 17, ein rekombinantes DNA-Molekül nach Anspruch 18, einen lebenden rekombinanten Träger nach Anspruch 19, eine Wirtszelle nach Anspruch 20, ein Protein nach Anspruch 21-44 oder ein immunogenes Fragment davon oder Antikörper gegen ein Protein nach Anspruch 21-44 sowie einen pharmazeutisch unbedenklichen Trägerstoff zusammengibt.

**Revendications**

**1.** Acide nucléique codant pour une protéine immunogène d'*Ornithobacterium rhinotracheale* de 59,8 kD ou pour une partie dudit acide nucléique qui code pour un fragment immunogène de ladite protéine, ledit acide nucléique ou ladite partie de ce dernier ayant une homologie d'au moins 80 % avec l'acide nucléique du gène de la protéine d'*Ornithobacterium rhinotracheale* ayant la séquence SEQ ID N° 1.

**2.** Acide nucléique ou partie de ce dernier selon la revendication 1, **caractérisé en ce que** la séquence a une homologie d'au moins 85 %, de préférence de 90 %, plus particulièrement de 95 %, avec l'acide nucléique du gène de la protéine d'*Ornithobacterium rhinotracheale* ayant la séquence SEQ ID N° 1.

**3.** Acide nucléique codant pour une protéine immunogène d'*ornithobacterium rhinotracheale* de 58,2 kD ou pour une partie dudit acide nucléique qui code pour un fragment immunogène de ladite protéine, ledit acide nucléique ou ladite partie de ce dernier ayant une homologie d'au moins 80 % avec l'acide nucléique du gène de la protéine d'*Ornithobacterium rhinotracheale* ayant la séquence SEQ ID N° 3.

**4.** Acide nucléique ou partie de ce dernier selon la revendication 3, **caractérisé en ce que** la séquence a une homologie d'au moins 85 %, de préférence de 90 %, plus particulièrement de 95 %, avec l'acide nucléique du gène de la protéine d'*Ornithobacterium rhinotracheale* ayant la séquence SEQ ID N° 3.

**5.** Acide nucléique codant pour une protéine immunogène d'*Ornithobacterium rhinotracheale* de 46,0 kD ou pour une partie dudit acide nucléique qui code pour un fragment immunogène de ladite protéine, ledit acide nucléique ou ladite partie de ce dernier ayant une homologie d'au moins 80 % avec l'acide nucléique du gène de la protéine d'*Ornithobacterium rhinotracheale* ayant la séquence SEQ ID N° 5.

**6.** Acide nucléique ou partie de ce dernier selon la revendication 5, **caractérisé en ce que** la séquence a une homologie d'au moins 85 %, de préférence de 90 %, plus particulièrement de 95 %, avec l'acide nucléique du gène de la protéine d'*Ornithobacterium rhinotracheale* ayant la séquence SEQ ID N° 5.

**7.** Acide nucléique codant pour une protéine immunogène d'*Ornithobacterium rhinotracheale* de 37,2 kD ou pour une partie dudit acide nucléique qui code pour un fragment immunogène de ladite protéine, ledit acide nucléique ou ladite partie de ce dernier ayant une homologie d'au moins 80 % avec l'acide nucléique du gène de la protéine d'*Ornithobacterium rhinotracheale* ayant la séquence SEQ ID N° 7.

**8.** Acide nucléique ou partie de ce dernier selon la revendication 7, **caractérisé en ce que** la séquence a une homologie d'au -moins 85 %, de préférence de 90 %, plus particulièrement de 95 %, avec l'acide nucléique du gène de la protéine d'*Ornithobacterium rhinotracheale* ayant la séquence SEQ ID N° 7.

**9.** Acide nucléique codant pour une protéine immunogène d'*Ornithobacterium rhinotracheale* de 45,6 kD ou pour une partie dudit acide nucléique qui code pour un fragment immunogène de ladite protéine, ledit acide nucléique ou ladite partie de ce dernier ayant une homologie d'au moins 80 % avec l'acide nucléique du gène de la protéine d'*Ornithobacterium rhinotracheale* ayant la séquence SEQ ID N° 9.

10. Acide nucléique ou partie de ce dernier selon la revendication 9, **caractérisé en ce que** la séquence a une homologie d'au moins 85 %, de préférence de 90 %, plus particulièrement de 95 %, avec l'acide nucléique du gène de la protéine d'*Ornithobacterium rhinotracheale* ayant la séquence SEQ ID N° 9.

11. Acide nucléique codant pour une protéine immunogène d'*Ornithobacterium rhinotracheale* de 42,2 kD ou pour une partie dudit acide nucléique qui code pour un fragment immunogène de ladite protéine, ledit acide nucléique ou ladite partie de ce dernier ayant une homologie d'au moins 80 % avec l'acide nucléique du gène de la protéine d'*Ornithobacterium rhinotracheale* ayant la séquence SEQ ID N° 11.

12. Acide nucléique ou partie de ce dernier selon la revendication 11, **caractérisé en ce que** la séquence a une homologie d'au moins 85 %, de préférence de 90 %, plus particulièrement de 95 %, avec l'acide nucléique du gène de la protéine d'*Ornithobacterium rhinotracheale* ayant la séquence SEQ ID N° 11.

13. Acide nucléique codant pour une protéine immunogène d'*Ornithobacterium rhinotracheale* de 34,0 kD ou pour une partie dudit acide nucléique qui code pour un fragment immunogène de ladite protéine, ledit acide nucléique ou ladite partie de ce dernier ayant une homologie d'au moins 80 % avec l'acide nucléique du gène de la protéine d'*Ornithobacterium rhinotracheale* ayant la séquence SEQ ID N° 13.

14. Acide nucléique ou partie de ce dernier selon la revendication 13, **caractérisé en ce que** la séquence a une homologie d'au moins 85 %, de préférence de 90 %, plus particulièrement de 95 %, avec l'acide nucléique du gène de la protéine d'*Ornithobacterium rhinotracheale* ayant la séquence SEQ ID N° 13.

15. Acide nucléique codant pour une protéine immunogène d'*Ornithobacterium rhinotracheale* de 32,9 kD ou pour une partie dudit acide nucléique qui code pour un fragment immunogène de ladite protéine, ledit acide nucléique ou ladite partie de ce dernier ayant une homologie d'au moins 80 % avec l'acide nucléique du gène de la protéine d'*Ornithobacterium rhinotracheale* ayant la séquence SEQ ID N° 15.

16. Acide nucléique ou partie de ce dernier selon la revendication 15, **caractérisé en ce que** la séquence a une homologie d'au moins 85 %, de préférence de 90 %, plus particulièrement de 95 %, avec l'acide nucléique du gène de la protéine d'*Ornithobacterium rhinotracheale* ayant la séquence SEQ ID N° 15.

17. Fragment d'ADN comprenant un acide nucléique selon les revendications 1 à 16.

18. Molécule d'ADN recombinant comprenant un acide nucléique selon les revendications 1 à 16 ou un fragment d'ADN selon la revendication 17, sous le contrôle d'un promoteur fonctionnellement lié.

19. Support recombinant vivant comprenant un acide nucléique selon les revendications 1 à 16, un fragment d'ADN selon la revendication 17 ou une molécule d'ADN recombinant selon la revendication 18.

20. Cellule hôte comprenant un acide nucléique selon les revendications 1 à 16, un fragment d'ADN selon la revendication 17, une molécule d'ADN recombinant selon la revendication 18 ou un support recombinant vivant selon la revendication 19.

21. Protéine immunogène d'*Ornithobacterium rhinotracheale* de 59,8 kD ou fragment immunogène de ladite protéine, ladite protéine ou ledit fragment immunogène de cette dernière ayant une homologie de séquence d'acides aminés d'au moins 80 % avec la séquence d'acides aminés SEQ ID N° 2.

22. Protéine immunogène d'*Ornithobacterium rhinotracheale* ou fragment immunogène de ladite protéine selon la revendication 21, ladite protéine ou ledit fragment immunogène de cette dernière ayant une homologie de séquence d'acides aminés d'au moins 85 %, de préférence de 90 %, plus particulièrement de 95 % avec la séquence d'acides aminés SEQ ID N° 2.

23. Protéine immunogène d'*Ornithobacterium rhinotracheale* de 59,8 kD, ou fragment immunogène de cette dernière, **caractérisée en ce qu'**elle est codée par un acide nucléique selon la revendication 1 ou 2.

24. Protéine immunogène d'*Ornithobacterium rhinotracheale* de 58,2 kD ou fragment immunogène de ladite protéine, ladite protéine ou ledit fragment immunogène de cette dernière ayant une homologie de séquence d'acides aminés d'au moins 80 % avec la séquence d'acides aminés SEQ ID N° 4..

25. Protéine immunogène d'*Ornithobacterium rhinotracheale* ou fragment immunogène de ladite protéine selon la revendication 24, ladite protéine ou ledit fragment immunogène de cette dernière ayant une homologie de séquence d'acides aminés d'au moins 85 %, de préférence de 90 %, plus particulièrement de 95 % avec la séquence d'acides aminés SEQ ID N° 4.

26. Protéine immunogène d'*Ornithobacterium rhinotracheale* de 58,2 kD, ou fragment immunogène de cette dernière, **caractérisée en ce qu'**elle est codée par un acide nucléique selon la revendication 3 ou 4.

27. Protéine immunogène d'*Ornithobacterium rhinotracheale* de 46,0 kD ou fragment immunogène de ladite protéine, ladite protéine ou ledit fragment immunogène de cette dernière ayant une homologie de séquence d'acides aminés d'au moins 80 % avec la séquence d'acides aminés SEQ ID N° 6.

28. Protéine immunogène d'*Ornithobacterium rhinotracheale* ou fragment immunogène de ladite protéine selon la revendication 27, ladite protéine ou ledit fragment immunogène de cette dernière ayant une homologie de séquence d'acides aminés d'au moins 85 %, de préférence de 90 %, plus particulièrement de 95 % avec la séquence d'acides aminés SEQ ID N° 6.

29. Protéine immunogène d'*Ornithobacterium rhinotracheale* de 46,0 kD, ou fragment immunogène de cette dernière, **caractérisée en ce qu'**elle est codée par un acide nucléique selon la revendication 5 ou 6.

30. Protéine immunogène d'*Ornithobacterium rhinotracheale* de 37,2 kD ou fragment immunogène de ladite protéine, ladite protéine ou ledit fragment immunogène de cette dernière ayant une homologie de séquence d'acides aminés d'au moins 80 % avec la séquence d'acides aminés SEQ ID N° 8.

31. Protéine immunogène d'*Ornithobacterium rhinotracheale* ou fragment immunogène de ladite protéine selon la revendication 30, ladite protéine ou ledit fragment immunogène de cette dernière ayant une homologie de séquence d'acides aminés d'au moins 85 %, de préférence de 90 %, plus particulièrement de 95 % avec la séquence d'acides aminés SEQ ID N° 8.

32. Protéine immunogène d'*Ornithobacterium rhinotracheale* de 37,2 kD, ou fragment immunogène de cette dernière, **caractérisée en ce qu'**elle est codée par un acide nucléique selon la revendication 7 ou 8.

33. Protéine immunogène d'*Ornithobacterium rhinotracheale* de 45,6 kD ou fragment immunogène de ladite protéine, ladite protéine ou ledit fragment immunogène de cette dernière ayant une homologie de séquence d'acides aminés d'au moins 80 % avec la séquence d'acides aminés SEQ ID N° 10.

34. Protéine immunogène d'*Ornithobacterium rhinotracheale* ou fragment immunogène de ladite protéine selon la revendication 33, ladite protéine ou ledit fragment immunogène de cette dernière ayant une homologie de séquence d'acides aminés d'au moins 85 %, de préférence de 90 %, plus particulièrement de 95 % avec la séquence d'acides aminés SEQ ID N° 10.

35. Protéine immunogène d'*Ornithobacterium rhinotracheale* de 45,6 kD, ou fragment immunogène de cette dernière, **caractérisée en ce qu'**elle est codée par un acide nucléique selon la revendication 9 ou 10.

36. Protéine immunogène d'*Ornithobacterium rhinotracheale* de 42,2 kD ou fragment immunogène de ladite protéine, ladite protéine ou ledit fragment immunogène de cette dernière ayant une homologie de séquence d'acides aminés d'au moins 80 % avec la séquence d'acides aminés SEQ ID N° 12.

37. Protéine immunogène d'*Ornithobacterium rhinotracheale* ou fragment immunogène de ladite protéine selon la revendication 36, ladite protéine ou ledit fragment immunogène de cette dernière ayant une homologie de séquence d'acides aminés d'au moins 85 %, de préférence de 90 %, plus particulièrement de 95 % avec la séquence d'acides aminés SEQ ID N° 12.

38. Protéine immunogène d'*Ornithobacterium rhinotracheale* de 42,2 kD, ou fragment immunogène de cette dernière, **caractérisée en ce qu'**elle est codée par un acide nucléique selon la revendication 11 ou 12.

39. Protéine immunogène *d'Ornithobacterium rhinotracheale* de 34,0 kD ou fragment immunogène de ladite protéine, ladite protéine ou ledit fragment immunogène de cette dernière ayant une homologie de séquence d'acides aminés d'au moins 80 % avec la séquence d'acides aminés SEQ ID N° 14.

40. Protéine immunogène d'*Ornithobacterium rhinotracheale* ou fragment immunogène de ladite protéine selon la revendication 39, ladite protéine ou ledit fragment immunogène de cette dernière ayant une homologie de séquence d'acides aminés d'au moins 85 %, de préférence de 90 %, plus particulièrement de 95 % avec la séquence d'acides aminés SEQ ID N° 14.

41. Protéine immunogène d'*Ornithobacterium rhinotracheale* de 34,0 kD, ou fragment immunogène de cette dernière, **caractérisée en ce qu'**elle est codée par un acide nucléique selon la revendication 13 ou 14.

42. Protéine immunogène *d'Ornithobacterium rhinotracheale* de 32,9 kD ou fragment immunogène de ladite protéine, ladite protéine ou ledit fragment immunogène de cette dernière ayant une homologie de séquence d'acides aminés d'au moins 80 % avec la séquence d'acides aminés SEQ ID N° 16.

43. Protéine immunogène d'*Ornithobacterium rhinotracheale* ou fragment immunogène de ladite protéine selon la revendication 42, ladite protéine ou ledit fragment immunogène de cette dernière ayant une homologie de séquence d'acides aminés d'au moins 85 %, de préférence de 90 %, plus particulièrement de 95 % avec la séquence d'acides aminés SEQ ID N° 16.

44. Protéine immunogène d'*Ornithobacterium rhinotracheale de* 32,9 kD, ou fragment immunogène de cette dernière, **caractérisée en ce qu'**elle est codée par un acide nucléique selon la revendication 15 ou 16.

45. Acide nucléique codant pour les revendications 1 à 16, fragment d'ADN selon la revendication 17, molécule d'ADN recombinant selon la revendication 18, support recombinant vivant selon la revendication 19, cellule hôte selon la revendication 20 ou protéine selon les revendications 21 à 44 ou fragment immunogène de cette dernière, pour utilisation dans un vaccin.

46. Utilisation d'un acide nucléique selon les revendications 1 à 16, d'un fragment d'ADN selon la revendication 17, d'une molécule d'ADN recombinant selon la revendication 18, d'un support recombinant vivant selon la revendication 19, d'une cellule hôte selon la revendication 20 ou d'une protéine selon les revendications 21 à 44 ou d'un fragment immunogène de cette dernière, pour la fabrication d'un vaccin destiné à combattre une infection à *Ornithobacterium rhinotracheale.*

47. Vaccin pour combattre une infection à *Ornithobacterium rhinotracheale,* **caractérisé en ce qu'**il comprend un acide nucléique selon les revendications 1 à 16, un fragment d'ADN selon la revendication 17, une molécule d'ADN recombinant selon la revendication 18, un support recombinant vivant selon la revendication 19, une cellule hôte selon la revendication 20 ou une protéine selon les revendications 21 à 44 ou un fragment immunogène de cette dernière, et un excipient acceptable d'un point de vue pharmaceutique.

48. Vaccin pour combattre une infection à *Ornithobacterium rhinotracheale,* **caractérisé en ce qu'**il comprend des anticorps contre une protéine selon les revendications 21 à 44 ou un fragment immunogène de ladite protéine, et un excipient acceptable d'un point de vue pharmaceutique.

49. Vaccin selon la revendication 47, **caractérisé en ce qu'**il comprend un adjuvant.

50. Vaccin selon les revendications 47 à 49, **caractérisé en ce qu'**il comprend un antigène additionnel qui dérive d'un virus ou d'un microorganisme pathogène pour la volaille, un anticorps contre un tel antigène ou une information génétique codant pour ledit antigène.

51. Vaccin selon la revendication 50, **caractérisé en ce que** ledit virus ou microorganisme pathogène pour la volaille est choisi dans le groupe consistant en le virus de la variole aviaire, le virus de la bronchite infectieuse, la bursite infectieuse des volailles (maladie de Gumboro), le virus de la maladie de Marek, l'agent de l'anémie du poulet, le réovirus aviaire, *Mycoplasma gallisepticum,* le virus de la rhinotrachéite de la dinde, *Haemophilus paragallinarum* (coryza), le virus varicelle-zona du poulet, le virus de l'encéphalomyélite du poulet, le virus de la peste du canard, le virus de la maladie de Newcastle, le virus du syndrome chute de ponte, le virus de la laryngotrachéite infectieuse, l'herpesvirus de la dinde, *Eimeria species, Ornithobacterium rhinotracheale, Pasteurella multocida, Mycoplasma synoviae,* l'espèce *Salmonella* et *E. coli.*

52. Procédé de préparation d'un vaccin selon les revendications 47 à 51, ledit procédé comprenant le mélange d'un acide nucléique selon les revendications 1 à 16, d'un fragment d'ADN selon la revendication 17, d'une molécule d'ADN recombinant selon la revendication 18, d'un support recombinant vivant selon la revendication 19, d'une cellule hôte selon la revendication 20, d'une protéine selon les revendications 21 à 44 ou d'un fragment immunogène de cette dernière, ou d'anticorps contre une protéine selon les revendications 21 à 44, et d'un excipient acceptable d'un point de vue pharmaceutique.

Figure 1.

Figure 2.

| Strip | Protein on blot: | Sera from birds vaccinated with: |
|-------|------------------|----------------------------------|
| 1 | Or01 | Or01 |
| 2 | Or02 | Or02 |
| 3 | Or03 | Or03 |
| 4 | Or04 | Or04 |
| 5 | Or11 | Or11 |
| 6 | Or77 | Or77 |
| 7 | Or98A | Or98A |
| 8 | Or98B | Or98B |

Figure 3.

Figure 4.

EP 1 716 169 B1

Figure 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0625190 A
- FR 2714074
- US 08043109 B, Hoffman, S. and Rogers, W.
- EP 0473210A2 A
- WO 8403564 A
- WO 8606487 A

- US 4833092 A
- US 4554101 A
- US 07005885 B
- EP 109942 A
- EP 180564 A
- EP 242380 A

**Non-patent literature cited in the description**

- **van Empel, P.C.M. ; Hafez, H.M.** *Avian Pathology,* 1999, vol. 28, 217-227
- **S.J. Sprenger.** *Avian Diseases,* 2000, vol. 44, 549-555
- **V. Lopes.** *Avian Diseases,* 2002, vol. 46, 177-185
- **Tatiana A. Tatusova ; Thomas L. Madden.** *FEMS Microbiol. Letters,* 1999, vol. 174, 247-250
- **Meinkoth ; Wahl.** Hybridization of nucleic acids immobilized on solid supports. *Anal. Biochem.,* 1984, vol. 138, 267-284
- **Sambrook, J.** Molecular cloning: a laboratory manual. 1989
- **Goeddel et al.** *NucL Acids Res.,* 1980, vol. 8, 4057
- **Chang et al.** *Nature,* 1978, vol. 275, 615
- **Nakamura, K. ; Inouge, M.** *EMBO J.,* 1982, vol. 1, 771-775
- **Remaut, E. et al.** *Nucl. Acids Res.,* 1983, vol. 11, 4677-4688
- **Smith, G.E. et al.** *Mol. Cell. Biol.,* 1983, vol. 3, 2156-65
- **Seed, B. et al.** *Nature,* 1987, vol. 329, 840-842
- **Fynan, E.F. et al.** *PNAS,* 1993, vol. 90, 11478-11482
- **Ulmer, J.B. et al.** *Science,* 1993, vol. 259, 1745-1748
- **Gorman, C.M. et al.** *PNAS,* 1982, vol. 79, 6777-6781
- **Stacey et al.** *J. Virology,* 1984, vol. 50, 725-732
- **Sprague J. et al.** *J. Virology,* 1983, vol. 45, 773
- **Berman, P.W. et al.** *Science,* 1983, vol. 222, 524-527
- **Brinster, R.L. et al.** *Nature,* 1982, vol. 296, 39-42

- **Voellmy et al.** *Proc. Natl. Acad. Sci. USA,* 1985, vol. 82, 4949-53
- **Tang et al.** *Nature,* 1992, vol. 356, 152-154
- **Vermeulen, A. N.** *Int. Journ. Parasitol.,* 1998, vol. 28, 1121-1130
- **Panicali et al.** *Proc. Natl. Acad. Sci. USA,* 1982, vol. 79, 4927
- Experimental Haematology today. Springer Verlag, 1988, 92-99
- **Luckow et al.** *Bio-technology,* 1988, vol. 6, 47-55
- **Barton, K.A. et al.** *Cell,* 1983, vol. 32, 1033
- **Neurath et al.** The Proteins. Academic Press, 1979
- **Dayhof, M.D.** Atlas of protein sequence and structure. Nat. Biomed. Res. Found, 1978, vol. 5
- *Science,* 1985, vol. 227, 1435-1441
- *Proc. Natl Acad. Sci.,* 1984, vol. 81, 3998-4002
- *J. Imm. Meth.,* 1987, vol. 102, 259-274
- **Hopp ; Woods.** *Proc. Natl. Acad. Sci.,* 1981, vol. 78, 38248-3828
- *Advances in Enzymology,* 1987, vol. 47, 45-148
- *Science,* 1987, vol. 235, 1059-1062
- **Shan Lu.** common principles. *Tibtech,* 1991, vol. 9, 238-242
- **Good et al.** Malaria epitopes. *Science,* 1987, vol. 235, 1059-1062
- *Vaccine,* 1992, vol. 10, 3-7
- **Berzofsky.** HIV-epitopes. *The FASEB Journal,* 1991, vol. 5, 2412-2418
- **Cortese, R. et al.** *Trends Biotechn.,* 1994, vol. 12, 262-267
- **Clackson, T. ; Wells, J.A.** *Trends Biotechn.,* 1994, vol. 12, 173-183
- **Marks, J.D. et al.** *J. Biol. Chem.,* 1992, vol. 267, 16007-16010

- **Winter, G. et al.** *Annu. Rev. Immunol.,* 1994, vol. 12, 433-455
- **Little, M. et al.** *Biotechn. Adv.,* 1994, vol. 12, 539-555
- **Muyldermans, S. ; Lauwereys, M.** *Journ. Molec. Recogn.,* 1999, vol. 12, 131-140
- **Ghahroudi, M.A. et al.** *FEBS Letters,* 1997, vol. 414, 512-526
- **Donnelly et al.** *The Immunologist,* 1993, vol. 2, 20-26
- **Bovarnik et al.** *J. Bacteriology,* 1950, vol. 59, 509
- **Sambrook, J. et al.** Molecular cloning: a laboratory manual.
- **Towbin, H. ; Staehlin, T. ; Gordon, J.** *Proc. Nat. Acad. Sci.,* 1979, vol. 76, 43-50

SEQUENCE LISTING

<110> AKZO Nobel N.V.

<120> *Ornithobacterium rhinotracheale* subunit vaccines

<130> 2004.011

<160> 16

<170> Patent In version 3.2

<210> 1
<211> 1614
<212> DNA
<213> *Ornithobacterium rhinotracheale*

<220>
<221> CDS
<222> (1)..(1614)

<400> 1

```
atg gct gaa att ata aaa atg cca aga ttg agc gat acc atg gaa gaa      48
Met Ala Glu Ile Ile Lys Met Pro Arg Leu Ser Asp Thr Met Glu Glu
1               5                   10                  15


ggt aaa gtg gaa tct tgg aac aaa aaa gta gga gat aaa gta tca tac      96
Gly Lys Val Glu Ser Trp Asn Lys Lys Val Gly Asp Lys Val Ser Tyr
                20                  25                  30


ggc gac atc tta gcc gaa atc gaa aca gat aaa gcg gtt caa gaa ttt     144
Gly Asp Ile Leu Ala Glu Ile Glu Thr Asp Lys Ala Val Gln Glu Phe
            35                  40                  45


gaa aca gat gta gaa ggt act ctt tta tac atc ggt gta gag gct ggt     192
Glu Thr Asp Val Glu Gly Thr Leu Leu Tyr Ile Gly Val Glu Ala Gly
        50                  55                  60


caa gca gca cca gtt gat agt att tta gct atc atc ggt gca gaa ggc     240
Gln Ala Ala Pro Val Asp Ser Ile Leu Ala Ile Ile Gly Ala Glu Gly
65                  70                  75                  80


gaa gac atc agc ggt ttg gta agc ggt gga ggt gct agc caa tca gcg     288
Glu Asp Ile Ser Gly Leu Val Ser Gly Gly Gly Ala Ser Gln Ser Ala
```

                    85                          90                          95

cca gct caa gaa gct gcc gct cct gca gaa gaa cca caa gcg gaa gct        336
Pro Ala Gln Glu Ala Ala Ala Pro Ala Glu Glu Pro Gln Ala Glu Ala
            100                     105                     110

gca cca gcg gct gaa gtt cca gaa aat gta act atc gtt tct atg cca        384
Ala Pro Ala Ala Glu Val Pro Glu Asn Val Thr Ile Val Ser Met Pro
            115                     120                     125

aga ttg agc gat acc atg gaa gaa ggt aaa gta gaa tct tgg aac aaa        432
Arg Leu Ser Asp Thr Met Glu Glu Gly Lys Val Glu Ser Trp Asn Lys
        130                     135                     140

aaa gta gga gat aaa gta tca tac ggc gac atc tta gcc gaa atc gaa        480
Lys Val Gly Asp Lys Val Ser Tyr Gly Asp Ile Leu Ala Glu Ile Glu
145                     150                     155                     160

aca gat aaa gcg gtt caa gaa ttt gaa aca gat gta gaa ggt act tta        528
Thr Asp Lys Ala Val Gln Glu Phe Glu Thr Asp Val Glu Gly Thr Leu
                165                     170                     175

tta tat ata ggt gta gaa gct ggg caa tca gca cca gtt gat agc att        576
Leu Tyr Ile Gly Val Glu Ala Gly Gln Ser Ala Pro Val Asp Ser Ile
            180                     185                     190

ttg gca atc atc gga cct gaa gga aca gat gtt tct gca atc gta gca        624
Leu Ala Ile Ile Gly Pro Glu Gly Thr Asp Val Ser Ala Ile Val Ala
            195                     200                     205

gga ggt ggt gca aaa cca gct gct aaa gcg gaa gct cca aag gct gaa        672
Gly Gly Gly Ala Lys Pro Ala Ala Lys Ala Glu Ala Pro Lys Ala Glu
        210                     215                     220

gca cct aag caa gct gct cca gca caa gag aaa aaa gaa act cca gcg        720
Ala Pro Lys Gln Ala Ala Pro Ala Gln Glu Lys Lys Glu Thr Pro Ala
225                     230                     235                     240

cct gct gct cca aaa gca caa gct acc aac aat tca ggt aga gta ttt        768
Pro Ala Ala Pro Lys Ala Gln Ala Thr Asn Asn Ser Gly Arg Val Phe
                245                     250                     255

att tct cca ttg gct aaa aaa ttg gct gat gaa aaa gga tac gat atc        816
Ile Ser Pro Leu Ala Lys Lys Leu Ala Asp Glu Lys Gly Tyr Asp Ile
            260                     265                     270

17

```
aat caa att caa ggt aca gga gac aac gga aga atc atc aaa aaa gat        864
Asn Gln Ile Gln Gly Thr Gly Asp Asn Gly Arg Ile Ile Lys Lys Asp
        275                 280                 285


gtt gaa aac ttt act cca caa gct gct gcg gct aag cca gct gtt gct        912
Val Glu Asn Phe Thr Pro Gln Ala Ala Ala Ala Lys Pro Ala Val Ala
        290                 295                 300


ggt cca gtt gca ttg gaa gta gga gaa gat act gta atc cct aac tct        960
Gly Pro Val Ala Leu Glu Val Gly Glu Asp Thr Val Ile Pro Asn Ser
305                 310                 315                 320


caa atg aga aaa gtg att gct aag cgt ctt tct gaa agt aaa ttt aca        1008
Gln Met Arg Lys Val Ile Ala Lys Arg Leu Ser Glu Ser Lys Phe Thr
                    325                 330                 335


gca cca cac tac tac tta acc att gaa gta gat atg gat aat gtg atg        1056
Ala Pro His Tyr Tyr Leu Thr Ile Glu Val Asp Met Asp Asn Val Met
                340                 345                 350


gcg gct cgt aag caa atc aac caa att cca aat aca aaa gta tct ttc        1104
Ala Ala Arg Lys Gln Ile Asn Gln Ile Pro Asn Thr Lys Val Ser Phe
                355                 360                 365


aac gat atc gta ttg aag gct act gct atg gct gtg aaa aaa cac cca        1152
Asn Asp Ile Val Leu Lys Ala Thr Ala Met Ala Val Lys Lys His Pro
        370                 375                 380


gtg gta aat tca act tgg aaa gat aac gaa atc gta caa tac gct gct        1200
Val Val Asn Ser Thr Trp Lys Asp Asn Glu Ile Val Gln Tyr Ala Ala
385                 390                 395                 400


gta aac atc ggt gtt gca gtt gct gtt cca gat ggg ctt gta gta cct        1248
Val Asn Ile Gly Val Ala Val Ala Val Pro Asp Gly Leu Val Val Pro
                405                 410                 415


gta gtg aaa aat aca gat tta aaa tca tta tct caa att tct gct gag        1296
Val Val Lys Asn Thr Asp Leu Lys Ser Leu Ser Gln Ile Ser Ala Glu
                420                 425                 430


gta aaa gat tta gct aca aga tca aga gat aga aaa atc aaa gct gat        1344
Val Lys Asp Leu Ala Thr Arg Ser Arg Asp Arg Lys Ile Lys Ala Asp
        435                 440                 445
```

18

```
gag atg gaa ggt tct acc ttt aca gtt tct aac cta gga gct tac ggt    1392
Glu Met Glu Gly Ser Thr Phe Thr Val Ser Asn Leu Gly Ala Tyr Gly
    450             455             460

gta gaa agc ttt aca tca atc atc aat cag cca aac tct tgt atc ctt    1440
Val Glu Ser Phe Thr Ser Ile Ile Asn Gln Pro Asn Ser Cys Ile Leu
465             470             475             480

tct gta ggt gcg att gta gaa aaa cca gtt gtt aaa aac gga caa atc    1488
Ser Val Gly Ala Ile Val Glu Lys Pro Val Val Lys Asn Gly Gln Ile
                485             490             495

gta gtt ggt cac aca atg aaa ctt tgt tta gct tgc gat cac aga act    1536
Val Val Gly His Thr Met Lys Leu Cys Leu Ala Cys Asp His Arg Thr
            500             505             510

gtg gac gga gca act gga agt act ttc cta caa act tta aaa caa tac    1584
Val Asp Gly Ala Thr Gly Ser Thr Phe Leu Gln Thr Leu Lys Gln Tyr
        515             520             525

tta gag act cca atg tct atg ctt gtg tag                            1614
Leu Glu Thr Pro Met Ser Met Leu Val
        530             535
```

<210> 2
<211> 537
<212> PRT
<213> *Ornithobacterium rhinotracheale*

<400> 2

```
Met Ala Glu Ile Ile Lys Met Pro Arg Leu Ser Asp Thr Met Glu Glu
1               5               10              15


Gly Lys Val Glu Ser Trp Asn Lys Lys Val Gly Asp Lys Val Ser Tyr
            20              25              30


Gly Asp Ile Leu Ala Glu Ile Glu Thr Asp Lys Ala Val Gln Glu Phe
        35              40              45


Glu Thr Asp Val Glu Gly Thr Leu Leu Tyr Ile Gly Val Glu Ala Gly
```

```
                50                    55                    60


Gln Ala Ala Pro Val Asp Ser Ile Leu Ala Ile Ile Gly Ala Glu Gly
65                  70              75                  80


Glu Asp Ile Ser Gly Leu Val Ser Gly Gly Gly Ala Ser Gln Ser Ala
                85                  90                  95


Pro Ala Gln Glu Ala Ala Ala Pro Ala Glu Glu Pro Gln Ala Glu Ala
                100                 105                 110


Ala Pro Ala Ala Glu Val Pro Glu Asn Val Thr Ile Val Ser Met Pro
            115                 120                 125


Arg Leu Ser Asp Thr Met Glu Glu Gly Lys Val Glu Ser Trp Asn Lys
        130                 135                 140


Lys Val Gly Asp Lys Val Ser Tyr Gly Asp Ile Leu Ala Glu Ile Glu
145             150                 155                 160


Thr Asp Lys Ala Val Gln Glu Phe Glu Thr Asp Val Glu Gly Thr Leu
                165                 170                 175


Leu Tyr Ile Gly Val Glu Ala Gly Gln Ser Ala Pro Val Asp Ser Ile
            180                 185                 190


Leu Ala Ile Ile Gly Pro Glu Gly Thr Asp Val Ser Ala Ile Val Ala
            195                 200                 205


Gly Gly Gly Ala Lys Pro Ala Ala Lys Ala Glu Ala Pro Lys Ala Glu
        210                 215                 220


Ala Pro Lys Gln Ala Ala Pro Ala Gln Glu Lys Lys Glu Thr Pro Ala
225                 230                 235                 240
```

```
Pro Ala Ala Pro Lys Ala Gln Ala Thr Asn Asn Ser Gly Arg Val Phe
              245                 250                 255

Ile Ser Pro Leu Ala Lys Lys Leu Ala Asp Glu Lys Gly Tyr Asp Ile
              260                 265                 270

Asn Gln Ile Gln Gly Thr Gly Asp Asn Gly Arg Ile Ile Lys Lys Asp
              275                 280                 285

Val Glu Asn Phe Thr Pro Gln Ala Ala Ala Lys Pro Ala Val Ala
              290                 295                 300

Gly Pro Val Ala Leu Glu Val Gly Glu Asp Thr Val Ile Pro Asn Ser
305                 310                 315                 320

Gln Met Arg Lys Val Ile Ala Lys Arg Leu Ser Glu Ser Lys Phe Thr
                  325                 330                 335

Ala Pro His Tyr Tyr Leu Thr Ile Glu Val Asp Met Asp Asn Val Met
                  340                 345                 350

Ala Ala Arg Lys Gln Ile Asn Gln Ile Pro Asn Thr Lys Val Ser Phe
              355                 360                 365

Asn Asp Ile Val Leu Lys Ala Thr Ala Met Ala Val Lys Lys His Pro
              370                 375                 380

Val Val Asn Ser Thr Trp Lys Asp Asn Glu Ile Val Gln Tyr Ala Ala
385                 390                 395                 400

Val Asn Ile Gly Val Ala Val Ala Val Pro Asp Gly Leu Val Val Pro
                  405                 410                 415
```

```
Val Val Lys Asn Thr Asp Leu Lys Ser Leu Ser Gln Ile Ser Ala Glu
            420               425               430

Val Lys Asp Leu Ala Thr Arg Ser Arg Asp Arg Lys Ile Lys Ala Asp
            435               440               445

Glu Met Glu Gly Ser Thr Phe Thr Val Ser Asn Leu Gly Ala Tyr Gly
        450               455               460

Val Glu Ser Phe Thr Ser Ile Ile Asn Gln Pro Asn Ser Cys Ile Leu
465               470               475               480

Ser Val Gly Ala Ile Val Glu Lys Pro Val Val Lys Asn Gly Gln Ile
                485               490               495

Val Val Gly His Thr Met Lys Leu Cys Leu Ala Cys Asp His Arg Thr
            500               505               510

Val Asp Gly Ala Thr Gly Ser Thr Phe Leu Gln Thr Leu Lys Gln Tyr
            515               520               525

Leu Glu Thr Pro Met Ser Met Leu Val
            530               535
```

<210> 3
<211> 1572
<212> DNA
<213> *Ornithobacterium rhinotracheale*

<220>
<221> CDS
<222> (1)..(1572)

<400> 3

```
atg aaa ata aat tac aaa aat ata ctt tta agt gct agc gtt ctc ttt        48
```

```
              Met Lys Ile Asn Tyr Lys Asn Ile Leu Leu Ser Ala Ser Val Leu Phe
              1               5                   10                  15


ttt gca gca tgt agc gat ttt gat tac aat gta gaa aac cca aac ctc          96
Phe Ala Ala Cys Ser Asp Phe Asp Tyr Asn Val Glu Asn Pro Asn Leu
              20                  25                  30


acg aag gga gag gct gat ttc tct aaa tat gta gct tta gga aat tct         144
Thr Lys Gly Glu Ala Asp Phe Ser Lys Tyr Val Ala Leu Gly Asn Ser
          35                  40                  45


ctc act tct ggt tat tca gac gga gcc tta tat cgc tcg gca caa gag         192
Leu Thr Ser Gly Tyr Ser Asp Gly Ala Leu Tyr Arg Ser Ala Gln Glu
          50                  55                  60


aat tca tac ccc gca atc att gcc aaa caa atg aaa tat gta ggc ggt         240
Asn Ser Tyr Pro Ala Ile Ile Ala Lys Gln Met Lys Tyr Val Gly Gly
65                  70                  75                  80


ggc gag ttc tct caa cct ttg atg aaa gac aac att ggt ggt ttt tcg         288
Gly Glu Phe Ser Gln Pro Leu Met Lys Asp Asn Ile Gly Gly Phe Ser
                  85                  90                  95


gat ttg ttt gaa gca agt aaa cac acc gca ttt tac gga aaa tta gaa         336
Asp Leu Phe Glu Ala Ser Lys His Thr Ala Phe Tyr Gly Lys Leu Glu
              100                 105                 110


tta aaa atc gta gac ggt gca cct acg cca gtg cct tct gtg cct aag         384
Leu Lys Ile Val Asp Gly Ala Pro Thr Pro Val Pro Ser Val Pro Lys
          115                 120                 125


ttt agt tta gct caa acc ttc gta aaa ggg aat ttt aat aat ttg ggc         432
Phe Ser Leu Ala Gln Thr Phe Val Lys Gly Asn Phe Asn Asn Leu Gly
          130                 135                 140


gtg cca ggg gct aaa tct tat cat tta tta gct caa ggt tac gga aat         480
Val Pro Gly Ala Lys Ser Tyr His Leu Leu Ala Gln Gly Tyr Gly Asn
145                 150                 155                 160


att gct aat ctg aag gag agt aaa gcc aat cca tat ttt gtg cga ttt         528
Ile Ala Asn Leu Lys Glu Ser Lys Ala Asn Pro Tyr Phe Val Arg Phe
              165                 170                 175


gct agc caa cca aat gcc agc gtg ctg agc gat gct ttg gca caa aaa         576
Ala Ser Gln Pro Asn Ala Ser Val Leu Ser Asp Ala Leu Ala Gln Lys
```

23

```
              180                185                190

cct aca ttc ttt acc tta tgg atc ggg aac aac gat gtt tta ggc tat    624
Pro Thr Phe Phe Thr Leu Trp Ile Gly Asn Asn Asp Val Leu Gly Tyr
        195                200                205

gcc atg aat ggc gca gca agc aca gat cga aaa ggg aac cct gat gta    672
Ala Met Asn Gly Ala Ala Ser Thr Asp Arg Lys Gly Asn Pro Asp Val
    210                215                220

aca aca tat aat tca aat gat ttg tct gat gct aac ttg gtg gca ggc    720
Thr Thr Tyr Asn Ser Asn Asp Leu Ser Asp Ala Asn Leu Val Ala Gly
225                230                235                240

tct att caa aaa tta gta aaa gca ctt aca gat tca ggc gca aaa ggt    768
Ser Ile Gln Lys Leu Val Lys Ala Leu Thr Asp Ser Gly Ala Lys Gly
            245                250                255

gct gta gcg aat ttg cct tat gtc gaa gac att ccg tat ttt aca acc    816
Ala Val Ala Asn Leu Pro Tyr Val Glu Asp Ile Pro Tyr Phe Thr Thr
        260                265                270

gtg ccg gct gag cct tta agc cct tta aac aaa agt tac gct aca caa    864
Val Pro Ala Glu Pro Leu Ser Pro Leu Asn Lys Ser Tyr Ala Thr Gln
        275                280                285

att gaa aat ttg aat aaa ttt tat gct agc cta aat aaa gtt ttt gat    912
Ile Glu Asn Leu Asn Lys Phe Tyr Ala Ser Leu Asn Lys Val Phe Asp
        290                295                300

gcc cta gga gca agc gat aga aaa atc aca ttt aat gcc gat aaa gca    960
Ala Leu Gly Ala Ser Asp Arg Lys Ile Thr Phe Asn Ala Asp Lys Ala
305                310                315                320

agc ggt gct gtg att gta gat aaa agt ttg cca gat tta agt caa aaa   1008
Ser Gly Ala Val Ile Val Asp Lys Ser Leu Pro Asp Leu Ser Gln Lys
            325                330                335

atc tta gca acc tta cta aaa tta gaa ttc cca aac gaa aaa gct aaa   1056
Ile Leu Ala Thr Leu Leu Lys Leu Glu Phe Pro Asn Glu Lys Ala Lys
        340                345                350

ctt tta gca caa acc ttt ggt caa gtg cgc caa tct aaa gca gga gat   1104
Leu Leu Ala Gln Thr Phe Gly Gln Val Arg Gln Ser Lys Ala Gly Asp
        355                360                365
```

```
tta ttg cca ctt aca gcg agt aga aca ctt ggg aaa tta aat agt gaa      1152
Leu Leu Pro Leu Thr Ala Ser Arg Thr Leu Gly Lys Leu Asn Ser Glu
        370             375             380

aga ctt gct act ttg aca aaa tta gga tta cca aag gaa aac gcc gct      1200
Arg Leu Ala Thr Leu Thr Lys Leu Gly Leu Pro Lys Glu Asn Ala Ala
385             390             395             400

caa ctt tct atg aac gga ctt act tat cca ttg caa gat gcc gat gtt      1248
Gln Leu Ser Met Asn Gly Leu Thr Tyr Pro Leu Gln Asp Ala Asp Val
            405             410             415

tta acc aaa aat gaa gtt tca aca att cac gaa aga gta aac gaa atc      1296
Leu Thr Lys Asn Glu Val Ser Thr Ile His Glu Arg Val Asn Glu Ile
            420             425             430

aat caa ggc ata caa gca gtg gca aaa caa ttc aac att gca tat gtg      1344
Asn Gln Gly Ile Gln Ala Val Ala Lys Gln Phe Asn Ile Ala Tyr Val
            435             440             445

gac atg aat gcc gaa atg caa aaa ctc act aaa ggc ttt aaa ttc aac      1392
Asp Met Asn Ala Glu Met Gln Lys Leu Thr Lys Gly Phe Lys Phe Asn
        450             455             460

ggg gta gac tac aac gca agt ttt gtg act ggt gga gct ttt tcg ctt      1440
Gly Val Asp Tyr Asn Ala Ser Phe Val Thr Gly Gly Ala Phe Ser Leu
465             470             475             480

gat gga gtg cat tta aac agc cga gga tat gcc cat aca gct aat aca      1488
Asp Gly Val His Leu Asn Ser Arg Gly Tyr Ala His Thr Ala Asn Thr
            485             490             495

ttt att cgt gcc atc aat cag caa tat aag gca agc att ccg ttg gta      1536
Phe Ile Arg Ala Ile Asn Gln Gln Tyr Lys Ala Ser Ile Pro Leu Val
            500             505             510

gat atc aac gct ttc cca ggc aca caa tta cct taa                      1572
Asp Ile Asn Ala Phe Pro Gly Thr Gln Leu Pro
            515             520
```

<210> 4
<211> 523
<212> PRT

<213> *Ornithobacterium rhinotracheale*

<400> 4

Met Lys Ile Asn Tyr Lys Asn Ile Leu Leu Ser Ala Ser Val Leu Phe
1               5                   10                  15

Phe Ala Ala Cys Ser Asp Phe Asp Tyr Asn Val Glu Asn Pro Asn Leu
                20                  25                  30

Thr Lys Gly Glu Ala Asp Phe Ser Lys Tyr Val Ala Leu Gly Asn Ser
        35                  40                  45

Leu Thr Ser Gly Tyr Ser Asp Gly Ala Leu Tyr Arg Ser Ala Gln Glu
        50                  55                  60

Asn Ser Tyr Pro Ala Ile Ile Ala Lys Gln Met Lys Tyr Val Gly Gly
65                  70                  75                  80

Gly Glu Phe Ser Gln Pro Leu Met Lys Asp Asn Ile Gly Gly Phe Ser
                85                  90                  95

Asp Leu Phe Glu Ala Ser Lys His Thr Ala Phe Tyr Gly Lys Leu Glu
                100                 105                 110

Leu Lys Ile Val Asp Gly Ala Pro Thr Pro Val Pro Ser Val Pro Lys
                115                 120                 125

Phe Ser Leu Ala Gln Thr Phe Val Lys Gly Asn Phe Asn Asn Leu Gly
        130                 135                 140

Val Pro Gly Ala Lys Ser Tyr His Leu Leu Ala Gln Gly Tyr Gly Asn
145                 150                 155                 160

Ile Ala Asn Leu Lys Glu Ser Lys Ala Asn Pro Tyr Phe Val Arg Phe

165   170   175

Ala Ser Gln Pro Asn Ala Ser Val Leu Ser Asp Ala Leu Ala Gln Lys  
    180    185    190

Pro Thr Phe Phe Thr Leu Trp Ile Gly Asn Asn Asp Val Leu Gly Tyr  
   195    200    205

Ala Met Asn Gly Ala Ala Ser Thr Asp Arg Lys Gly Asn Pro Asp Val  
   210    215    220

Thr Thr Tyr Asn Ser Asn Asp Leu Ser Asp Ala Asn Leu Val Ala Gly  
225    230    235    240

Ser Ile Gln Lys Leu Val Lys Ala Leu Thr Asp Ser Gly Ala Lys Gly  
    245    250    255

Ala Val Ala Asn Leu Pro Tyr Val Glu Asp Ile Pro Tyr Phe Thr Thr  
    260    265    270

Val Pro Ala Glu Pro Leu Ser Pro Leu Asn Lys Ser Tyr Ala Thr Gln  
    275    280    285

Ile Glu Asn Leu Asn Lys Phe Tyr Ala Ser Leu Asn Lys Val Phe Asp  
   290    295    300

Ala Leu Gly Ala Ser Asp Arg Lys Ile Thr Phe Asn Ala Asp Lys Ala  
305    310    315    320

Ser Gly Ala Val Ile Val Asp Lys Ser Leu Pro Asp Leu Ser Gln Lys  
    325    330    335

Ile Leu Ala Thr Leu Leu Lys Leu Glu Phe Pro Asn Glu Lys Ala Lys  
    340    345    350

```
Leu Leu Ala Gln Thr Phe Gly Gln Val Arg Gln Ser Lys Ala Gly Asp
        355                 360                 365

Leu Leu Pro Leu Thr Ala Ser Arg Thr Leu Gly Lys Leu Asn Ser Glu
        370                 375                 380

Arg Leu Ala Thr Leu Thr Lys Leu Gly Leu Pro Lys Glu Asn Ala Ala
385                 390                 395                 400

Gln Leu Ser Met Asn Gly Leu Thr Tyr Pro Leu Gln Asp Ala Asp Val
                405                 410                 415

Leu Thr Lys Asn Glu Val Ser Thr Ile His Glu Arg Val Asn Glu Ile
                420                 425                 430

Asn Gln Gly Ile Gln Ala Val Ala Lys Gln Phe Asn Ile Ala Tyr Val
                435                 440                 445

Asp Met Asn Ala Glu Met Gln Lys Leu Thr Lys Gly Phe Lys Phe Asn
        450                 455                 460

Gly Val Asp Tyr Asn Ala Ser Phe Val Thr Gly Gly Ala Phe Ser Leu
465                 470                 475                 480

Asp Gly Val His Leu Asn Ser Arg Gly Tyr Ala His Thr Ala Asn Thr
                485                 490                 495

Phe Ile Arg Ala Ile Asn Gln Gln Tyr Lys Ala Ser Ile Pro Leu Val
                500                 505                 510

Asp Ile Asn Ala Phe Pro Gly Thr Gln Leu Pro
                515                 520
```

<210> 5
<211> 1242
<212> DNA
<213> *Ornithobacterium rhinotracheale*

<220>
<221> CDS
<222> (1)..(1242)

<400> 5

```
atg aaa aaa cta tca tat tta tta ttc tca att cca cta ttg tgg gga      48
Met Lys Lys Leu Ser Tyr Leu Leu Phe Ser Ile Pro Leu Leu Trp Gly
1               5                   10                  15


ttg aat gct tgt acc gaa gat ttt gaa ccc aca ttt tca gaa aac gca      96
Leu Asn Ala Cys Thr Glu Asp Phe Glu Pro Thr Phe Ser Glu Asn Ala
                20                  25                  30


act cag cgt tat ata aat gtt cag aac gaa att aca gaa ttt ctt agt     144
Thr Gln Arg Tyr Ile Asn Val Gln Asn Glu Ile Thr Glu Phe Leu Ser
            35                  40                  45


acc cct gat gct gat ttt att tta caa tac ttc cca gat gat aat cag     192
Thr Pro Asp Ala Asp Phe Ile Leu Gln Tyr Phe Pro Asp Asp Asn Gln
        50                  55                  60


tct tat gga gga tat aat tat ttc ttg aaa ttc tca gga aaa gat aag     240
Ser Tyr Gly Gly Tyr Asn Tyr Phe Leu Lys Phe Ser Gly Lys Asp Lys
65                  70                  75                  80


gta agt gcg gaa tca gag acc aat gaa caa gct gta agt tct act ttt     288
Val Ser Ala Glu Ser Glu Thr Asn Glu Gln Ala Val Ser Ser Thr Phe
                85                  90                  95


cga att ctt caa aat gga ggt gca gtt ctt acc ttt gat tta tac aat     336
Arg Ile Leu Gln Asn Gly Gly Ala Val Leu Thr Phe Asp Leu Tyr Asn
            100                 105                 110


gag gag cta cat gaa ttt gca act cct agt cca tca gaa tat cgt gca     384
Glu Glu Leu His Glu Phe Ala Thr Pro Ser Pro Ser Glu Tyr Arg Ala
            115                 120                 125


aaa cga gga gat ttt gaa ttt ttg atc ctt aaa aaa agt aat gac aca     432
```

```
              Lys Arg Gly Asp Phe Glu Phe Leu Ile Leu Lys Lys Ser Asn Asp Thr
                  130             135             140

              ctt tat cta aaa gga aag aaa aca gga aat tac atg aag cta tat aaa       480
              Leu Tyr Leu Lys Gly Lys Lys Thr Gly Asn Tyr Met Lys Leu Tyr Lys
              145             150             155             160

              gca ggg aat att caa gag att aaa agt aac att aga aaa gta gca act       528
              Ala Gly Asn Ile Gln Glu Ile Lys Ser Asn Ile Arg Lys Val Ala Thr
                              165             170             175

              aca att gat agg gta gat ctt cca gct caa ggt act ata ggt aca gag       576
              Thr Ile Asp Arg Val Asp Leu Pro Ala Gln Gly Thr Ile Gly Thr Glu
                          180             185             190

              cct ttg gta ttg tca aca gga gga act aga aat att att ttt agt act       624
              Pro Leu Val Leu Ser Thr Gly Gly Thr Arg Asn Ile Ile Phe Ser Thr
                          195             200             205

              tta aat ggg ggg agt ata gag tct aca gaa gca tcg tat att ttt aca       672
              Leu Asn Gly Gly Ser Ile Glu Ser Thr Glu Ala Ser Tyr Ile Phe Thr
                          210             215             220

              gaa aac gga att aag ttt tac aaa cca gtt gaa att aag ggg aaa gtt       720
              Glu Asn Gly Ile Lys Phe Tyr Lys Pro Val Glu Ile Lys Gly Lys Val
              225             230             235             240

              tac ggt gga tta att ttt gac gaa agt act caa aca tta aag tca gaa       768
              Tyr Gly Gly Leu Ile Phe Asp Glu Ser Thr Gln Thr Leu Lys Ser Glu
                          245             250             255

              gat ggt gta att gta att aat ttg aaa ttt gtt cct atc aac ttt aaa       816
              Asp Gly Val Ile Val Ile Asn Leu Lys Phe Val Pro Ile Asn Phe Lys
                          260             265             270

              tca aaa gct tgg ttt ttg gat atg agc aaa tca gag aat aca tcg gaa       864
              Ser Lys Ala Trp Phe Leu Asp Met Ser Lys Ser Glu Asn Thr Ser Glu
                          275             280             285

              ggt tat aag aaa gcc aga gca ggc gat agt ctt ttg cat ggt atg att       912
              Gly Tyr Lys Lys Ala Arg Ala Gly Asp Ser Leu Leu His Gly Met Ile
                          290             295             300

              cta agt aaa ttt aag tta caa gat ttc tat gtg tta ggt aat ttt aga       960
              Leu Ser Lys Phe Lys Leu Gln Asp Phe Tyr Val Leu Gly Asn Phe Arg
```

```
                305                     310                     315                     320


        gat aat gta gga ttc aat act ttt gtt gag ggc tat aac gga gca ttt        1008
        Asp Asn Val Gly Phe Asn Thr Phe Val Glu Gly Tyr Asn Gly Ala Phe
                        325                     330                     335


        gca att tat ggt tta agt ttc aaa gga gaa gat tca aat cca aat ctt        1056
        Ala Ile Tyr Gly Leu Ser Phe Lys Gly Glu Asp Ser Asn Pro Asn Leu
                        340                     345                     350


        atc cac att gag aaa aca aaa cct gtt gaa ttt gat gct tat ttc aaa        1104
        Ile His Ile Glu Lys Thr Lys Pro Val Glu Phe Asp Ala Tyr Phe Lys
                    355                     360                     365


        tat gtg aat gga gtt tta gat aaa atc act aaa aat tca cct tat att        1152
        Tyr Val Asn Gly Val Leu Asp Lys Ile Thr Lys Asn Ser Pro Tyr Ile
                370                     375                     380


        gta gag gag gtt cag tca gat cct aaa cgt gtg aag cta ata agt aaa        1200
        Val Glu Glu Val Gln Ser Asp Pro Lys Arg Val Lys Leu Ile Ser Lys
            385                     390                     395                     400


        aat gat caa gaa tta tgg ttt att ctt gat ttg ctt aaa tga             1242
        Asn Asp Gln Glu Leu Trp Phe Ile Leu Asp Leu Leu Lys
                        405                     410
```

<210> 6
<211> 413
<212> PRT
<213> *Ornithobacterium rhinotracheale*

<400> 6

```
Met Lys Lys Leu Ser Tyr Leu Leu Phe Ser Ile Pro Leu Leu Trp Gly
1               5                   10                  15

Leu Asn Ala Cys Thr Glu Asp Phe Glu Pro Thr Phe Ser Glu Asn Ala
            20                  25                  30

Thr Gln Arg Tyr Ile Asn Val Gln Asn Glu Ile Thr Glu Phe Leu Ser
            35                  40                  45
```

```
Thr Pro Asp Ala Asp Phe Ile Leu Gln Tyr Phe Pro Asp Asp Asn Gln
    50                  55                  60


Ser Tyr Gly Gly Tyr Asn Tyr Phe Leu Lys Phe Ser Gly Lys Asp Lys
65                  70                  75                  80


Val Ser Ala Glu Ser Glu Thr Asn Glu Gln Ala Val Ser Ser Thr Phe
                85                  90                  95


Arg Ile Leu Gln Asn Gly Gly Ala Val Leu Thr Phe Asp Leu Tyr Asn
            100                 105                 110


Glu Glu Leu His Glu Phe Ala Thr Pro Ser Pro Ser Glu Tyr Arg Ala
            115                 120                 125


Lys Arg Gly Asp Phe Glu Phe Leu Ile Leu Lys Lys Ser Asn Asp Thr
    130                 135                 140


Leu Tyr Leu Lys Gly Lys Lys Thr Gly Asn Tyr Met Lys Leu Tyr Lys
145                 150                 155                 160


Ala Gly Asn Ile Gln Glu Ile Lys Ser Asn Ile Arg Lys Val Ala Thr
                165                 170                 175


Thr Ile Asp Arg Val Asp Leu Pro Ala Gln Gly Thr Ile Gly Thr Glu
            180                 185                 190


Pro Leu Val Leu Ser Thr Gly Gly Thr Arg Asn Ile Ile Phe Ser Thr
            195                 200                 205


Leu Asn Gly Gly Ser Ile Glu Ser Thr Glu Ala Ser Tyr Ile Phe Thr
    210                 215                 220
```

34

Glu Asn Gly Ile Lys Phe Tyr Lys Pro Val Glu Ile Lys Gly Lys Val
225                 230                 235                 240


Tyr Gly Gly Leu Ile Phe Asp Glu Ser Thr Gln Thr Leu Lys Ser Glu
                245                 250                 255


Asp Gly Val Ile Val Ile Asn Leu Lys Phe Val Pro Ile Asn Phe Lys
            260                 265                 270


Ser Lys Ala Trp Phe Leu Asp Met Ser Lys Ser Glu Asn Thr Ser Glu
            275                 280                 285


Gly Tyr Lys Lys Ala Arg Ala Gly Asp Ser Leu Leu His Gly Met Ile
        290                 295                 300


Leu Ser Lys Phe Lys Leu Gln Asp Phe Tyr Val Leu Gly Asn Phe Arg
305                 310                 315                 320


Asp Asn Val Gly Phe Asn Thr Phe Val Glu Gly Tyr Asn Gly Ala Phe
                325                 330                 335


Ala Ile Tyr Gly Leu Ser Phe Lys Gly Glu Asp Ser Asn Pro Asn Leu
                340                 345                 350


Ile His Ile Glu Lys Thr Lys Pro Val Glu Phe Asp Ala Tyr Phe Lys
            355                 360                 365


Tyr Val Asn Gly Val Leu Asp Lys Ile Thr Lys Asn Ser Pro Tyr Ile
        370                 375                 380


Val Glu Glu Val Gln Ser Asp Pro Lys Arg Val Lys Leu Ile Ser Lys
385                 390                 395                 400


Asn Asp Gln Glu Leu Trp Phe Ile Leu Asp Leu Leu Lys

35

405                    410

<210> 7
<211> 1023
<212> DNA
<213> *Ornithobacterium rhinotracheale*

<220>
<221> CDS
<222> (1)..(1023)

<400> 7

```
atg aaa gat ata ttt gaa tat aca ctt cta gca tta ggt ggt ttg cta      48
Met Lys Asp Ile Phe Glu Tyr Thr Leu Leu Ala Leu Gly Gly Leu Leu
1               5                   10                  15

ctc acc aat tgc tat gat agc gat gag att gaa gta att aaa ttt gat      96
Leu Thr Asn Cys Tyr Asp Ser Asp Glu Ile Glu Val Ile Lys Phe Asp
                20                  25                  30

gat tct ttt act cca gct ccg ccc acc gaa aaa aaa aga gac act ccg     144
Asp Ser Phe Thr Pro Ala Pro Pro Thr Glu Lys Lys Arg Asp Thr Pro
                35                  40                  45

cta ata aat tta tta gat gat ttt gta ttc ttt aaa aaa gat gta gta     192
Leu Ile Asn Leu Leu Asp Asp Phe Val Phe Phe Lys Lys Asp Val Val
        50                  55                  60

aca att ccg gta gat aaa gac aat tta gcc acc aat aat gtc atc agt     240
Thr Ile Pro Val Asp Lys Asp Asn Leu Ala Thr Asn Asn Val Ile Ser
65                  70                  75                  80

ggt gaa gtc ttt aca aat aga aaa atg tct gaa aat ttt gag tat cag     288
Gly Glu Val Phe Thr Asn Arg Lys Met Ser Glu Asn Phe Glu Tyr Gln
                85                  90                  95

ctt gaa tta gac caa gat tgg att agt agc aat ccg gac tta caa gcc     336
Leu Glu Leu Asp Gln Asp Trp Ile Ser Ser Asn Pro Asp Leu Gln Ala
                100                 105                 110

att cca aac gga gct ttt aca atc tct gga caa aca ctc aac aaa gat     384
Ile Pro Asn Gly Ala Phe Thr Ile Ser Gly Gln Thr Leu Asn Lys Asp
                115                 120                 125
```

36

```
gaa aga aat ggt act ttc aaa att cag ctt aat gca gag gtg gcg aaa        432
Glu Arg Asn Gly Thr Phe Lys Ile Gln Leu Asn Ala Glu Val Ala Lys
    130             135             140

gag cta gga ggc acc tac tat ctc ccg cta aaa ttg gtt tct aaa aat        480
Glu Leu Gly Gly Thr Tyr Tyr Leu Pro Leu Lys Leu Val Ser Lys Asn
145             150             155             160

gat aat tta aac att tta aag gga tat gaa agt ggc gtt ttt aag cta        528
Asp Asn Leu Asn Ile Leu Lys Gly Tyr Glu Ser Gly Val Phe Lys Leu
                165             170             175

gta ttc aaa aaa tcg tat cca atc cca gaa ggt aac aat gtt gaa gga        576
Val Phe Lys Lys Ser Tyr Pro Ile Pro Glu Gly Asn Asn Val Glu Gly
                180             185             190

aaa aaa gga tat tat ttt gat ggt tta ggc aat aat ata cct aga aca        624
Lys Lys Gly Tyr Tyr Phe Asp Gly Leu Gly Asn Asn Ile Pro Arg Thr
            195             200             205

gat tta tcg ttt aat tca aat tac gcc ccc gat cat ctt ttt aaa tta        672
Asp Leu Ser Phe Asn Ser Asn Tyr Ala Pro Asp His Leu Phe Lys Leu
        210             215             220

aat gat gga aac caa caa ggg gct aat tgg tgg gca gac act gat gat        720
Asn Asp Gly Asn Gln Gln Gly Ala Asn Trp Trp Ala Asp Thr Asp Asp
225             230             235             240

aac aca aca tat ctt gat gta aaa ttc cct att aat aca ata aaa gct        768
Asn Thr Thr Tyr Leu Asp Val Lys Phe Pro Ile Asn Thr Ile Lys Ala
                245             250             255

ata aaa tta tac act aaa agc tat tgg caa aat gct gta ggc agt gta        816
Ile Lys Leu Tyr Thr Lys Ser Tyr Trp Gln Asn Ala Val Gly Ser Val
            260             265             270

aaa att gaa gtt tct aat gat aat ggc aat act tgg aaa gaa cag gga        864
Lys Ile Glu Val Ser Asn Asp Asn Gly Asn Thr Trp Lys Glu Gln Gly
        275             280             285

att gct aac ttt ggg caa tat tca aca gtg tct act att gta ttc act        912
Ile Ala Asn Phe Gly Gln Tyr Ser Thr Val Ser Thr Ile Val Phe Thr
    290             295             300
```

```
caa cca att gac att aat gct gtc aga ata tct aac ttc act aga ggg        960
Gln Pro Ile Asp Ile Asn Ala Val Arg Ile Ser Asn Phe Thr Arg Gly
305             310             315             320


gga agt agt aat ttc att aac att aac gag gtg gaa gta ttc aaa ata       1008
Gly Ser Ser Asn Phe Ile Asn Ile Asn Glu Val Glu Val Phe Lys Ile
                325             330             335


cca agt gaa gaa taa                                                    1023
Pro Ser Glu Glu
            340
```

<210> 8
<211> 340
<212> PRT
<213> *Ornithobacterium rhinotracheale*

<400> 8

```
Met Lys Asp Ile Phe Glu Tyr Thr Leu Leu Ala Leu Gly Gly Leu Leu
1               5                   10                  15


Leu Thr Asn Cys Tyr Asp Ser Asp Glu Ile Glu Val Ile Lys Phe Asp
                20                  25                  30


Asp Ser Phe Thr Pro Ala Pro Pro Thr Glu Lys Lys Arg Asp Thr Pro
            35                  40                  45


Leu Ile Asn Leu Leu Asp Asp Phe Val Phe Phe Lys Lys Asp Val Val
        50                  55                  60


Thr Ile Pro Val Asp Lys Asp Asn Leu Ala Thr Asn Asn Val Ile Ser
65                  70                  75                  80


Gly Glu Val Phe Thr Asn Arg Lys Met Ser Glu Asn Phe Glu Tyr Gln
                85                  90                  95


Leu Glu Leu Asp Gln Asp Trp Ile Ser Ser Asn Pro Asp Leu Gln Ala
```

                    100                     105                     110


Ile Pro Asn Gly Ala Phe Thr Ile Ser Gly Gln Thr Leu Asn Lys Asp
        115                 120                 125


Glu Arg Asn Gly Thr Phe Lys Ile Gln Leu Asn Ala Glu Val Ala Lys
        130                 135                 140


Glu Leu Gly Gly Thr Tyr Tyr Leu Pro Leu Lys Leu Val Ser Lys Asn
145                 150                 155                 160


Asp Asn Leu Asn Ile Leu Lys Gly Tyr Glu Ser Gly Val Phe Lys Leu
                165                 170                 175


Val Phe Lys Lys Ser Tyr Pro Ile Pro Glu Gly Asn Asn Val Glu Gly
            180                 185                 190


Lys Lys Gly Tyr Tyr Phe Asp Gly Leu Gly Asn Asn Ile Pro Arg Thr
        195                 200                 205


Asp Leu Ser Phe Asn Ser Asn Tyr Ala Pro Asp His Leu Phe Lys Leu
    210                 215                 220


Asn Asp Gly Asn Gln Gln Gly Ala Asn Trp Trp Ala Asp Thr Asp Asp
225                 230                 235                 240


Asn Thr Thr Tyr Leu Asp Val Lys Phe Pro Ile Asn Thr Ile Lys Ala
                245                 250                 255


Ile Lys Leu Tyr Thr Lys Ser Tyr Trp Gln Asn Ala Val Gly Ser Val
            260                 265                 270


Lys Ile Glu Val Ser Asn Asp Asn Gly Asn Thr Trp Lys Glu Gln Gly
        275                 280                 285

```
Ile Ala Asn Phe Gly Gln Tyr Ser Thr Val Ser Thr Ile Val Phe Thr
    290                 295                 300

Gln Pro Ile Asp Ile Asn Ala Val Arg Ile Ser Asn Phe Thr Arg Gly
305                 310                 315                 320

Gly Ser Ser Asn Phe Ile Asn Ile Asn Glu Val Glu Val Phe Lys Ile
                325                 330                 335

Pro Ser Glu Glu
            340
```

<210> 9
<211> 1230
<212> DNA
<213> *Ornithobacterium rhinotracheale*

<220>
<221> CDS
<222> (1)..(1230)

<400> 9

```
atg att aaa aaa gta ttt tta tcg ttt gtg ctc atg gca agc aca ggc        48
Met Ile Lys Lys Val Phe Leu Ser Phe Val Leu Met Ala Ser Thr Gly
1               5                   10                  15

att tta tgg gca ggc gga tac cga gtt tcg ctg caa ggt gta aga caa        96
Ile Leu Trp Ala Gly Gly Tyr Arg Val Ser Leu Gln Gly Val Arg Gln
            20                  25                  30

gcc gcc atg ggg gca caa ggt gta gca ctt tct cac gat gcg agt gtg       144
Ala Ala Met Gly Ala Gln Gly Val Ala Leu Ser His Asp Ala Ser Val
            35                  40                  45

gca ttt ttc aac ccc gca gca ttg gct ttt gta gat gat aaa tta agt       192
Ala Phe Phe Asn Pro Ala Ala Leu Ala Phe Val Asp Asp Lys Leu Ser
        50                  55                  60
```

```
att gct gtg gga ggt ttc gga att ggg att acc gca aaa tac caa aac     240
Ile Ala Val Gly Gly Phe Gly Ile Gly Ile Thr Ala Lys Tyr Gln Asn
65              70              75              80

cgc gaa acg ctc tat aaa gcc gaa acc gac aat ccg ctg ggg aca cca     288
Arg Glu Thr Leu Tyr Lys Ala Glu Thr Asp Asn Pro Leu Gly Thr Pro
                85              90              95

ctt tat ctt gct aca agc tat aag cct acg gaa aaa cta gcc tta ggc     336
Leu Tyr Leu Ala Thr Ser Tyr Lys Pro Thr Glu Lys Leu Ala Leu Gly
            100             105             110

gtg agc gta acc act ccg ttt ggg agc acc gta gac tgg gga gat aaa     384
Val Ser Val Thr Thr Pro Phe Gly Ser Thr Val Asp Trp Gly Asp Lys
            115             120             125

tgg gct gga cgc tac atc att gat aga att gcc ctc aaa tcg ttt ttt     432
Trp Ala Gly Arg Tyr Ile Ile Asp Arg Ile Ala Leu Lys Ser Phe Phe
        130             135             140

att cag ccc acg gca gcg tat aaa gta acc gat tgg ctc tct gtg ggg     480
Ile Gln Pro Thr Ala Ala Tyr Lys Val Thr Asp Trp Leu Ser Val Gly
145             150             155             160

gct ggt gcc atc atc gct cga ggc aat gta aac att aag cgt gca ata     528
Ala Gly Ala Ile Ile Ala Arg Gly Asn Val Asn Ile Lys Arg Ala Ile
            165             170             175

tct cta ggc aac caa gat gcg ggg cta gaa atc gac aaa aaa gga gct     576
Ser Leu Gly Asn Gln Asp Ala Gly Leu Glu Ile Asp Lys Lys Gly Ala
            180             185             190

cac gga aca ggg ttt aat gta ggg gtt tat gcc aaa cca aat gat aaa     624
His Gly Thr Gly Phe Asn Val Gly Val Tyr Ala Lys Pro Asn Asp Lys
            195             200             205

tta aat ata gga att gct tac cga tca gaa gtg aag atg aaa gcg gac     672
Leu Asn Ile Gly Ile Ala Tyr Arg Ser Glu Val Lys Met Lys Ala Asp
        210             215             220

aaa ggt gat gct gtt ttc aaa aat tta cca agt atc gta aag ggc aaa     720
Lys Gly Asp Ala Val Phe Lys Asn Leu Pro Ser Ile Val Lys Gly Lys
225             230             235             240

atg cct ttt tcg gct aaa tat ttt gat gct caa tta cct cta cca gca     768
```

42

```
          Met Pro Phe Ser Ala Lys Tyr Phe Asp Ala Gln Leu Pro Leu Pro Ala
                          245             250             255

          gaa ctt tta att ggg gcg aac tat aaa gta aca cca aaa ttg ctc gta      816
          Glu Leu Leu Ile Gly Ala Asn Tyr Lys Val Thr Pro Lys Leu Leu Val
                          260             265             270

          ggg gca gaa att ggg gct gta aaa tgg aac gcc tac gaa aca tta aat      864
          Gly Ala Glu Ile Gly Ala Val Lys Trp Asn Ala Tyr Glu Thr Leu Asn
                          275             280             285

          att aaa ctt tat aac aac gaa gag gaa tac aac aat act tct aac aaa      912
          Ile Lys Leu Tyr Asn Asn Glu Glu Glu Tyr Asn Asn Thr Ser Asn Lys
                  290             295             300

          aat tac aaa aac aca tta aat tat agt atc ggg gct gaa tat tta atc      960
          Asn Tyr Lys Asn Thr Leu Asn Tyr Ser Ile Gly Ala Glu Tyr Leu Ile
          305             310             315             320

          aat cca aaa gct gcc tta cgc tta ggg tat aaa ttc gac aaa tcg cct     1008
          Asn Pro Lys Ala Ala Leu Arg Leu Gly Tyr Lys Phe Asp Lys Ser Pro
                          325             330             335

          tcg cca gct gat tcg ttt aac cca gag acc cca acc att aat tat cac     1056
          Ser Pro Ala Asp Ser Phe Asn Pro Glu Thr Pro Thr Ile Asn Tyr His
                          340             345             350

          gca ttt aca act gga ttt gga tat gaa ttc gag aga ttt cgt gta gat     1104
          Ala Phe Thr Thr Gly Phe Gly Tyr Glu Phe Glu Arg Phe Arg Val Asp
                          355             360             365

          gcc atg gcg gaa tat tta cta gga aac gaa aga agc ttc cac aat aca     1152
          Ala Met Ala Glu Tyr Leu Leu Gly Asn Glu Arg Ser Phe His Asn Thr
                  370             375             380

          caa tat aac ttt ggg ggc gac atc aac act ggt ggc tat gtg ttt ggt     1200
          Gln Tyr Asn Phe Gly Gly Asp Ile Asn Thr Gly Gly Tyr Val Phe Gly
          385             390             395             400

          cta ggt tta tcg tat aga ctt gac aaa taa                             1230
          Leu Gly Leu Ser Tyr Arg Leu Asp Lys
                          405
```

<210> 10

<211> 409
<212> PRT
<213> *Ornithobacterium rhinotracheale*

<400> 10

```
Met Ile Lys Lys Val Phe Leu Ser Phe Val Leu Met Ala Ser Thr Gly
1               5                   10                  15

Ile Leu Trp Ala Gly Gly Tyr Arg Val Ser Leu Gln Gly Val Arg Gln
            20                  25                  30

Ala Ala Met Gly Ala Gln Gly Val Ala Leu Ser His Asp Ala Ser Val
            35                  40                  45

Ala Phe Phe Asn Pro Ala Ala Leu Ala Phe Val Asp Asp Lys Leu Ser
        50                  55                  60

Ile Ala Val Gly Gly Phe Gly Ile Gly Ile Thr Ala Lys Tyr Gln Asn
65                  70                  75                  80

Arg Glu Thr Leu Tyr Lys Ala Glu Thr Asp Asn Pro Leu Gly Thr Pro
                85                  90                  95

Leu Tyr Leu Ala Thr Ser Tyr Lys Pro Thr Glu Lys Leu Ala Leu Gly
                100                 105                 110

Val Ser Val Thr Thr Pro Phe Gly Ser Thr Val Asp Trp Gly Asp Lys
                115                 120                 125

Trp Ala Gly Arg Tyr Ile Ile Asp Arg Ile Ala Leu Lys Ser Phe Phe
                130                 135                 140

Ile Gln Pro Thr Ala Ala Tyr Lys Val Thr Asp Trp Leu Ser Val Gly
145                 150                 155                 160
```

Ala Gly Ala Ile Ile Ala Arg Gly Asn Val Asn Ile Lys Arg Ala Ile
165 170 175

Ser Leu Gly Asn Gln Asp Ala Gly Leu Glu Ile Asp Lys Lys Gly Ala
180 185 190

His Gly Thr Gly Phe Asn Val Gly Val Tyr Ala Lys Pro Asn Asp Lys
195 200 205

Leu Asn Ile Gly Ile Ala Tyr Arg Ser Glu Val Lys Met Lys Ala Asp
210 215 220

Lys Gly Asp Ala Val Phe Lys Asn Leu Pro Ser Ile Val Lys Gly Lys
225 230 235 240

Met Pro Phe Ser Ala Lys Tyr Phe Asp Ala Gln Leu Pro Leu Pro Ala
245 250 255

Glu Leu Leu Ile Gly Ala Asn Tyr Lys Val Thr Pro Lys Leu Leu Val
260 265 270

Gly Ala Glu Ile Gly Ala Val Lys Trp Asn Ala Tyr Glu Thr Leu Asn
275 280 285

Ile Lys Leu Tyr Asn Asn Glu Glu Glu Tyr Asn Asn Thr Ser Asn Lys
290 295 300

Asn Tyr Lys Asn Thr Leu Asn Tyr Ser Ile Gly Ala Glu Tyr Leu Ile
305 310 315 320

Asn Pro Lys Ala Ala Leu Arg Leu Gly Tyr Lys Phe Asp Lys Ser Pro
325 330 335

```
Ser Pro Ala Asp Ser Phe Asn Pro Glu Thr Pro Thr Ile Asn Tyr His
            340                 345             350


Ala Phe Thr Thr Gly Phe Gly Tyr Glu Phe Glu Arg Phe Arg Val Asp
        355                 360                 365


Ala Met Ala Glu Tyr Leu Leu Gly Asn Glu Arg Ser Phe His Asn Thr
    370                 375                 380


Gln Tyr Asn Phe Gly Gly Asp Ile Asn Thr Gly Gly Tyr Val Phe Gly
385                 390                 395                 400


Leu Gly Leu Ser Tyr Arg Leu Asp Lys
                405
```

<210> 11
<211> 1140
<212> DNA
<213> *Ornithobacterium rhinotracheale*

<220>
<221> CDS
<222> (1)..(1140)

<400> 11

```
atg aag aaa ata ctt tta gca att agc ttt tcg tct ttt gtt tta agc      48
Met Lys Lys Ile Leu Leu Ala Ile Ser Phe Ser Ser Phe Val Leu Ser
1               5                   10                  15

tgt agc agt gat gat tac act cca gcc aca cct aaa gaa aca gaa aag      96
Cys Ser Ser Asp Asp Tyr Thr Pro Ala Thr Pro Lys Glu Thr Glu Lys
                20                  25                  30

cct aag gaa gag gct gtg gtt cca aat aag cca gat gaa cca aag gct     144
Pro Lys Glu Glu Ala Val Val Pro Asn Lys Pro Asp Glu Pro Lys Ala
            35                  40                  45

gat gat gga aac gaa aat cca gaa aac act gga gat gaa gag aat gga     192
Asp Asp Gly Asn Glu Asn Pro Glu Asn Thr Gly Asp Glu Glu Asn Gly
```

46

```
                50                    55                    60

gat aat aca aac tcc gtt gtc ggg aag cct gat gat ttc cac atg ggg      240
Asp Asn Thr Asn Ser Val Val Gly Lys Pro Asp Asp Phe His Met Gly
65                  70                  75                  80

aat cgc tct tat gct agc tgg aaa gaa gat gtg gat tat atc gga ggt      288
Asn Arg Ser Tyr Ala Ser Trp Lys Glu Asp Val Asp Tyr Ile Gly Gly
                85                  90                  95

ttt gat att gaa act ctt tta agt ggg gct gat aat caa aaa tat gat      336
Phe Asp Ile Glu Thr Leu Leu Ser Gly Ala Asp Asn Gln Lys Tyr Asp
                    100                 105                 110

gcg gct tat ttt agc caa ttt atc aag ata ttc tca tct agt cca aac      384
Ala Ala Tyr Phe Ser Gln Phe Ile Lys Ile Phe Ser Ser Ser Pro Asn
                115                 120                 125

gga aac aat ttc tac act ttt cag gca gaa gac ttt aaa gat gtc gag      432
Gly Asn Asn Phe Tyr Thr Phe Gln Ala Glu Asp Phe Lys Asp Val Glu
                130                 135                 140

att aaa gac tta aag ttt gat att ggt aga aat gta att aca ttt aaa      480
Ile Lys Asp Leu Lys Phe Asp Ile Gly Arg Asn Val Ile Thr Phe Lys
145                 150                 155                 160

act agc tac aaa ggc gta aaa agt gaa att aca tct tct tta aaa ttt      528
Thr Ser Tyr Lys Gly Val Lys Ser Glu Ile Thr Ser Ser Leu Lys Phe
                165                 170                 175

gat ttg gct aat ttt tat gat cga aaa atc aaa ata aac gaa gat ttc      576
Asp Leu Ala Asn Phe Tyr Asp Arg Lys Ile Lys Ile Asn Glu Asp Phe
                180                 185                 190

gtt gca tct cac tac atg aga ggg att tat gag gag ctt gga ggt ttt      624
Val Ala Ser His Tyr Met Arg Gly Ile Tyr Glu Glu Leu Gly Gly Phe
                195                 200                 205

atc ggg aat tta tta aac tac gac gat gag aaa tac aat cta gag tta      672
Ile Gly Asn Leu Leu Asn Tyr Asp Asp Glu Lys Tyr Asn Leu Glu Leu
                210                 215                 220

gcg ggg tca aaa aac aaa gat gaa tcc aat aac tct tta ggt ttt agc      720
Ala Gly Ser Lys Asn Lys Asp Glu Ser Asn Asn Ser Leu Gly Phe Ser
225                 230                 235                 240
```

```
att cgc gta aca gat aaa aaa gat aag tat ata aca acg gtt tat aaa        768
Ile Arg Val Thr Asp Lys Lys Asp Lys Tyr Ile Thr Thr Val Tyr Lys
                245                 250                 255

aac atc tca gga ttt agg cct ctt tct agt ctg cag gag gag ctt tcc        816
Asn Ile Ser Gly Phe Arg Pro Leu Ser Ser Leu Gln Glu Glu Leu Ser
                260                 265                 270

att gct cct act tac gaa ttg cga gag aaa atc aag gag aaa ata gat        864
Ile Ala Pro Thr Tyr Glu Leu Arg Glu Lys Ile Lys Glu Lys Ile Asp
                275                 280                 285

aga aat aaa aga aac att agc cta ttg gag cta tta aaa cca tcg gta        912
Arg Asn Lys Arg Asn Ile Ser Leu Leu Glu Leu Leu Lys Pro Ser Val
        290                 295                 300

aac gaa tgg atg aag tct gcc gat ttc tac ttt aat aac act gat ttg        960
Asn Glu Trp Met Lys Ser Ala Asp Phe Tyr Phe Asn Asn Thr Asp Leu
305                 310                 315                 320

gaa tgg aga gga gat cat tat tca gct aga ggg ttt tta gat ttg tat       1008
Glu Trp Arg Gly Asp His Tyr Ser Ala Arg Gly Phe Leu Asp Leu Tyr
                325                 330                 335

ata ggt tcg cct aga ttt gag ctg att tta gca aca aaa gaa gac aat       1056
Ile Gly Ser Pro Arg Phe Glu Leu Ile Leu Ala Thr Lys Glu Asp Asn
                340                 345                 350

tgg ttg att ttg aaa gtg aaa gtg gtt cag ata aat gaa gtg cct acc       1104
Trp Leu Ile Leu Lys Val Lys Val Val Gln Ile Asn Glu Val Pro Thr
                355                 360                 365

gat ttg gtg tat agc tta aga gtt tca att aac taa                       1140
Asp Leu Val Tyr Ser Leu Arg Val Ser Ile Asn
                370                 375
```

<210> 12
<211> 379
<212> PRT
<213> *Ornithobacterium rhinotracheale*

<400> 12

48

```
Met Lys Lys Ile Leu Leu Ala Ile Ser Phe Ser Ser Phe Val Leu Ser
1               5               10              15


Cys Ser Ser Asp Asp Tyr Thr Pro Ala Thr Pro Lys Glu Thr Glu Lys
             20              25              30


Pro Lys Glu Glu Ala Val Val Pro Asn Lys Pro Asp Glu Pro Lys Ala
             35              40              45


Asp Asp Gly Asn Glu Asn Pro Glu Asn Thr Gly Asp Glu Glu Asn Gly
          50              55              60


Asp Asn Thr Asn Ser Val Val Gly Lys Pro Asp Asp Phe His Met Gly
65              70              75              80


Asn Arg Ser Tyr Ala Ser Trp Lys Glu Asp Val Asp Tyr Ile Gly Gly
             85              90              95


Phe Asp Ile Glu Thr Leu Leu Ser Gly Ala Asp Asn Gln Lys Tyr Asp
             100             105             110


Ala Ala Tyr Phe Ser Gln Phe Ile Lys Ile Phe Ser Ser Ser Pro Asn
             115             120             125


Gly Asn Asn Phe Tyr Thr Phe Gln Ala Glu Asp Phe Lys Asp Val Glu
             130             135             140


Ile Lys Asp Leu Lys Phe Asp Ile Gly Arg Asn Val Ile Thr Phe Lys
145             150             155             160


Thr Ser Tyr Lys Gly Val Lys Ser Glu Ile Thr Ser Ser Leu Lys Phe
             165             170             175


Asp Leu Ala Asn Phe Tyr Asp Arg Lys Ile Lys Ile Asn Glu Asp Phe
```

180                    185                    190

Val Ala Ser His Tyr Met Arg Gly Ile Tyr Glu Glu Leu Gly Gly Phe
        195                    200                    205

Ile Gly Asn Leu Leu Asn Tyr Asp Asp Glu Lys Tyr Asn Leu Glu Leu
        210                    215                    220

Ala Gly Ser Lys Asn Lys Asp Glu Ser Asn Asn Ser Leu Gly Phe Ser
225                    230                    235                    240

Ile Arg Val Thr Asp Lys Lys Asp Lys Tyr Ile Thr Thr Val Tyr Lys
                245                    250                    255

Asn Ile Ser Gly Phe Arg Pro Leu Ser Ser Leu Gln Glu Glu Leu Ser
                260                    265                    270

Ile Ala Pro Thr Tyr Glu Leu Arg Glu Lys Ile Lys Glu Lys Ile Asp
                275                    280                    285

Arg Asn Lys Arg Asn Ile Ser Leu Leu Glu Leu Leu Lys Pro Ser Val
        290                    295                    300

Asn Glu Trp Met Lys Ser Ala Asp Phe Tyr Phe Asn Asn Thr Asp Leu
        305                    310                    315                    320

Glu Trp Arg Gly Asp His Tyr Ser Ala Arg Gly Phe Leu Asp Leu Tyr
                325                    330                    335

Ile Gly Ser Pro Arg Phe Glu Leu Ile Leu Ala Thr Lys Glu Asp Asn
                340                    345                    350

Trp Leu Ile Leu Lys Val Lys Val Val Gln Ile Asn Glu Val Pro Thr
                355                    360                    365

```
        Asp Leu Val Tyr Ser Leu Arg Val Ser Ile Asn
            370                 375
```

<210> 13
<211> 918
<212> DNA
<213> *Ornithobacterium rhinotracheale*

<220>
<221> CDS
<222> (1)..(918)

<400> 13

```
        atg att gta aaa gac ttt tca gac tat aca ttc cga tgt tct caa tta      48
        Met Ile Val Lys Asp Phe Ser Asp Tyr Thr Phe Arg Cys Ser Gln Leu
        1               5                   10                  15


        ggt aag tta atg gtt ggt gtc aag cca cca tta acc cct aat caa gag      96
        Gly Lys Leu Met Val Gly Val Lys Pro Pro Leu Thr Pro Asn Gln Glu
                    20                  25                  30


        aag ttg ctc aca gac tta gag ggc aaa atg gaa gct ggg acc att acc     144
        Lys Leu Leu Thr Asp Leu Glu Gly Lys Met Glu Ala Gly Thr Ile Thr
                35                  40                  45


        aaa aag caa atc atc act tat ggt gaa ttg ctt tcc aag aaa aac caa     192
        Lys Lys Gln Ile Ile Thr Tyr Gly Glu Leu Leu Ser Lys Lys Asn Gln
            50                  55                  60


        aag ctt gaa tta tct gca agt gta aag tct tac tta gcc gac att cat     240
        Lys Leu Glu Leu Ser Ala Ser Val Lys Ser Tyr Leu Ala Asp Ile His
        65                  70                  75                  80


        aaa gaa gtc ttt ttt ggt cgt gat aag gaa ttg acc aat aaa tat cta     288
        Lys Glu Val Phe Phe Gly Arg Asp Lys Glu Leu Thr Asn Lys Tyr Leu
                        85                  90                  95


        tca aaa ggc att caa gta gaa gaa aag agc ata acg ctc tat tcc gat     336
        Ser Lys Gly Ile Gln Val Glu Glu Lys Ser Ile Thr Leu Tyr Ser Asp
                        100                 105                 110
```

```
gtc tgt aac aag tta ttc cta aag aat aaa aag ttt tac aaa aac gat       384
Val Cys Asn Lys Leu Phe Leu Lys Asn Lys Lys Phe Tyr Lys Asn Asp
        115                 120                 125

ttt att caa ggt acg cca gat aac acg caa gac aaa atc aga gat atc       432
Phe Ile Gln Gly Thr Pro Asp Asn Thr Gln Asp Lys Ile Arg Asp Ile
        130                 135                 140

aaa agt agt tgg gac ttc tca acc ttt cct cta cac gcc gat gaa acg       480
Lys Ser Ser Trp Asp Phe Ser Thr Phe Pro Leu His Ala Asp Glu Thr
145                 150                 155                 160

cca acc aaa gac tat gaa tgg cag ttg caa ggt tat atg gaa tta aca       528
Pro Thr Lys Asp Tyr Glu Trp Gln Leu Gln Gly Tyr Met Glu Leu Thr
                165                 170                 175

ggc tta aaa gaa gct gag ttg att tat tgc ttg gtt gat acg cct cat       576
Gly Leu Lys Glu Ala Glu Leu Ile Tyr Cys Leu Val Asp Thr Pro His
        180                 185                 190

aaa att gta gaa gat gaa atc cga aga atg gac tgg aag cat aat tta       624
Lys Ile Val Glu Asp Glu Ile Arg Arg Met Asp Trp Lys His Asn Leu
        195                 200                 205

ctt gac att aac ggc gaa gtg aga gcc gag aca aga gat tta gta gtt       672
Leu Asp Ile Asn Gly Glu Val Arg Ala Glu Thr Arg Asp Leu Val Val
        210                 215                 220

gag att gtg tct aac tta att tat acc aag caa ggc ttg gaa gac ttt       720
Glu Ile Val Ser Asn Leu Ile Tyr Thr Lys Gln Gly Leu Glu Asp Phe
225                 230                 235                 240

tgt cag cag tcc gca gtc ata aac aaa gat tgg ttc acg gac ttt gag       768
Cys Gln Gln Ser Ala Val Ile Asn Lys Asp Trp Phe Thr Asp Phe Glu
                245                 250                 255

gaa ata cca caa gaa ttg aga att aaa gtt ttt cac ttt gag cat caa       816
Glu Ile Pro Gln Glu Leu Arg Ile Lys Val Phe His Phe Glu His Gln
                260                 265                 270

aaa gag atg att agc gca ctc tac gag caa ata gga aga tgt aga gcg       864
Lys Glu Met Ile Ser Ala Leu Tyr Glu Gln Ile Gly Arg Cys Arg Ala
                275                 280                 285

cat tta aac gac ttg acc atg aaa atg gca aca cga tta gaa tta ata       912
```

```
    His Leu Asn Asp Leu Thr Met Lys Met Ala Thr Arg Leu Glu Leu Ile
        290                 295                 300


    gca taa                                                     918

    Ala
    305
```

<210> 14
<211> 305
<212> PRT
<213> *Ornithobacterium rhinotracheale*

<400> 14

```
Met Ile Val Lys Asp Phe Ser Asp Tyr Thr Phe Arg Cys Ser Gln Leu
1               5                   10                  15


Gly Lys Leu Met Val Gly Val Lys Pro Pro Leu Thr Pro Asn Gln Glu
            20                  25                  30


Lys Leu Leu Thr Asp Leu Glu Gly Lys Met Glu Ala Gly Thr Ile Thr
            35                  40                  45


Lys Lys Gln Ile Ile Thr Tyr Gly Glu Leu Leu Ser Lys Lys Asn Gln
        50                  55                  60


Lys Leu Glu Leu Ser Ala Ser Val Lys Ser Tyr Leu Ala Asp Ile His
65                  70                  75                  80


Lys Glu Val Phe Phe Gly Arg Asp Lys Glu Leu Thr Asn Lys Tyr Leu
                85                  90                  95


Ser Lys Gly Ile Gln Val Glu Glu Lys Ser Ile Thr Leu Tyr Ser Asp
            100                 105                 110


Val Cys Asn Lys Leu Phe Leu Lys Asn Lys Lys Phe Tyr Lys Asn Asp
            115                 120                 125
```

```
Phe Ile Gln Gly Thr Pro Asp Asn Thr Gln Asp Lys Ile Arg Asp Ile
    130                 135             140


Lys Ser Ser Trp Asp Phe Ser Thr Phe Pro Leu His Ala Asp Glu Thr
145                 150             155                 160


Pro Thr Lys Asp Tyr Glu Trp Gln Leu Gln Gly Tyr Met Glu Leu Thr
                165             170             175


Gly Leu Lys Glu Ala Glu Leu Ile Tyr Cys Leu Val Asp Thr Pro His
                180             185             190


Lys Ile Val Glu Asp Glu Ile Arg Arg Met Asp Trp Lys His Asn Leu
            195             200             205


Leu Asp Ile Asn Gly Glu Val Arg Ala Glu Thr Arg Asp Leu Val Val
    210             215             220


Glu Ile Val Ser Asn Leu Ile Tyr Thr Lys Gln Gly Leu Glu Asp Phe
225                 230             235                 240


Cys Gln Gln Ser Ala Val Ile Asn Lys Asp Trp Phe Thr Asp Phe Glu
                245             250             255


Glu Ile Pro Gln Glu Leu Arg Ile Lys Val Phe His Phe Glu His Gln
                260             265             270


Lys Glu Met Ile Ser Ala Leu Tyr Glu Gln Ile Gly Arg Cys Arg Ala
            275             280             285


His Leu Asn Asp Leu Thr Met Lys Met Ala Thr Arg Leu Glu Leu Ile
    290             295             300
```

55

```
                Ala
                305


        <210> 15
        <211> 888
        <212> DNA
        <213> Ornithobacterium rhinotracheale

        <220>
        <221> CDS
        <222> (1)..(888)

        <400> 15


        atg aac gaa tta gcg aaa aac gac atc aag tca ttg tta aaa agt gcc        48
        Met Asn Glu Leu Ala Lys Asn Asp Ile Lys Ser Leu Leu Lys Ser Ala
        1               5                   10                  15


        gac atc aac aaa aga ttt gag caa ttg ctc ggc aaa aaa gca caa ggc        96
        Asp Ile Asn Lys Arg Phe Glu Gln Leu Leu Gly Lys Lys Ala Gln Gly
                        20                  25                  30


        ttt atc tca tca gtc ttg cag acg gca caa aat aac aga ttg tta gcg       144
        Phe Ile Ser Ser Val Leu Gln Thr Ala Gln Asn Asn Arg Leu Leu Ala
                        35                  40                  45


        aca gcc gac cca aag acc att cta aac gct gca gta aca gcc gcg act       192
        Thr Ala Asp Pro Lys Thr Ile Leu Asn Ala Ala Val Thr Ala Ala Thr
                50                  55                  60


        tta gat ttg cca att aat cag aat tta ggt tac gcc tac atc gtg cct       240
        Leu Asp Leu Pro Ile Asn Gln Asn Leu Gly Tyr Ala Tyr Ile Val Pro
        65                  70                  75                  80


        tac aaa ggg cag gcg caa ttc caa tta ggc tgg aag ggc ttt gta gca       288
        Tyr Lys Gly Gln Ala Gln Phe Gln Leu Gly Trp Lys Gly Phe Val Ala
                        85                  90                  95


        tta gct aaa aga agt ggc gca tat ttg aaa atg aat gta gta act gtc       336
        Leu Ala Lys Arg Ser Gly Ala Tyr Leu Lys Met Asn Val Val Thr Val
                        100                 105                 110


        tat caa aat caa ttc aaa tcc tac aat cgc tta aca gaa gaa tta gat       384
```

```
Tyr Gln Asn Gln Phe Lys Ser Tyr Asn Arg Leu Thr Glu Glu Leu Asp
        115                 120                 125


gcc gat ttc aca atc gaa ggc aat ggt gaa gta gtt ggt tat gca gcc    432
Ala Asp Phe Thr Ile Glu Gly Asn Gly Glu Val Val Gly Tyr Ala Ala
        130                 135                 140


tat ttc aaa gaa atc aat ggt ttt gaa aag ctt tcg ttt tgg tca att    480
Tyr Phe Lys Glu Ile Asn Gly Phe Glu Lys Leu Ser Phe Trp Ser Ile
145                 150                 155                 160


gag caa gta aaa aaa cac gcc acc aaa tac tct caa act tat ggt aaa    528
Glu Gln Val Lys Lys His Ala Thr Lys Tyr Ser Gln Thr Tyr Gly Lys
                165                 170                 175


aaa tca cgc tcg ggg gca tta atg ttt tcg cct tgg aat gat gaa gac    576
Lys Ser Arg Ser Gly Ala Leu Met Phe Ser Pro Trp Asn Asp Glu Asp
                180                 185                 190


cag ttt gac gca atg gct atg aag act gtc tta aaa aac acg ctc tca    624
Gln Phe Asp Ala Met Ala Met Lys Thr Val Leu Lys Asn Thr Leu Ser
                195                 200                 205


aag ttt ggg aca ctc tca att gaa atg caa atg gcg caa atg gca gac    672
Lys Phe Gly Thr Leu Ser Ile Glu Met Gln Met Ala Gln Met Ala Asp
                210                 215                 220


caa gca gtc atc aag aac gag ggg gag tac gag tat ata gac aat acc    720
Gln Ala Val Ile Lys Asn Glu Gly Glu Tyr Glu Tyr Ile Asp Asn Thr
225                 230                 235                 240


ata gac att gaa gct gaa agt gcc gaa gaa gaa gcc aat cgt att atg    768
Ile Asp Ile Glu Ala Glu Ser Ala Glu Glu Glu Ala Asn Arg Ile Met
                245                 250                 255


aaa ttt att gat aaa gcc gaa agc att gaa gcc tta gag gaa tta aaa    816
Lys Phe Ile Asp Lys Ala Glu Ser Ile Glu Ala Leu Glu Glu Leu Lys
                260                 265                 270


tca tca gtt gat gag aat ggc gat tta gag tta tta gcc tat tac gac    864
Ser Ser Val Asp Glu Asn Gly Asp Leu Glu Leu Leu Ala Tyr Tyr Asp
                275                 280                 285


aac aga aaa aat gaa tta aaa tga                                    888
Asn Arg Lys Asn Glu Leu Lys
```

290                              295

<210> 16
<211> 295
<212> PRT
<213> *Ornithobacterium rhinotracheale*

<400> 16

```
Met Asn Glu Leu Ala Lys Asn Asp Ile Lys Ser Leu Leu Lys Ser Ala
1               5               10              15

Asp Ile Asn Lys Arg Phe Glu Gln Leu Leu Gly Lys Lys Ala Gln Gly
            20              25              30

Phe Ile Ser Ser Val Leu Gln Thr Ala Gln Asn Asn Arg Leu Leu Ala
        35              40              45

Thr Ala Asp Pro Lys Thr Ile Leu Asn Ala Ala Val Thr Ala Ala Thr
    50              55              60

Leu Asp Leu Pro Ile Asn Gln Asn Leu Gly Tyr Ala Tyr Ile Val Pro
65              70              75              80

Tyr Lys Gly Gln Ala Gln Phe Gln Leu Gly Trp Lys Gly Phe Val Ala
            85              90              95

Leu Ala Lys Arg Ser Gly Ala Tyr Leu Lys Met Asn Val Val Thr Val
            100             105             110

Tyr Gln Asn Gln Phe Lys Ser Tyr Asn Arg Leu Thr Glu Glu Leu Asp
            115             120             125

Ala Asp Phe Thr Ile Glu Gly Asn Gly Glu Val Val Gly Tyr Ala Ala
    130             135             140
```

```
Tyr Phe Lys Glu Ile Asn Gly Phe Glu Lys Leu Ser Phe Trp Ser Ile
145             150             155             160


Glu Gln Val Lys Lys His Ala Thr Lys Tyr Ser Gln Thr Tyr Gly Lys
                165             170             175


Lys Ser Arg Ser Gly Ala Leu Met Phe Ser Pro Trp Asn Asp Glu Asp
            180             185             190


Gln Phe Asp Ala Met Ala Met Lys Thr Val Leu Lys Asn Thr Leu Ser
            195             200             205


Lys Phe Gly Thr Leu Ser Ile Glu Met Gln Met Ala Gln Met Ala Asp
            210             215             220


Gln Ala Val Ile Lys Asn Glu Gly Glu Tyr Glu Tyr Ile Asp Asn Thr
225             230             235             240


Ile Asp Ile Glu Ala Glu Ser Ala Glu Glu Glu Ala Asn Arg Ile Met
                245             250             255


Lys Phe Ile Asp Lys Ala Glu Ser Ile Glu Ala Leu Glu Glu Leu Lys
            260             265             270


Ser Ser Val Asp Glu Asn Gly Asp Leu Glu Leu Leu Ala Tyr Tyr Asp
            275             280             285


Asn Arg Lys Asn Glu Leu Lys
    290             295
```